(19) Europäisches Patentamt
European Patent Office
Office européen des brevets

(11) EP 1 457 557 A1

(12) **EUROPEAN PATENT APPLICATION**
published in accordance with Art. 158(3) EPC

(43) Date of publication:
**15.09.2004 Bulletin 2004/38**

(21) Application number: **02805480.7**

(22) Date of filing: **19.12.2002**

(51) Int Cl.[7]: **C12N 15/09**, C12N 15/12, C07K 14/705, C07K 16/28, C12N 1/15, C12N 1/19, C12N 5/10, C12P 21/02, G01N 33/50, G01N 33/15, A61K 38/17

(86) International application number:
**PCT/JP2002/013290**

(87) International publication number:
**WO 2003/054190 (03.07.2003 Gazette 2003/27)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority:
**21.12.2001 JP 2001389361**
**25.12.2001 JP 2001392577**
**26.12.2001 JP 2001394947**
**26.12.2001 JP 2001395467**
**06.02.2002 JP 2002030010**
**08.02.2002 JP 2002033095**
**06.06.2002 JP 2002165336**

(71) Applicant: **Takeda Chemical Industries, Ltd.**
**Osaka-shi, Osaka 541-0045 (JP)**

(72) Inventors:
- **NAKANISHI, Atsushi**
  **Tsukuba-shi, Ibaraki 305-0025 (JP)**
- **SAGIYA, Yoji**
  **Tsukuba-shi, Ibaraki 305-0821 (JP)**
- **UNO, Yumiko**
  **Tsukuba-shi, Ibaraki 305-0035 (JP)**

(74) Representative: **Lewin, John Harvey**
**Takeda Patent Office,**
**11-12 Charles II Street**
**London SW1Y 4QU (GB)**

# (54) NOVEL PROTEINS AND DNAS THEREOF

(57) Novel glucose transporter proteins, novel vesicular glutamate transporter proteins and a novel potential-dependent $K^+$ channel protein, are useful as, for example, diagnostic markers, hyperlipemia, arteriosclerosis, etc. These proteins, compounds promoting or inhibiting the activities thereof, etc., which are obtained by a screening method with the use of these proteins, and so on, are usable as safe drugs for the prevention/treatment of the diseases above.



Printed by Jouve, 75001 PARIS (FR)

## Description

## TECHNICAL FIELD

The present invention provides novel glucose transporter proteins and novel vesicular glutamate transporter proteins as well as a novel potential-dependent $K^+$ channel protein, DNAs encoding these proteins, a method of screening compounds that promote or inhibit the activities of these proteins, compounds obtained by the screening method, etc.

## BACKGROUND ART

For intracellular or extracellular translocation of glucose, fructose or galactose, it is required that membrane proteins called glucose transporters are present on cell membranes. Transporters for glucose, fructose or galactose are broadly classified into glucose transporters (GLUT) and $Na^+$/glucose cotransporters (SGLT). GLUT is a transporter of facilitated diffusion type effecting transport dependent on a concentration gradient, and 8 isoforms exist that share a common structure to traverse the cell membrane of about 50,000 molecular weight 12 times. SGLT is a passive transporter which is coupled to $Na^+$ ion transport thereby to transport glucose against its concentration gradient, and shares a common structure to traverse the cell membrane of 75,000 molecular weight 14 times.

GLUT isoforms differ in their tissue or organ expression. For example, GLUT4 and GLUT1 are expressed in adipocytes. In pancreatic β cells and liver cells, it is considered that GLUT2 is mainly expressed. GLUT2 is characterized by its low affinity and maximum transport rate to glucose. In pancreatic β cells, GLUT2 is predicted to function as a glucose sensor showing a glucose level-dependent insulin secretion activity together with glucokinase by the uptake of glucose depending on blood sugar level. In liver cells, GLUT2 functions as a glucose transporter by taking up blood glucose into cells upon postprandial hyperglycemia and upon fasting by releasing glucose produced in cells into blood through glycogenolysis or gluconeogenesis, depending on glucose level gradient inside or outside the cells. Normally, GLUT4 and GLUT1 are present in vesicles beneath cell membrane and in response to insulin stimulation, the vesicles fuse to the cell membrane to promote glucose uptake. Without insulin stimulation, these transporters are taken up into the vesicles and get back in the cells. Through a series of this cycle, GLUT4 contributes to the regulation of blood glucose level. GLUT5 expressed in chorioepithelium of small intestine, sperm, etc. has no affinity to glucose but high affinity to fructose, and takes part in the uptake of dietary fructose from the intestinal tract (J. Biol. Chem., 267, 14523, 1992).

Glutamic acid or glutamate is a main excitatory neurotransmitter in the mammal central nervous system and has been suggested to play a role in the signal transduction, plasticity and neurogenesis of the brain/nervous system as well as in the neuronal apoptosis in various pathological conditions. Glutamate is bio-synthesized chiefly in the brain and its uptake from outside to inside cells is achieved by $Na^+$/$K^+$-dependent glutamate transporter. Intracellular glutamate is taken up into vesicles via a specific transporter present on synaptic vesicles and released from the vesicles by stimulation. Recently it has been unraveled that brain-specific $Na^+$-dependent inorganic phosphate transporter (BNPI) is a specific transporter for transporting glutamate into synaptic vesicles (Nature, 407, 189-194, 2000; Science, 289, 957-960, 2000) and BNPI has also been called vesicular glutamate transporter 1 (VGLUT1). Furthermore, it has been shown that differentiation-associated Na+-dependent inorganic phosphate transporter (DNPI) having a high homology to BNPI has a similar function and DNPI has also been termed VGLUT2 (J. Biol. Chem., 276, 43400-43406, 2001).

$K^+$ channels, which are membrane proteins for selectively passing $K^+$ ions, are found in ubiquitously from prokaryotes to human and are composed of so many families. The channels are distributed over a broad range of tissues in vivo and are deeply involved in critical physiological functions, including generation of resting membrane potential in cells, depolarization, regulation of action potential generation frequency, etc.

The structure of $K^+$ channels is diverse; in addition to 6, 2 and 1 transmembrane types, etc., there are also present channel molecules of coupled type wherein two of these basic domains are coupled.

Voltage-dependent $K^+$ channel (Kv) belongs to the 6 transmembrane type, in which 3 subunits of α, β, γ exist. Among these subunits, α-subunit has a structure with 6 transmembrane type and a pore region is present between the fifth and sixth transmembrane domain regions. Four α subunits are associated to form a $K^+$ ion selective channel. Beta-subunit, which is present in the cytoplasm, is considered to be a regulatory molecule for α-subunit. Gamma-subunit has a homology to α-subunit and possesses a structure of 6 transmembrane type. Though γ-subunit alone does not have any function as Kv, it is considered that γ-subunit will play a regulatory role for α-subunit through heteromultimer formation by α-subunit (Trends Pharmacol. Sci., 18, 474-483, 1999).

Currently available insulin secretagogues (SU agents) force to cause insulin secretion by blocking $K_{ATP}$ channels of pancreatic beta cells, irrespective of blood sugar levels. Thus, it is thought difficult to control blood sugar so that these insulin secretagogues involve side effects such as causing hypoglycemia or inducing obesity by overexpression of insulin. In addition, these drugs lead to SU failure in average 10 years, which phenomenon is called secondary failure of SU agents. It has been postulated that this failure is also due to SU-induced exhaustion of pancreatic beta cells. By activation of the GLUT functions it can be expected to increase the uptake of sugar into pancreatic β cells and stimulate blood sugar-dependent insulin secretion. Also, activators for the GLUT functions are expected to be free from the side effects of insulin secretagogues (SU agents) currently used.

On the other hand, hypoglycemic drugs have been developed by inhibiting sugar absorption in the body. Currently, α-glucosidase inhibitors are commercially available as drugs for suppressing postprandial hyperglycemia. These inhibitors exert their pharmaceutical effects by delaying conversion of polysaccharides into glucose. However, since the glucose level finally taken up in the body cannot be suppressed, its hypoglycemic activity over a long period of time is weak. Drugs specifically inhibiting reabsorption of sugar in the kidney or absorption of sugar in the intestinal tract are expected to be excellent hypoglycemic drugs.

It is considered that vesicular glutamate transporter (VGLUT) plays a key role in transporting glutamate into synaptic vesicles, but its detailed mechanism or relation to diseases is not well established. Glutamate is a main excitatory neurotransmitter, and it is suggested that VGLUT is likely to take part largely in various diseases in the central nervous system, especially diseases concerning learning/memory. To clarify the particular function of VGLUT will lead to development of therapeutic drugs for diseases in the central nervous system, etc., associated with glutamate.

A vast number of molecular species exist in Kv, and their expression sites are diverse. Furthermore, one cell often expresses a plurality of Kv. While the characteristic features of $K^+$ ionic currents by opening each Kv have been considerably clear, there are a few findings on the signaling properties when a plurality of Kv work or on the mutual regulating actions between the respective Kvs. It is considered that Kv bears many important functions for cells, and it will lead to development of therapeutic agents for various diseases associated with Kv to unravel the relationship between its particular functions and the respective molecular species.

## DISCLOSURE OF THE INVENTION

The present inventors made extensive investigations to solve the foregoing problems and as a result, have found novel glucose transporter proteins. The proteins show 59% homology on an amino acid level and 60% homology on a base level to GLUT5 (non-patent journal 1: J. Biol. Chem., 265, 13276, 1990), have the structure of 12 transmembrane type and are capable of functioning as facilitated diffusion type transporters for sugars (e.g., glucose, fructose, galactose, etc.). For suppressing the proteins, it is considered to reduce expression level of the proteins, for example, by inhibiting the binding of ligands to the proteins, inhibiting the neurotransmitter-responsive channel activity induced by the proteins, by suppressing transcription of the proteins, etc. For activation of the proteins, it is considered to increase expression level, for example, through activation of promoters for the proteins, stabilization of mRNA, etc. It is also considered to transfer the proteins from the inside of cells to the membrane surface to increase the number of proteins functioning on the cell membrane.

The present inventors made extensive investigations to solve the foregoing problems and as a result, have found novel vesicular glutamate transporter proteins. For suppressing the proteins, it is considered to reduce expression level of the proteins, for example, by inhibiting the transport of glutamate, suppressing transcription of the proteins, etc. For activation of the proteins, it is considered to increase expression level of the proteins, for example, through promotion of glutamate transport, activation of promoters for the proteins, stabilization of mRNA, etc.

The present inventors made extensive investigations to solve the foregoing problems and as a result, have found novel voltage-dependent $K^+$ channel proteins. For modulating the proteins, it is considered, for example, to reduce expression level of the protein genes by inhibition or promotion of the permeation of ions or suppressed transcription of the protein genes, or to increase expression level of the proteins through activation of promoters for the protein genes, stabilization of mRNA, etc.

Based on these findings, the present inventors have made further investigations and as a result, come to accomplish the present invention.

That is, the present invention provides the following features.

(1) A protein containing the same or substantially the same amino acid sequence as the amino acid sequence represented by SEQ ID NO: 1 or SEQ ID NO: 23, or a salt thereof;

(2) A protein consisting of the amino acid sequence represented by SEQ ID NO: 1, or a salt thereof;

(3) A protein consisting of the amino acid sequence represented by SEQ ID NO: 23, or a salt thereof;

(4) A partial peptide of the protein according to (1), or a salt thereof;

(5) A polynucleotide containing a polynucleotide encoding the protein according to (1), or the partial peptide according to (4);

(6) The polynucleotide according to (5), which is a DNA;

(7) A DNA consisting of the base sequence represented by SEQ ID NO: 2, SEQ ID NO: 14 or SEQ ID NO: 24;

(8) A recombinant vector containing the polynucleotide according to (5);
(9) A transformant transformed by the recombinant vector according to (8);
(10) A method of manufacturing the protein according to (1) or the partial peptide according to (4), or a salt thereof, which comprises culturing the transformant according to (9), producing/accumulating the protein according to (1) or the partial peptide according to (4), and collecting the same;
(11) A pharmaceutical comprising the protein according to (1) or the partial peptide according to (4), or a salt thereof;
(12) A pharmaceutical comprising the polynucleotide according to (5);
(13) An antibody to the protein according to (1) or the partial peptide according to (4), or a salt thereof;
(14) A pharmaceutical comprising the antibody according to (13);
(15) A diagnostic product comprising the antibody according to (13);
(16) A polynucleotide containing a base sequence complementary or substantially complementary to the base sequence of the polynucleotide according to (5), or a part of the base sequence;
(17) A pharmaceutical comprising the polypeptide according to (16);
(18) A method of screening a compound or its salt that promotes or inhibits the activity of the protein according to (1) or the partial peptide according to (4), or a salt thereof, which comprises using the protein according to (1) or the partial peptide according to (4), or a salt thereof;
(19) A kit for screening a compound or its salt that promotes or inhibits the activity of the protein according to (1) or the partial peptide according to (4), or a salt thereof, comprising the protein according to (1) or the partial peptide according to (4), or a salt thereof;
(20) A compound or its salt that promotes or inhibits the activity of the protein according to (1) or the partial peptide according to (4), or a salt thereof, which is obtainable using the screening method according to (18) or the screening kit according to (19);
(21) A pharmaceutical comprising the compound or its salt according to (20);
(22) A method of screening a compound or its salt that promotes or inhibits the expression of a gene for the protein according to (1), which comprises using the polypeptide according to (5);
(23) A kit for screening a compound or its salt that promotes or inhibits the expression of a gene for the protein according to (1), comprising the polypeptide according to (5);
(24) A compound or its salt that promotes or inhibits the expression of a gene for the protein according to (1), which is obtainable using the screening method according to
(22) or the screening kit according to (23);
(25) A pharmaceutical comprising the compound or its salt according to (24);
(26) The pharmaceutical according to (11), (12), (14), (17), (21) or (25), which is an agent for the prevention/treatment of diabetes mellitus, hyperlipemia or arteriosclerosis;
(27) A method of preventing/treating diabetes mellitus, hyperlipemia or arteriosclerosis, which comprises administering an effective dose of the compound or its salt according to (20) or (24) to a mammal;
(28) Use of the compound or its salt according to (20) or (24) for manufacturing an agent for the prevention/treatment of diabetes mellitus, hyperlipemia or arteriosclerosis;
(29) A protein containing the same or substantially the same amino acid sequence as the amino acid sequence represented by SEQ ID NO: 28 or SEQ ID NO: 46, or a salt thereof;
(30) A protein consisting of the amino acid sequence represented by SEQ ID NO: 28, or a salt thereof;
(31) A protein consisting of the amino acid sequence represented by SEQ ID NO: 46, or a salt thereof;
(32) A partial peptide of the protein according to (29), or a salt thereof;
(33) A polynucleotide containing a polynucleotide encoding the protein according to (29), or the partial peptide according to (32);
(34) The polynucleotide according to (33), which is a DNA;
(35) A DNA consisting of the base sequence represented by SEQ ID NO: 29, SEQ ID NO: 45, SEQ ID NO: 47 or SEQ ID NO: 58;
(36) A recombinant vector containing the polynucleotide according to (33);
(37) A transformant transformed by the recombinant vector according to (36);
(38) A method of manufacturing the protein according to (29) or the partial peptide according to (32), or a salt thereof, which comprises culturing the transformant according to (37), producing/accumulating the protein according to (29) or the partial peptide according to (32), and collecting the same;
(39) A pharmaceutical comprising the protein according to (29) or the partial peptide according to (32), or a salt thereof;
(40) A pharmaceutical comprising the polynucleotide according to (33);
(41) An antibody to the protein according to (29) or the partial peptide according to (32), or a salt thereof;
(42) A pharmaceutical comprising the antibody according to (41);
(43) A diagnostic product comprising the antibody according to (41);

(44) A polynucleotide containing a base sequence complementary or substantially complementary to the base sequence of the polynucleotide according to (33), or a part of the base sequence;
(45) A pharmaceutical comprising the polypeptide according to (44);
(46) A method of screening a compound or its salt that promotes or inhibits the activity of the protein according to (29) or the partial peptide according to (32), or a salt thereof, which comprises using the protein according to (29) or the partial peptide according to (32), or a salt thereof;
(47) A kit for screening a compound or its salt that promotes or inhibits the activity of the protein according to (29) or the partial peptide according to (32), or a salt thereof, comprising the protein according to (29) or the partial peptide according to (32), or a salt thereof;
(48) A compound or its salt that promotes or inhibits the activity of the protein according to (29) or the partial peptide according to (32), or a salt thereof, which is obtainable using the screening method according to (46) or the screening kit according to (47);
(49) A pharmaceutical comprising the compound or its salt according to (48);
(50) A method of screening a compound or its salt that promotes or inhibits the expression of a gene for the protein according to (29), which comprises using the polypeptide according to (33);
(51) A kit for screening a compound or its salt that promotes or inhibits the expression of a gene for the protein according to (29), comprising the polypeptide according to (33);
(52) A compound or its salt that promotes or inhibits the expression of a gene for the protein according to (29), which is obtainable using the screening method according to (50) or the screening kit according to (51);
(53) A pharmaceutical comprising the compound or its salt according to (52);
(54) The pharmaceutical according to (39), (40), (42), (45), (49) or (53), which is an agent for the prevention/treatment of central nervous system disorders, endocrine diseases or diabetes mellitus;
(55) A method of preventing/treating central nervous system disorders, endocrine diseases or diabetes mellitus, which comprises administering an effective dose of the compound or its salt according to (48) or (52) to a mammal;
(56) Use of the compound or its salt according to (48) or (52) for manufacturing an agent for the prevention/treatment of central nervous system disorders, endocrine diseases or diabetes mellitus;
(57) A protein containing the same or substantially the same amino acid sequence as the amino acid sequence represented by SEQ ID NO: 62, or a salt thereof;
(58) A protein consisting of the amino acid sequence represented by SEQ ID NO: 62, or a salt thereof;
(59) A partial peptide of the protein according to (57), or a salt thereof;
(60) A polynucleotide containing a polynucleotide encoding the protein according to (57), or the partial peptide according to (59);
(61) The polynucleotide according to (60), which is a DNA;
(62) A DNA consisting of the base sequence represented by SEQ ID NO: 63 or SEQ ID NO: 83;
(63) A recombinant vector containing the polynucleotide according to (60);
(64) A transformant transformed by the recombinant vector according to (63);
(65) A method of manufacturing the protein according to (57) or the partial peptide according to (59), or a salt thereof, which comprises culturing the transformant according to (64), producing/accumulating the protein according to (57) or the partial peptide according to (59), and collecting the same;
(66) A pharmaceutical comprising the protein according to (57) or the partial peptide according to (59), or a salt thereof;
(67) A pharmaceutical comprising the polynucleotide according to (60);
(68) An antibody to the protein according to (57) or the partial peptide according to (59), or a salt thereof;
(69) A pharmaceutical comprising the antibody according to (68);
(70) A diagnostic product comprising the antibody according to (68);
(71) A polynucleotide containing a base sequence complementary or substantially complementary to the base sequence of the polynucleotide according to (60), or a part of the base sequence;
(72) A pharmaceutical comprising the polypeptide according to (71);
(73) A method of screening a compound or its salt that regulates the activity of the protein according to (57) or the partial peptide according to (59), or a salt thereof, which comprises using the protein according to (57) or the partial peptide according to (59), or a salt thereof;
(74) A kit for screening a compound or its salt that regulates the activity of the protein according to (57) or the partial peptide according to (59), or a salt thereof, comprising the protein according to (57) or the partial peptide according to (59), or a salt thereof;
(75) A compound or its salt that regulates the activity of the protein according to (57) or the partial peptide according to (59), or a salt thereof, which is obtainable using the screening method according to (73) or the screening kit according to (74);
(76) A pharmaceutical comprising the compound or its salt according to (75);

(77) A method of screening a compound or its salt that regulates the expression of a gene for the protein according to (57), which comprises using the polypeptide according to (60);
(78) A kit for screening a compound or its salt that regulates the expression of a gene for the protein according to (57), comprising the polypeptide according to (60);
(79) A compound or its salt that regulates the expression of a gene for the protein according to (57), which is obtainable using the screening method according to (77) or the screening kit according to (78);
(80) A pharmaceutical comprising the compound or its salt according to (79);
(81) The pharmaceutical according to (66), (67), (69), (72), (76) or (80), which is an agent for the prevention/ treatment of central nervous system disorders or alimentary disorders;
(82) A method of preventing/treating central nervous system disorders or alimentary disorders, which comprises administering an effective dose of the compound or its salt according to (75) or (79) to a mammal;
(83) Use of the compound or its salt according to (75) or (79) for manufacturing an agent for the prevention/ treatment of central nervous system disorders or alimentary disorders; and the like.

## BRIEF DESCRIPTION OF THE DRAWING

FIG 1 shows comparison in amino acid sequences of human TCH099, human TCH099V and GLUT5. In the figure, TCH099, TCH099V and GLUT5 designate the amino acid sequence (SEQ ID NO: 1) of human TCH099, the amino acid sequence (SEQ ID NO: 23) of human TCH099V and the amino acid sequence of GLUT5, respectively. TM1 through TM12 designates transmembrane domains. Boxes designate amino acids which are identical in at least 2 sequences among 3 sequences.
FIG 2 shows the expression level of human TCH099 gene product in each tissue. The results on the expression level of human TCH099 on cDNA of each tissue in human determined by TaqMan PCR are shown. The expression level is expressed in terms of the copy number per 1 μl of cDNA solution.
FIG. 3 shows comparison in amino acid sequences among vesicular glutamate transporter 1 (VGLUT1), vesicular glutamate transporter 2 (VGLUT2) and human TCH177. In the figure, the transmembrane domains analyzed by transmembrane domain prediction software SOSUI [Bioinformatics (formerly CABIOS), 14, 378, 1998] are shown by TM1 through TM7.
FIG 4 shows the expression level of human TCH177 gene product in each tissue. The expression level is expressed in terms of the copy number per 1 μl of cDNA solution.
FIG. 5 shows comparison in amino acid sequences between mouse TCH177 (SEQ ID NO: 19) (mTCH177) and human TCH177 (SEQ ID NO: 1) (hTCH177).
FIG. 6 shows the expression level of mouse TCH177 gene product in each tissue. The expression level is expressed in terms of the copy number per 1 μl of cDNA solution.
FIG. 7 shows comparison in amino acid sequences between Kv6.2 and human TCH136. In the figure, the transmembrane domains and the pore region are shown by TM1 through TM6 and P, respectively.
FIG 8 (A) shows the expression level of human TCH136 gene product in each tissue. The expression level is expressed in terms of the copy number per 1 μl of cDNA solution.
(B) shows the results of the expression of human TCH136 detected. In the figure, symbols + and - designate the tissue where the expression was detected and the tissue where the expression was not detected, respectively.
FIG. 9 shows the expression level of mouse TCH099 gene product in each tissue. The results of the expression level of mouse TCH099 on cDNA of each tissue in BALB/c mouse of 7 weeks old, which were determined by TaqMan PCR, are shown. The expression level is expressed in terms of the value obtained by dividing the copy number of mouse TCH099 per 1 μl of cDNA solution by the copy number of rodent GAPDH in an equal amount of cDNA in each tissue.
FIG. 10 shows the expression level of mouse TCH177 gene product in each tissue. The results of the expression level of mouse TCH177 on cDNA of each tissue in BALB/c mouse of 7 weeks old, which were determined by TaqMan PCR, are shown. The expression level is expressed in terms of the value obtained by dividing the copy number of mouse TCH177 per 1 μl of cDNA solution by the copy number of rodent GAPDH in an equal amount of cDNA in each tissue.
FIG. 11 shows the expression level of rat TCH177 gene product in each tissue. The results of the expression level of rat TCH177 on cDNA of each tissue in Wistar male rat of 12 weeks old, which were determined by TaqMan PCR, are shown. The expression level is expressed in terms of the value obtained by dividing the copy number of rat TCH177 per 1 μl of cDNA solution by the copy number of rodent GAPDH in an equal amount of cDNA in each tissue.
FIG. 12 shows the expression level of mouse TCH136 gene product in each tissue. The results of the expression level of mouse TCH136 on cDNA of each tissue in BALB/c mouse of 7 weeks old, which were determined by TaqMan PCR, are shown. The expression level is expressed in terms of the value obtained by dividing the copy number of mouse TCH136 per 1 μl of cDNA solution by the copy number of rodent GAPDH in an equal amount of cDNA in each tissue.

FIG 13 shows the results obtained by assaying the uptake of 2-[1,2-$^3$H (N)]-deoxy-D-glucose in human TCH099 expressed CHO cell line. The amount of taken up is shown by a mean value in count (DPM) of independent 3 wells and a standard deviation. In the figure, Mock and TCH099 indicate vector pcDNA3.1(+)-transfected cells and human TCH099 expressed CHO cells, respectively.

## BEST MODE FOR CARRYING OUT THE INVENTION

The protein containing the same or substantially the same amino acid sequence as the amino acid sequence represented by SEQ ID NO: 1, SEQ ID NO: 23, SEQ ID NO: 28, SEQ ID NO: 46 or SEQ ID NO: 62 (hereinafter these proteins are sometimes collectively referred to as the protein of the present invention or the protein used in the present invention) may be any protein derived from any cells of human and other warm-blooded animals (e.g., guinea pig, rat, mouse, fowl, rabbit, swine, sheep, bovine, monkey, etc.) such as hepatocyte, splenocyte, nerve cells, glial cells, pancreatic β cells, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence having substantially the same amino acid sequence as that represented by SEQ ID NO: 1 includes amino acid sequences having at least 60% homology, preferably about 70% homology, more preferably at least about 80% homology, particularly preferably at least about 90% homology and most preferably at least about 95% homology, to the amino acid sequence shown by SEQ ID NO: 1; etc.

Preferred examples of the protein containing substantially the same amino acid sequence as the amino acid sequence represented by SEQ ID NO: 1 include proteins having substantially the same amino acid sequence as the amino acid sequence represented by SEQ ID NO: 1 and having a property substantially equivalent to that of the protein having the amino acid sequence represented by SEQ ID NO: 1, etc.

The amino acid sequence having substantially the same amino acid sequence as that represented by SEQ ID NO: 23 includes amino acid sequences having at least 60% homology, preferably about 70% homology, more preferably at least about 80% homology, particularly preferably at least about 90% homology and most preferably at least about 95% homology, to the amino acid sequence shown by SEQ ID NO: 23; etc.

Preferred examples of the protein containing substantially the same amino acid sequence as the amino acid sequence represented by SEQ ID NO: 23 include proteins having substantially the same amino acid sequence as the amino acid sequence represented by SEQ ID NO: 23 and having an activity substantially equivalent to that of the protein having the amino acid sequence represented by SEQ ID NO: 23, etc.

As the substantially equivalent activities, there are, for example, transport of sugars (e.g., glucose, fructose, galactose, etc.) and the like. The substantially equivalent is used to mean that the nature of these properties is equivalent in terms of quality (e.g., physiologically or pharmacologically). Thus, the transport of sugars (e.g., glucose, fructose, galactose, etc.) is preferably equivalent (e.g., about 0.01 to 100 times, preferably about 0.1 to 10 times, more preferably 0.5 to 2 times), but differences in degree such as a level of these activities, quantitative factors such as a molecular weight of the protein, etc. may be different.

The activity of transport of sugars (e.g., glucose, fructose, galactose, etc.) can be determined by publicly known methods with modifications. For example, the activity can be assayed by the method described in J. Biol. Chem., 275, 4607-4612, 2000, or with modifications thereof.

The amino acid sequence having substantially the same amino acid sequence as that represented by SEQ ID NO: 28 includes amino acid sequences having at least 75% homology, preferably about 80% homology, more preferably at least about 85% homology, particularly preferably at least about 90% homology and most preferably at least about 95% homology, to the amino acid sequence shown by SEQ ID NO: 28; etc.

Preferred examples of the protein containing substantially the same amino acid sequence as the amino acid sequence represented by SEQ ID NO: 28 include proteins having substantially the same amino acid sequence as the amino acid sequence represented by SEQ ID NO: 28 and having a property substantially equivalent to that of the protein having the amino acid sequence represented by SEQ ID NO: 28, etc.

The amino acid sequence having substantially the same amino acid sequence as that represented by SEQ ID NO: 46 includes amino acid sequences having at least 75% homology, preferably about 80% homology, more preferably at least about 85% homology, particularly preferably at least about 90% homology and most preferably at least about 95% homology, to the amino acid sequence shown by SEQ ID NO: 46; etc.

Preferred examples of the protein containing substantially the same amino acid sequence as the amino acid sequence represented by SEQ ID NO: 46 include proteins having substantially the same amino acid sequence as the amino acid sequence represented by SEQ ID NO: 46 and having an activity substantially equivalent to that of the protein having the amino acid sequence represented by SEQ ID NO: 46, etc.

As the substantially equivalent activities, there are, for example, transport of glutamic acid, glutamate, or the like. The substantially equivalent is used to mean that the nature of these properties is equivalent in terms of quality (e.g., physiologically or pharmacologically). Thus, the transport of glutamate is preferably equivalent (e.g., about 0.01 to 100 times, preferably about 0.1 to 10 times, more preferably 0.5 to 2 times), but differences in degree such as a level of these activities, quantitative factors such as a molecular weight of the protein, etc. may be different.

The glutamate transport activity can be determined by publicly known methods with modifications. For example, the activity can be assayed by the method described in Nature, 407, 189-194, 2000, J. Biol. Chem., 276, 43400-43406, 2001, or with modifications thereof.

The amino acid sequence having substantially the same amino acid sequence as that represented by SEQ ID NO: 62 includes amino acid sequences having at least about 50% homology, preferably about 60% homology, more preferably at least about 70% homology, particularly preferably at least about 80% homology and most preferably at least about 95% homology, to the amino acid sequence shown by SEQ ID NO: 62; etc.

Preferred examples of the protein containing substantially the same amino acid sequence as the amino acid sequence represented by SEQ ID NO: 62 include proteins having substantially the same amino acid sequence as the amino acid sequence represented by SEQ ID NO: 62 and having an activity substantially equivalent to that of the protein having the amino acid sequence represented by SEQ ID NO: 62, etc.

As the substantially equivalent activities, there are, for example, permeation of $K^+$ ions, and the like. The substantially equivalent is used to mean that the nature of these properties is equivalent in terms of quality (e.g., physiologically or pharmacologically). Thus, the permeation of $K^+$ ions is preferably equivalent (e.g., about 0.01 to 100 times, preferably about 0.1 to 10 times, more preferably 0.5 to 2 times), but differences in degree such as a level of these activities, quantitative factors such as a molecular weight of the protein, etc. may be different.

The activity of the permeation of $K^+$ ions can be assayed by publicly known methods with modifications. For example, the activity can be assayed by the method described in Receptors and Channels, 6, 337-350, 1999, or with modifications thereof.

Examples of the protein used in the present invention include (1) so-called muteins such as proteins containing 1) the amino acid sequence represented by SEQ ID NO: 1 or SEQ ID NO: 23, of which at least 1 or 2 (for example, about 1 to about 200, preferably about 1 to about 150, preferably about 1 to about 100, more preferably about 1 to about 30, much more preferably about 1 to about 10, and most preferably several (1 to 5)) amino acids are deleted; 2) the amino acid sequence represented by SEQ ID NO: 1 or SEQ ID NO: 23, to which at least 1 or 2(for example, about 1 to about 200, preferably about 1 to about 150, preferably about 1 to about 100, more preferably about 1 to about 30, much more preferably about 1 to about 10, and most preferably several (1 to 5)) amino acids are added; 3) the amino acid sequence represented by SEQ ID NO: 1 or SEQ ID NO: 23, in which at least 1 or 2 (for example, about 1 to about 200, preferably about 1 to about 150, preferably about 1 to about 100, more preferably about 1 to about 30, much more preferably about 1 to about 10, and most preferably several (1 to 5)) amino acids are inserted; 4) the amino acid sequence represented by SEQ ID NO: I or SEQ ID NO: 23, in which at least 1 or 2 (for example, about 1 to about 200, preferably about 1 to about 150, preferably about 1 to about 100, more preferably about 1 to about 30, much more preferably about 1 to about 10, and most preferably several (1 to 5)) amino acids are substituted by other amino acids; or 5) a combination of these amino acid sequences;

(2) so-called muteins such as proteins containing 1) the amino acid sequence represented by SEQ ID NO: 28 or SEQ ID NO: 46, of which at least 1 or 2 (for example, about 1 to about 200, preferably about 1 to about 150, preferably about 1 to about 100, more preferably about 1 to about 30, much more preferably about 1 to about 10, and most preferably several (1 to 5)) amino acids are deleted; 2) the amino acid sequence represented by SEQ ID NO: 28 or SEQ ID NO: 46, to which at least 1 or 2(for example, about 1 to about 200, preferably about 1 to about 150, preferably about 1 to about 100, more preferably about 1 to about 30, much more preferably about 1 to about 10, and most preferably several (1 to 5)) amino acids are added; 3) the amino acid sequence represented by SEQ ID NO: 28 or SEQ ID NO: 46, in which at least 1 or 2 (for example, about 1 to about 200, preferably about I to about 150, preferably about 1 to about 100, more preferably about 1 to about 30, much more preferably about 1 to about 10, and most preferably several (1 to 5)) amino acids are inserted; 4) the amino acid sequence represented by SEQ ID NO: 28 or SEQ ID NO: 46, in which at least 1 or 2 (preferably about 1 to about 30, more preferably about 1 to about 10 and most preferably several (1 to 5)) amino acids are substituted by other amino acids; or 5) a combination of these amino acid sequences; and,

(3) so-called muteins such as proteins containing 1) the amino acid sequence represented by SEQ ID NO: 62, of which at least 1 or 2 (for example, about 1 to about 200, preferably about 1 to about 150, preferably about 1 to about 100, more preferably about 1 to about 30, much more preferably about 1 to about 10, and most preferably several (1 to 5)) amino acids are deleted; 2) the amino acid sequence represented by SEQ ID NO: 62, to which at least 1 or 2(for example, about 1 to about 200, preferably about 1 to about 150, preferably about 1 to about 100, more preferably about 1 to about 30, much more preferably about 1 to about 10, and most preferably several (1 to 5)) amino acids are added; 3) the amino acid sequence represented by SEQ ID NO: 62, in which at least 1 or 2 (for example, about 1 to about 200, preferably about 1 to about 150, preferably about 1 to about 100, more preferably about 1 to about 30, much more preferably about 1 to about 10, and most preferably several (1 to 5)) amino acids are inserted; 4) the amino acid sequence represented by SEQ ID NO: 62, in which at least 1 or 2 (preferably about 1 to about 30, more preferably about 1 to about 10 and most preferably several (1 to 5)) amino acids are substituted by other amino acids; or 5) a combination of these amino acid sequences.

Where the amino acid sequences are inserted, deleted or substituted as described above, the position to be inserted, deleted or substituted is not particularly limited.

Throughout the specification, the proteins are represented in accordance with the conventional way of describing peptides, that is, the N-terminus (amino terminus) at the left hand and the C-terminus (carboxyl terminus) at the right hand. In the protein used in the present invention including the protein containing the amino acid sequence shown by SEQ ID NO: 1, the C-terminus may be in any form of a carboxyl group (-COOH), a carboxylate (-COO-), an amide ($-CONH_2$) and an ester (-COOR).

Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl such as a phenyl-$C_{1-2}$ alkyl group, e.g., benzyl, phenethyl, etc.; an α-naphthyl-$C_{1-2}$ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains; etc.

Specific examples of the protein used in the present invention are a protein containing the amino acid sequence represented by SEQ ID NO: 1, a protein containing the amino acid sequence represented by SEQ ID NO: 23, a protein containing the amino acid sequence represented by SEQ ID NO: 28, a protein containing the amino acid sequence represented by SEQ ID NO: 46, a protein containing the amino acid sequence represented by SEQ ID NO: 62, and the like.

As partial peptides of the protein used in the present invention, any partial peptide can be used so long as it is a partial peptide of the protein used in the present invention described above, preferably has the property equivalent to that of the protein used in the present invention described above.

For example, there are used peptides containing at least 5, preferably at least 10, preferably at least 15, preferably at least 20, preferably at least 50, more preferably at least 70, much more preferably at least 100, and most preferably at least 200, amino acids in the constituent amino acid sequence of the protein of the present invention, and the like.

The partial peptide used in the present invention may be peptides containing the amino acid sequence, of which at least 1 or 2 (preferably about 1 to about 20, more preferably about 1 to about 10 and most preferably several (1 to 5)) amino acids may be deleted; peptides, to which at least 1 or 2 (preferably about 1 to about 20, more preferably about 1 to about 10 and most preferably several (1 to 5)) amino acids may be added; peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 10, more preferably several and most preferably about 1 to about 5) amino acids may be substituted by other amino acids.

Examples of the partial peptides of the present invention include peptides containing the amino acid sequence represented by SEQ ID NO: 1, in which the 46-78, 219-283 or 464-512 amino acid sequence is contained (peptides containing the amino acid sequence represented by SEQ ID NO: 23, in which the 55-87, 228-292 or 473-521 amino acid sequence is contained), etc.; peptides containing the amino acid sequence represented by SEQ ID NO: 28, in which the 200-234 or 336-347 amino acid sequence is contained, etc.; SEQ ID NO: 46, in which the 213-247 or 349-360 amino acid sequence is contained, etc.; peptides containing the amino acid sequence represented by SEQ ID NO: 62, in which the 192-220, 274-288 or 344-360 amino acid sequence is contained, etc.

In the partial peptide used in the present invention, the C-terminus may be in any form of a carboxyl group (-COOH), a carboxylate (-COO-), an amide (-$CONH_2$) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as so-called glycoproteins having sugar chains; etc., as in the protein used in the present invention described above.

The partial peptide used in the present invention may also be used as an antigen for producing antibodies. For the purpose of preparing the antibodies of the present invention, examples include peptides containing the amino acid sequence represented by SEQ ID NO: 1, in which the 46-78, 219-283 or 464-512 amino acid sequence is contained (peptides containing the amino acid sequence represented by SEQ ID NO: 23, in which the 55-87, 228-292 or 473-521 amino acid sequence is contained), etc.; peptides containing the amino acid sequence represented by SEQ ID NO: 28, in which the 200-234 or 336-347 amino acid sequence is contained, etc.; SEQ ID NO: 46, in which the 213-247 or 349-360 amino acid sequence is contained, etc.; peptides containing the amino acid sequence represented by SEQ ID NO: 62, in which the 192-220, 274-288 or 344-360 amino acid sequence is contained, etc.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts among others. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by a publicly known method used to purify a protein from human or other warm-blooded animal cells or tissues described above. Alternatively, they may also be manufactured by culturing transformants containing DNAs encoding the protein. Furthermore, they may also be manufactured by a modification of the methods for peptide synthesis, which will be described hereinafter.

Where these proteins are manufactured from human or other mammalian tissues or cells, human or other mammalian tissues or cells are homogenized, extracted with an acid or the like, and the extract is isolated and purified by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which $\alpha$-amino groups and functional groups on the side chains are appropriately protected, are condensed on the resin in the order of the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; pyridine, ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To obtain the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide of the present invention are condensed with the remaining part. Where the product contains protecting groups, these protecting groups are removed to give the desired peptide. Publicly known methods for condensation and elimination of the protecting groups are described in 1) to 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: Peptide Gosei-no-Kiso to Jikken (Basics and experiments of peptide synthesis), published by Maruzen Co. (1975)
4) Haruaki Yajima & Shunpei Sakakibara: Seikagaku Jikken Koza (Biochemical Experiment) 1, Tanpakushitsu no Kagaku (Chemistry of Proteins) IV, 205 (1977)
5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method; when the partial peptide is obtained in a salt form, it can be converted into a free form or other different salt form by a publicly known method.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid, ane the like. The DNA may also be directly amplified by reverse transcriptase polymerase chain reaction (hereinafter abbreviated as RT-PCR) using the total RNA or mRNA fraction prepared from the cells/tissues described above.

The DNA encoding the protein of the present invention may be, for example,

(1) any one of a DNA having the base sequence represented by SEQ ID NO: 2 or SEQ ID NO: 14, or any DNA having a base sequence hybridizable to a DNA having the base sequence represented by SEQ ID NO: 2 or SEQ ID NO: 14 under high stringent conditions and encoding a protein which has the properties substantially equivalent to those of the protein containing the amino acid sequence represented by SEQ ID NO: 1,
(2) any DNA containing the base sequence represented by SEQ ID NO: 24, or any DNA having a base sequence hybridizable to a DNA having the base sequence represented by SEQ ID NO: 24 under high stringent conditions and encoding a protein which has the properties substantially equivalent to those of the protein containing the amino acid sequence represented by SEQ ID NO: 23,
(3) any DNA containing the base sequence represented by SEQ ID NO: 29 or SEQ ID NO: 45, or any DNA having a base sequence hybridizable to a DNA having the base sequence represented by SEQ ID NO: 29 or SEQ ID NO: 45 under high stringent conditions and encoding a protein which has the properties substantially equivalent to those of the protein containing the amino acid sequence represented by SEQ ID NO: 28,
(4) any DNA containing the base sequence represented by SEQ ID NO: 47 or SEQ ID NO: 58, or any DNA having a base sequence hybridizable to a DNA having the base sequence represented by SEQ ID NO: 47 or SEQ ID NO: 58 under high stringent conditions and encoding a protein which has the properties substantially equivalent to those of the protein containing the amino acid sequence represented by SEQ ID NO: 46,
(5) any DNA containing the base sequence represented by SEQ ID NO: 63 or SEQ ID NO: 83, or any DNA having a base sequence hybridizable to a DNA having the base sequence represented by SEQ ID NO: 63 or SEQ ID NO: 83 under high stringent conditions and encoding a protein which has the properties substantially equivalent to those of the protein containing the amino acid sequence represented by SEQ ID NO: 62, etc.

Specific examples of the DNA that is hybridizable to a DNA having the base sequence represented by SEQ ID NO: 2 or SEQ ID NO: 14 under high stringent conditions include DNAs having at least 65% homology, preferably at least about 70% homology, preferably at least about 80% homology, more preferably at least about 85% homology, particularly preferably at least about 90% and most preferably at least about 95% homology, to the base sequence represented by SEQ ID NO: 2 or SEQ ID NO: 14.

Specific examples of the DNA that is hybridizable to a DNA having the base sequence represented by SEQ ID NO: 24 under high stringent conditions include DNAs having at least 65% homology, preferably at least about 70% homology, preferably at least about 80% homology, more preferably at least about 85% homology, particularly preferably at least about 90% and most preferably at least about 95% homology, to the base sequence represented by SEQ ID NO: 24.

Specific examples of the DNA that is hybridizable to a DNA having the base sequence represented by SEQ ID NO: 29 or SEQ ID NO: 45 under high stringent conditions include DNAs having at least 65% homology, preferably at least about 70% homology, preferably at least about 80% homology, more preferably at least about 85% homology, particularly preferably at least about 90% and most preferably at least about 95% homology, to the base sequence represented by SEQ ID NO: 29 or SEQ ID NO: 45.

Specific examples of the DNA that is hybridizable to a DNA having the base sequence represented by SEQ ID NO: 47 or SEQ ID NO: 58 under high stringent conditions include DNAs having at least about 70% homology, preferably at least about 80% homology, more preferably at least about 85% homology, particularly preferably at least about 90% and most preferably at least about 95% homology, to the base sequence represented by SEQ ID NO: 47 or SEQ ID NO: 58.

Specific examples of the DNA that is hybridizable to a DNA having the base sequence represented by SEQ ID NO: 63 or SEQ ID NO: 83 under high stringent conditions include DNAs having at least 60% homology, preferably at least about 70% homology, preferably at least about 80% homology, more preferably at least about 85% homology, particularly preferably at least about 90% and most preferably at least about 95% homology, to the base sequence represented by SEQ ID NO: 63 or SEQ ID NO: 83.

The hybridization can be carried out by publicly known methods or by a modification thereof, for example, according to the method described in Molecular Cloning, 2nd (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the protein containing the amino acid sequence represented by SEQ ID NO: 1, there may be employed a DNA containing a DNA containing the base sequence represented by SEQ ID NO: 2 or SEQ ID NO: 14, etc.; as the DNA encoding the protein containing the amino acid sequence represented by SEQ ID NO: 23, there may be employed a DNA containing a DNA containing the base sequence represented by SEQ ID NO: 24, etc.; as the DNA encoding the protein containing the amino acid sequence represented by SEQ ID NO: 28, there may be employed a DNA containing a DNA containing the base sequence represented by SEQ ID NO: 29 or SEQ ID NO: 45, etc.; and as the DNA encoding the protein containing the amino acid sequence represented by SEQ ID NO: 46, there may be employed a DNA containing a DNA containing the base sequence represented by SEQ ID NO: 47 or SEQ ID NO: 58, etc.; as the DNA encoding the protein containing the amino acid sequence represented by SEQ ID NO: 62, there may be employed a DNA containing a DNA containing the base sequence represented by SEQ ID NO: 63 or SEQ ID NO: 83, etc. The DNA encoding the partial peptide used in the present invention may be any DNA so long as it contains the base sequence encoding the partial peptide used in the present invention described above. Also, the DNA may be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, (1) a DNA containing a part of DNA containing the base sequence represented by SEQ ID NO: 2, SEQ ID NO: 14, SEQ ID NO: 24, SEQ ID NO: 29, SEQ ID NO: 45, SEQ ID NO: 47, SEQ ID NO: 58, SEQ ID NO: 63 or SEQ ID NO: 83, or (2) a DNA containing a base sequence hybridizable to the base sequence represented by SEQ ID NO: 2, SEQ ID NO: 14, SEQ ID NO: 24, SEQ ID NO: 29, SEQ ID NO: 45, SEQ ID NO: 47, SEQ ID NO: 58, SEQ ID NO: 63 or SEQ ID NO: 83 under high stringent conditions and containing a part of DNA encoding a protein having the activities substantially equivalent to those of the protein of the present invention, etc.

The DNA hybridizable to the base sequence represented by SEQ ID NO: 2, SEQ ID NO: 14, SEQ ID NO: 24, SEQ ID NO: 29, SEQ ID NO: 45, SEQ ID NO: 47, SEQ ID NO: 58, SEQ ID NO: 63 or SEQ ID NO: 83 has the same significance as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence encoding the protein of the present invention, or the DNA inserted into an appropriate vector can be screened by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Conversion of the base sequence of DNA can be effected by publicly known methods such as the ODA-LA PCR method, the Gapped duplex method, the Kunkel method, etc., or its modification, using PCR or a publicly known kit available as Mutan™-super Express Km or Mutan™-K (both manufactured by Takara Shuzo Co., Ltd.), etc.

The cloned DNA encoding the protein of the present invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adaptor.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, $\lambda P_L$ promoter, 1pp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)), C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO ($dhfr^-$) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20°C to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, in the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be appropriately modified to partially remove the polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide used in the present invention, or its salts.

The antibodies to the protein or partial peptide used in the present invention, or its salts, (hereinafter they are sometimes collectively referred to as the protein of the present invention in the description of the antibodies) can be produced by a publicly known method of producing an antibody or antiserum, using the protein of the present invention as an antigen.

**[Preparation of monoclonal antibody]**

(a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

**[Preparation of polyclonal antibody]**

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantial complementary base sequence to the DNA encoding the protein or partial peptide used in the present invention (hereinafter these DNAs are sometimes collectively referred to as the DNA of the present invention in the description of antisense polynucleotide) can be any antisense polynucleotide, so long as it possesses a base sequence. complementary or substantially complementary base sequence to that of the DNA of the present invention and capable of suppressing expression of the DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the full-length base sequence or to the partial base sequence (i.e., complementary strand to the DNA of the present invention), and the like. Especially in the full-length base sequence of the complementary strand to the DNA of the present invention, preferred is an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon).

Specific examples include an antisense polynucleotide containing the entire or part of a base sequence complementary or substantially complementary to a base sequence of DNA containing the base sequence represented by SEQ ID NO: 2, SEQ ID NO: 14, SEQ ID NO: 24, SEQ ID NO: 29, SEQ ID NO: 45, SEQ ID NO: 47, SEQ ID NO: 58, SEQ ID NO: 63 or SEQ ID NO: 83, etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. These antisense nucleotides may be synthesized using a publicly known DNA synthesizer, etc.

According to the present invention, the antisense polynucleotide (nucleic acid) that can inhibit replication or expression of a gene for the protein of the present invention can be designed and synthesized based on the base sequence information of the cloned or determined DNA encoding the protein. Such a polynucleotide (nucleic acid) is hybridizable to RNA of a gene for the protein of the present invention to inhibit the synthesis or function of the RNA or is capable of modulating/controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating/controlling the in vivo and in vitro expression of a gene for the protein of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the gene for the protein, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the gene for the protein.

The relationship between the targeted nucleic acids and the polynucleotides complementary to at least a part of the target region, specifically the relationship between the target nucleic acids and the polynucleotides hybridizable to the target region, can be denoted to be "antisense" to the polynucleotides in the said target region. Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cell permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the polynucleotide at the 3' or 5' ends thereof and may also be attached thereto through a base, sugar, or intramolecular nucleoside linkage. Other moieties may be capping groups specifically placed at the 3' or 5' ends of the polynucleotide to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense polypeptide can be examined using the transformant of the present invention, the gene expression system of the present invention in vivo and in vitro, or the translation system of the protein of the present invention in vivo and in vitro. The nucleic acid itself can be applied to cells by a variety of publicly known methods.

Hereinafter, the protein or partial peptide of the present invention, or salts thereof (hereinafter sometimes merely referred to as the protein of the present invention), the polypeptide encoding the protein or partial peptide of the present invention, preferably DNA (hereinafter sometimes merely referred to as the DNA of the present invention), the antibodies to the protein of the present invention, its partial peptides, or salts thereof (hereinafter sometimes referred to as the antibodies of the present invention) and the antisense polynucleotides to the DNA of the present invention (hereinafter sometimes merely referred to as the antisense polynucleotides of the present invention) are specifically described for their applications.

Also, the protein containing the amino acid sequence represented by SEQ ID NO: 1 or SEQ ID NO: 23, the protein containing the amino acid sequence represented by SEQ ID NO: 28 or SEQ ID NO: 46 and the protein containing the amino acid sequence represented by SEQ ID NO: 62 are sometimes referred to as "the protein A of the present invention," "the protein B of the present invention" and "the protein C of the present invention," respectively.

**[1] Agents for the prevention/treatment of various diseases with which the protein of the invention is associated**

The protein A of the present invention has the transport activity of sugars (e.g., glucose, fructose, galactose, etc.), etc. and is responsible for the transport of sugars (e.g., glucose, fructose, galactose, etc.).

Therefore, where the DNA encoding the protein A of the present invention is abnormal or deficient, or where the expression level of the protein of the present invention is reduced, such causes a variety of diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like.

Therefore, the protein A of the present invention and the DNA encoding the same can be used as pharmaceuticals, including agents for the prevention/treatment of diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of diabetes mellitus, hyperlipemia, arteriosclerosis, etc.

For example, when the transport of sugars (e.g., glucose, fructose, galactose, etc.) cannot be expected in a patient sufficiently or normally due to a decrease or deficiency of the protein A of the present invention in the body, the role of the protein of the present invention can be exhibited sufficiently or normally: (a) by administering the DNA of the present invention directly to the patient thereby to express the protein A of the present invention; (b) by inserting the DNA of the present invention into cells to express the protein A of the present invention and transplant the cells to the patient; or (c) by administering the protein A of the present invention to the patient.

The protein B of the present invention has the glutamate transport activity and is responsible for the transport of glutamate and at the same time, plays a critical role for glutamate metabolism, etc.

Therefore, the protein B of the present invention and the DNA encoding the same can be used as pharmaceuticals, including agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, endocrine disorders, diabetes mellitus, etc.

For example, when the glutamate transport cannot be expected in a patient sufficiently or normally due to a decrease or deficiency of the protein B of the present invention in the body, the role of the protein B of the present invention can be exhibited sufficiently or normally: (a) by administering the DNA of the present invention directly to the patient thereby to express the protein B of the present invention; (b) by inserting the DNA of the present invention into cells to express the protein B of the present invention and transplant the cells to the patient; or (c) by administering the protein B of the present invention to the patient.

The protein C of the present invention has the activity of $K^+$ ion permeation, etc. and is responsible for the $K^+$ ion permeation and at the same time, plays an important role for generation of cell membrane potential.

Therefore, the protein C of the present invention and the DNA encoding the same can be used as pharmaceuticals, including agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

For example, when the K+ ion permeation activity cannot be expected in a patient sufficiently or normally due to a decrease or deficiency of the protein C of the present invention in the body, the role of the protein C of the present invention can be exhibited sufficiently or normally: (a) by administering the DNA of the present invention directly to the patient thereby to express the protein C of the present invention; (b) by inserting the DNA of the present invention into cells to express the protein C of the present invention and transplant the cells to the patient; or (c) by administering the protein C of the present invention to the patient.

When the polynucleotide (e.g., DNA) of the present invention is used as the agents for the prevention/treatment described above, the DNA alone of the present invention is administered directly, or the DNA is inserted into an appropriate vector such as retrovirus vector, adenovirus vector, adenovirus-associated virus vector, etc., followed by administering the same to human or other warm-blooded animal in a conventional manner. The DNA of the present invention may also be administered as it stands, or may be prepared in pharmaceutical preparations together with a physiologically acceptable carrier to assist its uptake, which are then administered by gene gun or through a catheter such as a catheter with a hydrogel.

Where the protein of the present invention is used as the agents for the prevention/treatment described above, it is preferred to use the protein purified to at least 90%, preferably at least 95%, more preferably at least 98% and most preferably at least 99%.

The protein of the present invention can be used orally, for example, in the form of tablets which may be sugar coated if necessary, capsules, elixirs, microcapsules, etc., or parenterally in the form of injectable preparations such as a sterile solution, a suspension, etc. in water or with other pharmaceutically acceptable liquid. These preparations can be prepared, for example, by mixing the protein, etc. of the present invention with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil or cherry, and the like. When the unit dosage is in the form of capsules, liquid carriers such as oils and fats may further be used together with the additives described above. A sterile composition for injection may be formulated by conventional procedures used to make pharmaceutical preparations, e.g., by dissolving or suspending the active ingredients in a vehicle such as water for injection with a naturally occurring vegetable oil such as sesame oil, coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol, polyethylene glycol, etc.), a nonionic surfactant (e.g., polysorbate 80™, HCO-50, etc.), and the like. Examples of the oily medium include sesame oil and soybean oil, which may also be used in combination with a dissolution aid such as benzyl benzoate, benzyl alcohol, etc. The agent may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector inserted with the DNA of the present invention is prepared into pharmaceutical preparations as described above and normally provided for use parenterally.

Since the thus obtained pharmaceutical preparation is safe and low toxic, the preparation can be administered to warm-blooded animals (e.g., human, rats, mice, guinea pigs, rabbits, fowl, sheep, swine, bovine, horses, cats, dogs, monkeys, chimpanzees, etc.).

The dose of the protein of the present invention may vary depending upon target disease, subject to be administered, route of administration, etc. In oral administration of the protein A of the present invention for the treatment of, e.g., diabetes mellitus, generally the protein A is administered to an adult (as 60 kg) in a dose of about 0.1 mg to 100 mg, preferably about 1.0 to 50 mg and more preferably about 1.0 to 20 mg per day. In parenteral administration, the single dose of the protein A may vary depending on subject to be administered, target disease, etc. When the protein A of the present invention is administered to an adult (as 60 kg body weight) in the form of injectable preparations for the treatment of, e.g., diabetes mellitus, it is advantageous to inject the protein at the affected area in a daily dose of about 0.01 to about 30 mg, preferably about 0.1 to about 20 mg and more preferably about 0.1 to about 10 mg. For other animal species, the corresponding dose as converted per 60 kg can be administered.

In oral administration of the protein B of the present invention for the treatment of, e.g., Alzheimer's disease, generally the protein B is administered to an adult (as 60 kg) in a dose of about 0.1 mg to 100 mg, preferably about 1.0 to 50 mg and more preferably about 1.0 to 20 mg per day. In parenteral administration, the single dose of the protein may vary depending on subject to be administered, target disease, etc. When the protein B of the present invention is administered to an adult (as 60 kg body weight) in the form of injectable preparations for the treatment of, e.g., Alzheimer's disease, it is advantageous to inject the protein at the affected area in a daily dose of about 0.01 to about 30 mg, preferably about 0.1 to about 20 mg and more preferably about 0.1 to about 10 mg. For other animal species, the corresponding dose as converted per 60 kg can be administered.

In oral administration of the protein C of the present invention for the treatment of, e.g., alimentary disease, generally the protein is administered to an adult (as 60 kg) in a dose of about 0.1 mg to 100 mg, preferably about 1.0 to 50 mg and more preferably about 1.0 to 20 mg per day. In parenteral administration, the single dose of the protein may vary depending on subject to be administered, target disease, etc. When the protein C of the present invention is administered to an adult (as 60 kg body weight) in the form of injectable preparations for the treatment of, e.g., alimentary disease, it is advantageous to inject the protein at the affected area in a daily dose of about 0.01 to about 30 mg, preferably about 0.1 to about 20 mg and more preferably about 0.1 to about 10 mg. For other animal species, the corresponding dose as converted per 60 kg can be administered.

**[2] Screening of drug candidate compounds for disease**

The proteins of the present invention are useful as reagents for screening compounds or salts thereof that promote or inhibit the activities of the proteins of the present invention.

The present invention provides (1) a method of screening a compound or its salt that promotes or inhibits the activity (e.g., transport of sugars (e.g., glucose, fructose, galactose, etc.), etc.) of the protein A of the present invention (hereinafter sometimes merely referred to as the promoter or the inhibitor, respectively), which comprises using the protein A of the present invention. More specifically, the present invention provides, for example:

(2) a method of screening a promoter or inhibitor, which comprises comparing (i) the transport of sugars (e.g., glucose, fructose, galactose, etc.) in cells capable of producing the protein A of the present invention and (ii) the transport of sugars (e.g., glucose, fructose, galactose, etc.) in a mixture of cells capable of producing the protein A of the present invention and a test compound.

Specifically, the screening method described above is characterized by measuring accumulation of glucose analogs such as $^{3}$H-labeled glucose or 2-deoxy-glucose, etc. into cells by radioactivity in the cases of (i) and (ii) and comparing them in terms of the sugar uptake activity as an indicator.

The transport activity of sugars (e.g., glucose, fructose, galactose, etc.), etc. of the protein A of the present invention can be assayed by publicly known methods, e.g., by the method described in J. Biol. Chem., 275, 4607-4612, 2000, or its modifications.

For example, when a test compound promotes the transport of sugars (e.g., glucose, fructose, galactose, etc.) in the case (ii) described above by at least about 20%, preferably at least 30%, more preferably at least about 50%, as compared to the case (i) described above, the test compound can be selected to be a compound capable of promoting the activity of the protein A of the present invention, or a salt of the compound.

For example, when a test compound inhibits the transport of sugars (e.g., glucose, fructose, galactose, etc.) in the case (ii) described above by at least about 20%, preferably at least 30%, more preferably at least about 50%, as compared to the case (i) described above, the test compound can be selected to be a compound capable of inhibiting the activity of the protein A of the present invention, or a salt of the compound.

The present invention provides (1') a method of screening a compound or its salt that promotes or inhibits the activity (e.g., transport of glutamate, etc.) of the protein B of the present invention (hereinafter sometimes merely referred to as the promoter or the inhibitor, respectively), which comprises using the protein B of the present invention. More specifically, the present invention provides, for example: (2') a method of screening a promoter or inhibitor, which comprises comparing (i') the transport of glutamate in cells capable of producing the protein B of the present invention and (ii') the transport of glutamate in a mixture of cells capable of producing the protein B of the present invention and a test compound.

Specifically, the screening method described above is characterized by measuring, e.g., the transport of glutamate with a radioisotope-labeled substrate or a fluorescent dye in the cases of (i') and (ii') and comparing them in terms of the transport of glutamate as an indicator.

The glutamate transport activity, etc. of the protein B of the present invention can be determined by publicly known methods, e.g., by the method described in Nature, 407, 189-194, 2000, J. Biol. Chem., 276, 43400-43406, 2001, etc., or with modifications of these methods.

For example, when a test compound promotes the glutamate transport in the case (ii') described above by at least about 20%, preferably at least 30%, more preferably at least about 50%, as compared to the case (i') described above, the test compound can be selected to be a compound capable of promoting the activity of the protein B of the present invention, or a salt of the compound.

For example, when a test compound inhibits the glutamate transport in the case (ii') described above by at least about 20%, preferably at least 30%, more preferably at least about 50%, as compared to the case (i') described above, the test compound can be selected to be a compound capable of inhibiting the activity of the protein B of the present invention, or a salt of the compound.

The present invention provides (1") a method of screening a compound or its salt that promotes or inhibits the activity (e.g., permeation of $K^+$ ions, etc.) of the protein C of the present invention (hereinafter sometimes merely referred to as the promoter or the inhibitor, respectively), which comprises using the protein C of the present invention. More specifically, the present invention provides, for example:

(2") a method of screening a promoter or inhibitor, which comprises comparing (i") the $K^+$ ion permeation in cells capable of producing the protein C of the present invention and (ii") the transport of glutamate in a mixture of cells capable of producing the protein C of the present invention and a test compound.

Specifically, the screening method described above is characterized by measuring, e.g., change in membrane potentials accompanied by the permeation of $K^+$ ions with a fluorescent dye in the cases of (i") and (ii") and comparing them as an indicator of the $K^+$ ion permeation.

The $K^+$ ion permeation activity, etc. of the protein C of the present invention can be determined by publicly known methods, e.g., by the method described in Receptors and Channels, 6, 1337-350, 1999, etc., or with modifications of these methods.

For example, when a test compound promotes the permeation of $K^+$ ions in the case (ii") described above by at least about 20%, preferably at least 30%, more preferably at least about 50%, as compared to the case (i") described above, the test compound can be selected to be a compound capable of regulating (promoting or inhibiting) the activity of the protein C of the present invention, or a salt of the compound.

For example, when a test compound inhibits the permeation of $K^+$ ions in the case (ii") described above by at least about 20%, preferably at least 30%, more preferably at least about 50%, as compared to the case (i") described above, the test compound can be selected to be a compound capable of regulating (promoting or inhibiting) the activity of the protein C of the present invention, or a salt of the compound.

Examples of the test compound include peptides, proteins, non-peptide compounds, synthetic compounds, fermentation products, cell extracts, plant extracts, animal tissue extracts, etc. These compounds may be novel compounds or publicly known compounds.

To perform the screening methods described above, the cells capable of producing the protein of the present invention are suspended in a buffer suitable for the screening to prepare the cell suspension. Any buffer is usable as far as it is a buffer such as a phosphate buffer, borate buffer, etc. having pH of approximately 4 to 10 (preferably pH of about 6 to about 8) that does not interfere the transport of sugars (e.g., glucose, fructose, galactose, etc.) of the protein of the present invention.

As the cells capable of producing the protein of the present invention, there is employed, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the membrane such as endoplasmic reticulum membrane, Golgi membrane, cell membrane, etc., for example, by culturing through the procedure described above, is preferably employed.

Furthermore, the compound or its salt that promotes or inhibits the expression of the protein of the present invention (i.e., promotes or inhibits the activity of the protein of the present invention) can also be screened by inserting a gene for secreted alkaline phosphatase, luciferase, etc. at the downstream of a promoter for a gene of the protein of the present invention, expressing the gene in the various cells described above, and investigating such a compound or its salt that activates or inhibits the enzyme activity when the test compound described above is brought in contact with the cells.

The polynucleotide encoding the protein of the present invention is useful as a reagent for screening the compound or its salt that promotes or inhibits a gene for the protein of the present invention.

The present invention provides (3) a method of screening a compound or its salt that promotes or inhibits the expression of a gene for the protein of the present invention (hereinafter sometimes merely referred to as the promoter or the inhibitor, respectively), which comprises using the polynucleotide encoding the protein of the present invention. More specifically, the present invention provides, for example: (4) a method of screening the promoter or the inhibitor, which comprises comparing (iii) the case where cells capable of producing the protein of the present invention are cultured and (iv) the case where a mixture of cells capable of producing the protein of the present invention and a test compound is cultured.

In the screening method described above, for example, the expression level of the gene for the protein of the present invention (specifically, the level of the protein of the present invention or the level of mRNA encoding the aforesaid protein) is measured in the cases (iii) and (iv) and comparison is made therebetween.

Examples of the test compound include peptides, proteins, non-peptide compounds, synthetic compounds, fermentation products, cell extracts, plant extracts, animal tissue extracts, etc. These compounds may be novel compounds or publicly known compounds.

To perform the screening methods described above, the cells capable of producing the protein of the present invention are suspended in a buffer suitable for the screening to prepare the cell suspension. Any buffer is usable as far as it is a buffer such as a phosphate buffer, borate buffer, etc. having pH of approximately 4 to 10 (preferably pH of about 6 to about 8) that does not interfere expression of the protein of the present invention.

As the cells capable of producing the protein of the present invention, there is employed, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the membrane such as endoplasmic reticulum membrane, Golgi membrane, cell membrane, etc., for example, by culturing through the procedure described above, is preferably employed.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The expression level of the gene for the protein of the present invention can be determined by publicly known methods, e.g., in accordance with methods including Northern blotting, reverse transcription-polymerase chain reaction (RT-PCR), real time PCR monitoring system (manufactured by ABI, TaqMan polymerase chain reaction), etc., or their modifications.

For example, when a test compound promotes the expression level of the gene for the protein of the present invention in the case (iv) described above by at least about 20%, preferably at least 30%, more preferably at least about 50%, as compared to the case (iii) described above, the test compound can be selected to be a compound capable of promoting the expression of the gene for the protein of the present invention, or a salt of the compound.

For example, when a test compound inhibits the expression level of the gene for the protein of the present invention in the case (iv) described above by at least about 20%, preferably at least 30%, more preferably at least about 50%, as compared to the case (iii) described above, the test compound can be selected to be a compound capable of inhibiting the expression of the gene for the protein of the present invention, or a salt of the compound.

In addition, the antibody of the present invention is useful as a reagent for screening the compound or its salt that promotes or inhibits expression of the protein of the present invention.

The present invention provides (5) a method of screening a compound or its salt that promotes or inhibits expression of the protein of the present invention (hereinafter sometimes merely referred to as the promoter or the inhibitor, respectively), which comprises using the antibody of the present invention. More specifically, the present invention provides, for example:

(6) a method of screening the promoter or the inhibitor, which comprises comparing (v) the case where cells capable of producing the protein of the present invention are cultured and (vi) the case where a mixture of cells capable of producing the protein of the present invention and a test compound is cultured.

In the screening method described above, for example, the expression level of the protein of the present invention (specifically, the level of the protein of the present invention) is determined (e.g., detection of expression of the protein of the present invention, quantification of the expression level of the protein of the present invention, etc.) in the cases (v) and (vi) using the antibody of the present invention, and comparison is made therebetween.

For the test compound, for example, peptides, proteins, non-peptide compounds, synthetic compounds, fermentation products, cell extracts, plant extracts, animal tissue extracts, and the like are used. These compounds may be novel or known compounds.

To perform the screening method described above, cells capable of producing the protein of the present invention are suspended in a buffer suitable for the screening to prepare the cell suspension. Any buffer is usable so long as it is a buffer such as a phosphate buffer, borate buffer, etc. having pH of approximately 4 to 10 (preferably pH of about 6 to about 8), which does not interfere the expression of the protein of the present invention.

As the cells capable of producing the protein of the present invention, there is used, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the membrane such as endoplasmic reticulum membrane, Golgi membrane, cell membrane, etc., for example, by culturing through the procedure described above, is preferably employed.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

For example, when a test compound promotes the expression level of the protein of the present invention in the case (vi) described above by at least about 20%, preferably at least 30%, more preferably at least about 50%, as compared to the case (v) described above, the test compound can be selected to be a compound capable of promoting the expression of the protein of the present invention, or a salt of the compound.

For example, when a test compound inhibits the expression level of the protein of the present invention in the case (vi) described above by at least about 20%, preferably at least 30%, more preferably at least about 50%, as compared to the case (v) described above, the test compound can be selected to be a compound capable of inhibiting the expression of the protein of the present invention, or a salt of the compound.

The screening kit of the present invention comprises the protein or partial peptide used in the present invention or a salt thereof, or the cell capable of producing the protein used in the present invention or its partial peptide.

The compound or its salt obtained using the screening method or screening kit of the present invention is the test compound described above, the compound selected from, for example, peptides, proteins, non-peptide compounds, synthetic compounds, fermentation products, cell extracts, plant extracts, animal tissue extracts, plasma, etc., which is a compound or its salt that promotes or inhibits the activity of the protein of the present invention.

The salts of these compounds used are those given above as the salts of the protein of the present invention.

(i) The compound or its salt that promotes the activity of the protein A of the present invention, (ii) the compound or its salt that promotes the expression of the gene for the protein A of the present invention, and (iii) the compound or its salt that promotes the expression of the protein A of the present invention are useful as low toxic and safe pharmaceuticals such as agents for the prevention/treatment of, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of diabetes mellitus, hyperlipemia, arteriosclerosis, etc.

(i) The compound or its salt that inhibits the activity of the protein A of the present invention, (ii) the compound or its salt that inhibits the expression of the gene for the protein A of the present invention, and (iii) the compound or its salt that inhibits the expression of the protein A of the present invention are useful as low toxic and safe pharmaceuticals such as agents for the prevention/treatment of, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g:, infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of diabetes mellitus, hyperlipemia, arteriosclerosis, etc.

(i) The compound or its salt that promotes the activity of the protein B of the present invention, (ii) the compound or its salt that promotes the expression of the gene for the protein B of the present invention, and (iii) the compound or its salt that promotes the expression of the protein B of the present invention are useful as low toxic and safe pharmaceuticals such as agents for the prevention/treatment of, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant; etc. Preferred are agents for the prevention/treatment of central nervous system disorders, endocrine disorders, diabetes mellitus, etc.

(i) The compound or its salt that inhibits the activity of the protein B of the present invention, (ii) the compound or its salt that inhibits the expression of the gene for the protein B of the present invention, and (iii) the compound or its salt that inhibits the expression of the protein B of the present invention are useful as low toxic and safe pharmaceuticals, including agents for the prevention/treatment of, for example, central nervous system disorders (e.g., Alzheimer'. disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant; etc. Preferred are agents for the prevention/treatment of central nervous system disorders, endocrine disorders, diabetes mellitus, etc.

(i) The compound or its salt that regulates (e.g., promotes) the activity of the protein C of the present invention, (ii) the compound or its salt that regulates (e.g., promotes) the expression of the gene for the protein C of the present invention, and (iii) the compound or its salt that regulates (e.g., promotes) the expression of the protein C of the present invention are useful as low toxic and safe pharmaceuticals such as agents for the prevention/treatment of, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

(i) The compound or its salt that regulates (e.g., inhibits) the activity of the protein C of the present invention, (ii) the compound or its salt that regulates (e.g., inhibits) the expression of the gene for the protein C of the present invention, and (iii) the compound or its salt that regulates (e.g., inhibits) the expression of the protein C of the present invention are useful as low toxic and safe pharmaceuticals such as agents for the prevention/treatment of, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

When the compounds or their salt forms obtained by the screening methods or screening kits of the present invention are used as agents for the prevention/treatment described above, pharmaceutical preparations can be prepared following the conventional methods. For example, the compounds can be prepared into tablets, capsules, elixir, microcapsules, aseptic solution, suspension, etc.

Since the thus obtained pharmaceutical preparation is safe and low toxic, the preparation can be administered orally or parenterally to human or warm-blooded animals (e.g., mice, rats, rabbits, sheep, swine, bovine, horses, fowl, cats, dogs, monkeys, chimpanzees, etc.).

The dose of the compound or its salt may vary depending upon target disease, subject to be administered, route of administration, etc. In oral administration of the compound or its salt that promotes the activity of the protein A of the present invention for the treatment of, e.g., diabetes mellitus, generally the compound or its salt is administered to an adult (as 60 kg body weight) in a dose of about 0.1 mg to 100 mg, preferably about 1.0 to 50 mg and more preferably about 1.0 to 20 mg per day. In parenteral administration, the single dose of the compound or its salt may vary depending on subject to be administered, target disease, etc. When the compound or its salt that promotes the activity of the protein A of the present invention is administered to an adult (as 60 kg body weight) in the form of injectable preparations for the treatment of, e.g., diabetes mellitus, it is advantageous to inject the compound or its salt intravenously in a daily dose of about 0.01 to about 30 mg, preferably about 0.1 to about 20 mg and more preferably about 0.1 to about 10 mg. For other animal species, the corresponding dose as converted per 60 kg can be administered.

In oral administration of the compound or its salt that promotes the activity of the protein B of the present invention for the treatment of, e.g., Alzheimer's disease, generally the compound or its salt is administered to an adult (as 60 kg body weight) in a dose of about 0.1 mg to 100 mg, preferably about 1.0 to 50 mg and more preferably about 1.0 to 20 mg per day. In parenteral administration, the single dose of the compound or its salt may vary depending on subject to be administered, target disease, etc. When the compound or its salt that promotes the activity of the protein B of the present invention is administered to an adult (as 60 kg body weight) in the form of injectable preparations for the treatment of, e.g., Alzheimer's disease, it is advantageous to inject the compound or its salt intravenously in a daily dose of about 0.01 to about 30 mg, preferably about 0.1 to about 20 mg and more preferably about 0.1 to about 10 mg. For other animal species, the corresponding dose as converted per 60 kg can be administered.

In oral administration of the compound or its salt that regulates (e.g., promotes) the activity of the protein C of the present invention for the treatment of, e.g., alimentary disease, generally the compound or its salt is administered to an adult (as 60 kg body weight) in a dose of about 0.1 mg to 100 mg, preferably about 1.0 to 50 mg and more preferably about 1.0 to 20 mg per day. In parenteral administration, the single dose of the compound or its salt may vary depending on subject to be administered, target disease, etc. When the compound or its salt that regulates (e.g., promotes) the activity of the protein C of the present invention is administered to an adult (as 60 kg body weight) in the form of injectable preparations for the treatment of, e.g., alimentary disease, it is advantageous to inject the compound or its salt intravenously in a daily dose of about 0.01 to about 30 mg, preferably about 0.1 to about 20 mg and more preferably about 0.1 to about 10 mg. For other animal species, the corresponding dose as converted per 60 kg can be administered.

**[3] Quantification for the protein of the present invention, it partial peptide or salts thereof**

The antibody of the present invention is capable of specifically recognizing the protein of the present invention, and thus can be used for quantification of the protein of the present invention in a test sample fluid, in particular, for quantification by sandwich immunoassay; etc.

That is, the present invention provides:

(i) a method for quantification of the protein of the present invention in a test sample fluid, which comprises competitively reacting the antibody of the present invention, a test sample fluid and a labeled form of the protein of the present invention, and r measuring the ratio of the labeled form of the protein of the present invention bound to the antibody; and,

(ii) a method for quantification of the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or $F(ab')_2$, Fab' or Fab fractions of the antibody molecule may also be used.

The method for quantification of the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [$^{125}$I], [$^{131}$I], [$^{3}$H], [$^{14}$C], etc. The enzymes described above are preferably enzymes, which are stable and have a high specific activity, and include, e.g., □-galactosidase, □-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F. separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity, using the antibody of the present invention.

Furthermore, when a decreased level of the protein A of the present invention is detected by quantifying the level of the protein of the present invention using the antibody of the present invention, it can be diagnosed that it is highly likely to suffer from diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. Further when an increased level of the protein A of the present invention is detected, it can be diagnosed that it is highly likely to suffer from diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like.

When a decreased level of the protein B of the present invention is detected, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. Further when an increased level of the protein B of the present invention is detected, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like.

When a decreased level of the protein C of the present invention is detected, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. When an increased level of the protein C of the present invention is detected, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e. g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.).

Moreover, the antibody of the present invention can be used to detect the protein of the present invention, which is present in a test sample fluid such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

## [4] Gene diagnostic agent

By using the DNA of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.) can be detected. Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When increased expression of the gene for the protein A of the present invention is detected, e.g., by the Northern hybridization, it can be diagnosed that it is highly likely to suffer from diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. When decreased expression of the gene is detected or DNA mutation is detected by the PCR-SSCP assay, it can be diagnosed that it is highly likely to suffer from diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like.

When increased expression of the gene for the protein B of the present invention is detected, e.g., by the Northern hybridization, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. When decreased expression of the gene is detected or DNA mutation is detected by the PCR-SSCP assay, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like.

When increased expression of the gene for the protein C of the present invention is detected, e.g., by the Northern hybridization, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. When decreased expression of the gene is detected or DNA mutation is detected by the PCR-SSCP assay, it can be diagnosed that it is highly likely to suffer from diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like.

### [5] Pharmaceutical comprising the antisense polynucleotide

The antisense polynucleotide of the present invention that binds to the DNA encoding the protein A of the present invention complementarily to inhibit expression of the DNA is low toxic and can suppress in vivo the functions (e.g., the transport of sugars (e.g., glucose, fructose, galactose, etc.), etc.) of the protein A of the present invention or the DNA described above. Thus, the antisense polynucleotide can be used as agents for the prevention/treatment of diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant. Preferred are agents for the prevention/treatment of diabetes mellitus, hyperlipemia, arteriosclerosis, etc.

The antisense polynucleotide of the present invention that binds to the DNA encoding the protein B of the present invention complementarily to suppress expression of the DNA is low toxic and can suppress in vivo the functions (e.g., the transport of glutamate, etc.) of the protein B of the present invention or the DNA described above. Thus, the antisense polynucleotide can be used as agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.

g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant; etc. Preferred are agents for the prevention/treatment of central nervous system disorders, endocrine disorders, diabetes mellitus, etc.

The antisense polynucleotide of the present invention that binds to the DNA encoding the protein C of the present invention complementarily to suppress expression of the DNA is low toxic and can regulate in vivo the functions (e.g., the $K^+$ ion permeation activity, etc.) of the protein C of the present invention or the DNA described above. Thus, the antisense polynucleotide can be used as agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

When the antisense polynucleotide is used as the agent for the prevention/treatment described above, it can be prepared into pharmaceutical preparations by publicly known methods, and the preparations are administered.

For example, when the antisense polynucleotide is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or other mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel.

The dose of the antisense polynucleotide may vary depending on target disease, subject to be administered, route for administration, etc. When the antisense polynucleotide of the present invention is administered to a particular digestive organ for the treatment of, e.g., diabetes mellitus, the antisense polynucleotide is generally administered to an adult (body weight of 60 kg) in a daily dose of about 0.1 to about 100 mg.

In addition, the antisense polynucleotide may also be used as an oligonucleotide probe for diagnosis to examine the presence of the DNA of the present invention in tissues or cells and states of its expression.

The present invention further provides:

(1) Double-stranded RNA containing a part of RNA encoding the protein of the present invention;
(2) A pharmaceutical comprising said double-stranded RNA;
(3) A ribozyme containing a part of RNA encoding the protein of the present invention;
(4) A pharmaceutical comprising said ribozyme; and
(5) An expression vector containing a gene (DNA) encoding said ribozyme; etc.

As the antisense polynucleotide described above can, the double-stranded RNA, ribozyme, etc. can also disrupt RNA transcribed from the DNA of the present invention or can suppress its functions to suppress the in vivo function of the protein A of the present invention or the DNA encoding the same, and thus can be used as agents for the prevention/treatment of, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g.,

myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of diabetes mellitus, hyperlipemia, arteriosclerosis, etc.

As the antisense polynucleotide described above can, the double-stranded RNA, ribozyme, etc. can also disrupt RNA transcribed from the DNA of the present invention or can suppress its functions to regulate the in vivo function of the protein C of the present invention or the DNA encoding the same, and thus can be used as agents for the prevention/treatment of, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant; etc. Preferred are agents for the prevention/treatment of central nervous system disorders, endocrine disorders, diabetes mellitus, etc.

As the antisense polynucleotide described above can, the double-stranded RNA, ribozyme, etc. can also disrupt RNA transcribed from the DNA of the present invention or can suppress its functions to suppress the in vivo function of the protein C of the present invention or the DNA encoding the same, and thus can be used as agents for the prevention/treatment of, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by replacing a part of the RNA encoding the protein of the present invention for a part of publicly known ribozyme. A part of the RNA encoding the protein of the present invention includes sequences near the consensus sequence NUX (wherein N shows all bases and X shows bases other than G), etc., which can be cleaved by a publicly known ribozyme.

Where the double-stranded RNA or ribozyme described above can be used as the agents for the prevention/treatment described above, they can be prepared into pharmaceutical preparations and the preparations can be provided for administration, as in the antisense polynucleotide. Also, the expression vector described in (5) above is used in a similar way to publicly known gene therapy, etc. and used as the agents for the prevention/treatment described above.

**[6] Preparation of animal bearing the DNA of the present invention**

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).
That is, the present invention provides:

(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.
Examples of the non-human mammal that can be used include bovine, swine, sheep, goat, rabbits, dogs, cats, guinea pigs, hamsters, mice, rats, etc. Above all, preferred are rodents, especially mice (e.g., C57BL/6 strain, DBA2 strain, etc. for a pure line and for a cross line, $B6C3F_1$ strain, $BDF_1$ strain $B6D2F_1$ strain, BALB/c strain, ICR strain, etc.), rats (Wistar, SD, etc.) or the like, since they are relatively short in ontogeny and life cycle from a standpoint of creating model animals for human disease.
"Mammals" in a recombinant vector that can be expressed in the mammals include the aforesaid non-human mammals and human.
The exogenous DNA of the present invention refers to the DNA of the present invention that is once isolated and extracted from mammals, not the DNA of the present invention inherently possessed by the non-human mammals.
The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.
The abnormal DNA is intended to mean DNA that expresses the abnormal protein of the present invention and exemplified by the DNA that expresses a protein for suppressing the function of the normal protein of the present invention, etc.
The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.
As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.
Examples of these promoters for regulating expression of the DNA described above include (1) promoters for DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal polypeptide obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening of an agent for the treatment of diseases associated with the protein of the present invention.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

A mammal bearing the abnormal exogenous DNA of the present invention is also expected to serve in screening a candidate drug for the treatment of the function inactive type inadaptability of the protein of the present invention, since a free form of the protein of the present invention is increased in such an animal.

Other potential applications of two kinds of the DNA transgenic animals of the present invention described above further include:

(1) Use as a cell source for tissue culture;
(2) Elucidation of the relation to a protein that is specifically expressed or activated by the protein of the present invention, by direct analysis of DNA or RNA in tissues of the DNA transgenic animal of the present invention or by analysis of the polypeptide tissues expressed by the DNA;
(3) Research on the function of cells derived from tissues that are usually cultured only with difficulty, using cells in tissues bearing the DNA cultured by a standard tissue culture technique;
(4) Screening a drug that enhances the functions of cells using the cells described in (3) above; and,
(5) Isolation and purification of the variant protein of the present invention and preparation of an antibody thereto; etc.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

**[7] Knockout animal**

The present invention provides a non-human mammal embryonic stem cell bearing the DNA of the present invention inactivated and a non-human mammal deficient in expressing the DNA of the present invention.

Thus, the present invention provides:

(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli*);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from *Escherichia coli)* therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the capability of the non-human mammal to express the DNA by artificially mutating the DNA of the present invention, or the DNA has no substantial capability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal used, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually disrupting the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, a first selection of ES cells at the early stage of culture can be based on sex identification, and male cells can be selected early, which saves a significant amount of time at the early stage of culture.

Also, a second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples supra apply.

With respect to the non-human mammal deficient in expression of the DNA of the present invention, the DNA of the present invention can be made knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyte, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is rendered knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and a plurality of homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

**[7a] Method of screening a compound having a therapeutic/preventive effect on diseases caused by deficiency, damages, etc. of the DNA of the present invention**

The non-human mammal deficient in expression of the DNA of the present invention can be employed for screening of a compound having a therapeutic/preventive effect on diseases caused by deficiency, damages, etc. of the DNA of the present invention.

That is, the present invention provides a method of screening a compound having a therapeutic/preventive effect on diseases caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed in the screening method, the same examples as given hereinabove apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately selected depending on the administration route, nature of the test compound, etc.

For screening of the compound having a therapeutic effect on, e.g., diabetes mellitus, the non-human mammal deficient in expression of the DNA encoding the protein A of the present invention is subjected to a glucose tolerance treatment. A test compound is given to the animal before or after the glucose tolerance treatment and changes in blood sugar level, urine output, urine sugar, body weight, and the like of the animal, etc. are measured with passage of time.

In screening a compound having a preventive/therapeutic effect on, e.g., Alzheimer's disease, a test compound is administered to the non-human mammal deficient in expression of the DNA encoding the protein B of the present invention to determine the level of cerebral amyloid β protein in the animal brain, observe senile plaques, determine the level of abnormally phosphorylated tau protein in the brain, observe neural degeneration, observe changes in effects on the learning ability of the animal, etc.

In screening a compound having a preventive/therapeutic effect on, e.g., irritable bowel syndrome, a test compound is administered to the non-human mammal deficient in expression of the DNA encoding the protein C of the present invention, and changes in the amount of evacuated stool caused by restraint stress of the animal are measured with passage of time.

The compound obtained using the above screening method is a compound selected from the test compounds described above and exhibits a preventive/therapeutic effect on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), particularly preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered to an adult (as 60 kg body weight), generally the compound is administered to the patient with, e.g., diabetes mellitus in a daily dose of about 0.1 to about 100 mg, preferably about 1.0 to about 50 mg and, more preferably about 1.0 to about 20 mg. In parenteral administration, a single dose of the compound may vary depending upon target subject, target disease, etc. When the compound is administered to an adult (as 60 kg) patient with diabetes mellitus in the form of an injectable preparation, it is advantageous to administer the compound intravenously to the patient in a daily dose of about 0.01 to about 30 mg, preferably about 0.1 to about 20 mg, and more preferably about 0.1 to about 10 mg. For other animal species, the corresponding dose as converted per 60 kg can be administered.

### [7b] Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting expression of the reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds used for the screening.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are screened from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., organic acids, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes the promoter activity to the DNA encoding the protein A of the present invention can promote expression of the protein A of the present invention to promote the activity/function of the protein. Thus, the compound or its salt is useful as pharmaceuticals such as .agents for the prevention/treatment of diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of diabetes mellitus, hyperlipemia, arteriosclerosis, etc.

The compound or its salt that promotes the promoter activity to the DNA encoding the protein B of the present invention can promote expression of the protein B of the present invention to promote the activity/function of the protein. Thus, the compound or its salt is useful as pharmaceuticals such as agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant; etc. Preferred are agents for the prevention/treatment of central nervous system disorders, endocrine disorders, diabetes mellitus, etc.

The compound or its salt that promotes the promoter activity to the DNA encoding the protein C of the present invention can promote expression of the protein C of the present invention to promote the activity/function of the protein. Thus, the compound or its salt is useful as pharmaceuticals such as agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

The compound or its salt that inhibits the promoter activity to the DNA encoding the protein A of the present invention can inhibit expression of the protein A of the present invention to inhibit the activity/function of the protein. Thus, the compound or its salt is useful as pharmaceuticals such as agents for the prevention/treatment of diseases, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of diabetes mellitus, hyperlipemia, arteriosclerosis, etc.

The compound or its salt that inhibits the promoter activity to the DNA encoding the protein B of the present invention can inhibit expression of the protein B of the present invention to inhibit the activity/function of the protein. Thus, the compound or its salt is useful as pharmaceuticals such as agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; or anti-rejection drugs after organ transplant; etc. Preferred are agents for the prevention/treatment of central nervous system disorders, endocrine disorders, diabetes mellitus, etc.

The compound or its salt that inhibits the promoter activity to the DNA encoding the protein C of the present invention can inhibit expression of the protein C of the present invention to inhibit the activity/function of the protein. Thus, the compound or its salt is useful as pharmaceuticals such as agents for the prevention/treatment of diseases, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc. Preferred are agents for the prevention/treatment of central nervous system disorders, alimentary disorders, etc.

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention or its salts described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; when the compound that promotes the promoter activity to the DNA of the present invention is orally administered to an adult (as 60 kg body weight), the compound is administered to the patient with diabetes mellitus normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of promoting the promoter activity to the DNA of the present invention is administered to an adult (as 60 kg) in the form of injectable preparation, it is advantageous to administer the compound intravenously to the patient with diabetes mellitus in a daily dose of about 0.01 to about 30 mg, preferably about 0.1 to about 20 mg and more preferably about 0.1 to about 10 mg. For other animal species, the corresponding dose as converted per 60 kg can be administered.

On the other hand, when the compound that inhibits the promoter activity to the DNA of the present invention is orally administered an adult (as 60 kg body weight), the compound is administered to the patient with diabetes mellitus normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to an adult (as 60 kg) in the form of injectable preparation, it is advantageous to administer the compound intravenously to the patient with diabetes mellitus in a daily dose of about 0.01 to about 30 mg, preferably about 0.1 to about 20 mg and more preferably about 0.1 to about 10 mg. For other animal species, the corresponding dose as converted per 60 kg can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of preventive/therapeutic agent for these diseases.

Also, a so-called transgenic animal (gene transferred animal) can be prepared by using a DNA containing the promoter region of the DNA of the present invention, ligating genes encoding various proteins at the downstream and injecting the same into oocyte of an animal. It is thus possible to synthesize the polypeptide therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein of the present invention itself.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| Gly : | glycine |
| Ala : | alanine |
| Val : | valine |
| Leu : | leucine |
| Ile : | isoleucine |
| Ser : | serine |
| Thr : | threonine |
| Cys : | cysteine |
| Met : | methionine |
| Glu : | glutamic acid |
| Asp : | aspartic acid |
| Lys : | lysine |
| Arg : | arginine |
| His : | histidine |
| Phe : | phenylalanine |

Tyr : tyrosine
Trp : tryptophan
Pro : proline
Asn : asparagine
Gln : glutamine
pGlu : pyroglutamic acid

Substituents, protecting groups and reagents generally used in this specification are presented as the codes below.

Me : methyl group
Et : ethyl group
Bu : butyl group
Ph : phenyl group
TC : thiazolidine-4(R)-carboxamido group
Tos : p-toluenesulfonyl
CHO : formyl
Bzl : benzyl
$Cl_2$-Bzl : 2,6-dichlorobenzyl
Bom : benzyloxymethyl
Z : benzyloxycarbonyl
Cl-Z : 2-chlorobenzyloxycarbonyl
Br-Z : 2-bromobenzyl oxycarbonyl
Boc : t-butoxycarbonyl
DNP : dinitrophenol
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboxyimide
DCC : N,N'-dicyclohexylcarbodiimide

The sequence identification numbers in the sequence listing of the specification indicates the following sequence, respectively.

[SEQ ID NO: 1] This shows the amino acid sequence of human TCH099 protein acquired in EXAMPLE 1.
[SEQ ID NO: 2] This shows the base sequence of DNA encoding human TCH099 having the amino acid sequence represented by SEQ ID NO: 1.
[SEQ ID NO: 3] This shows the base sequence of primer A6 used in EXAMPLE 1.
[SEQ ID NO: 4] This shows the base sequence of primer B 16 used in EXAMPLE 1.
[SEQ ID NO: 5] This shows the base sequence of primer B6 used in EXAMPLE 1.
[SEQ ID NO: 6] This shows the base sequence of primer SP6 used in EXAMPLE 1.
[SEQ ID NO: 7] This shows the base sequence of primer T7 used in EXAMPLE 1 and EXAMPLE 12.
[SEQ ID NO: 8] This shows the base sequence of primer A2 used in EXAMPLE 1 and EXAMPLE 12.
[SEQ ID NO: 9] This shows the base sequence of primer A4 used in EXAMPLE 1 and EXAMPLE 12.
[SEQ ID NO: 10] This shows the base sequence of primer F1 used in EXAMPLE 1 and EXAMPLE 12.
[SEQ ID NO: 11] This shows the base sequence of primer B 1 used in EXAMPLE 1 and EXAMPLE 12.
[SEQ ID NO: 12] This shows the base sequence of primer R1 used in EXAMPLE 1 and EXAMPLE 12.
[SEQ ID NO: 13] This shows the base sequence of primer B3 used in EXAMPLE 1 and EXAMPLE 12.
[SEQ ID NO: 14] This shows the base sequence of cDNA containing the human TCH099 full-length gene acquired in EXAMPLE 1.
[SEQ ID NO: 15] This shows the base sequence of primer B8 used in EXAMPLE 2.
[SEQ ID NO: 16] This shows the base sequence of primer B9 used in EXAMPLE 2.
[SEQ ID NO: 17] This shows the base sequence of primer B11 used in EXAMPLE 2.
[SEQ ID NO: 18] This shows the base sequence of primer B12 used in EXAMPLE 2.
[SEQ ID NO: 19] This shows the base sequence of primer AP1 used in EXAMPLE 2.
[SEQ ID NO: 20] This shows the base sequence of primer AP2 used in EXAMPLE 2.

[SEQ ID NO: 21] This shows the base sequence of the RACE product acquired in EXAMPLE 2 using Set 2.
[SEQ ID NO: 22] This shows the amino acid sequence of the partial peptide encoded by SEQ ID NO: 21.
[SEQ ID NO: 23] This shows the amino acid sequence of human TCH099V acquired in EXAMPLE 2.
[SEQ ID NO: 24] This shows the base sequence encoding human TCH099V protein having the amino acid sequence represented by SEQ ID NO: 23.
[SEQ ID NO: 25] This shows the base sequence of primer A13 used in EXAMPLES 3, 14 and 23.
[SEQ ID NO: 26] This shows the base sequence of primer B18 used in EXAMPLES 14 and 23.
[SEQ ID NO: 27] This shows the base sequence of TaqMan probe T1 used in EXAMPLES 3, 14 and 23.
[SEQ ID NO: 28] This shows the amino acid sequence of human TCH177 protein of 589 amino acids acquired in EXAMPLE 4.
[SEQ ID NO: 29] This shows the base sequence of DNA encoding human TCH177 protein containing the amino acid sequence represented by SEQ ID NO: 28.
[SEQ ID NO: 30] This shows the base sequence of primer TF used in EXAMPLES 5, 20 and 23.
[SEQ ID NO: 31] This shows the base sequence of primer TR used in EXAMPLES 5, 20 and 23.
[SEQ ID NO: 32] This shows the base sequence of TaqMan probe T1 used in EXAMPLES 5, 20 and 23.
[SEQ ID NO: 33] This shows the base sequence of primer A3 used in EXAMPLE 4.
[SEQ ID NO: 34] This shows the base sequence of primer B3 used in EXAMPLE 4.
[SEQ ID NO: 35] This shows the base sequence of primer A4 used in EXAMPLE 4.
[SEQ ID NO: 36] This shows the base sequence of primer SP6 used in EXAMPLE 4.
[SEQ ID NO: 37] This shows the base sequence of primer T7 used in EXAMPLES 4 and 18.
[SEQ ID NO: 38] This shows the base sequence of primer A1 used in EXAMPLES 4 and 18.
[SEQ ID NO: 39] This shows the base sequence of primer B2 used in EXAMPLES 4 and 18.
[SEQ ID NO: 40] This shows the base sequence of primer F1 used in EXAMPLES 4 and 18.
[SEQ ID NO: 41] This shows the base sequence of primer R1 used in EXAMPLES 4 and 18.
[SEQ ID NO: 42] This shows the base sequence of primer R2 used in EXAMPLE 4.
[SEQ ID NO: 43] This shows the base sequence of primer 5-2 used in EXAMPLE 4.
[SEQ ID NO: 44] This shows the base sequence of primer 3-2 used in EXAMPLE 4.
[SEQ ID NO: 45] This shows the base sequence of cDNA containing the human TCH177 full-length gene acquired in EXAMPLE 4.
[SEQ ID NO: 46] This shows the amino acid sequence of mouse TCH177 protein of 601 amino acids acquired in EXAMPLE 6.
[SEQ ID NO: 47] This shows the base sequence of DNA encoding mouse TCH177 protein having the amino acid sequence represented by SEQ ID NO: 46.
[SEQ ID NO: 48] This shows the base sequence of primer mOF used in EXAMPLE 6.
[SEQ ID NO: 49] This shows the base sequence of primer mOR used in EXAMPLE 6.
[SEQ ID NO: 50] This shows the base sequence of primer mOF1 used in EXAMPLE 6.
[SEQ ID NO: 51] This shows the base sequence of primer mOR1 used in EXAMPLE 6.
[SEQ ID NO: 52] This shows the base sequence of primer mA1 used in EXAMPLES 6 and 16.
[SEQ ID NO: 53] This shows the base sequence of primer mB1 used in EXAMPLES 6 and 16.
[SEQ ID NO: 54] This shows the base sequence of primer mF1 used in EXAMPLE 6.
[SEQ ID NO: 55] This shows the base sequence of primer mF2 used in EXAMPLE 6.
[SEQ ID NO: 56] This shows the base sequence of primer mR1 used in EXAMPLE 6.
[SEQ ID NO: 57] This shows the base sequence of primer mR2 used in EXAMPLE 6.
[SEQ ID NO: 58] This shows the base sequence of cDNA containing the mouse TCH177 full-length gene acquired in EXAMPLE 6.
[SEQ ID NO: 59] This shows the base sequence of primer mTG used in EXAMPLES 7 and 15.
[SEQ ID NO: 60] This shows the base sequence of primer mTR used in EXAMPLES 7 and 15.
[SEQ ID NO: 61] This shows the base sequence of primer TaqMan probe mT1 used in EXAMPLES 7 and 15.
[SEQ ID NO: 62] This shows the amino acid sequence of human TCH136 protein acquired in EXAMPLE 8.
[SEQ ID NO: 63] This shows the base sequence of DNA encoding TCH136 protein having the amino acid sequence represented by SEQ ID NO: 62.
[SEQ ID NO: 64] This shows the base sequence of primer SP6 used in EXAMPLE 8.
[SEQ ID NO: 65] This shows the base sequence of primer T7 used in EXAMPLE 8.
[SEQ ID NO: 66] This shows the base sequence of primer A1 used in EXAMPLE 8.
[SEQ ID NO: 67] This shows the base sequence of primer A2 used in EXAMPLES 8 and 9.
[SEQ ID NO: 68] This shows the base sequence of primer B 1 used in EXAMPLE 8.
[SEQ ID NO: 69] This shows the base sequence of primer F1 used in EXAMPLE 8.
[SEQ ID NO: 70] This shows the base sequence of primer F2 used in EXAMPLE 8.

[SEQ ID NO: 71] This shows the base sequence of primer F3 used in EXAMPLE 8.
[SEQ ID NO: 72] This shows the base sequence of primer F4 used in EXAMPLE 8.
[SEQ ID NO: 73] This shows the base sequence of primer F5 used in EXAMPLE 8.
[SEQ ID NO: 74] This shows the base sequence of primer F6 used in EXAMPLE 8.
[SEQ ID NO: 75] This shows the base sequence of primer F7 used in EXAMPLE 8.
[SEQ ID NO: 76] This shows the base sequence of primer R1 used in EXAMPLE 8.
[SEQ ID NO: 77] This shows the base sequence of primer R2 used in EXAMPLE 8.
[SEQ ID NO: 78] This shows the base sequence of primer R3 used in EXAMPLE 8.
[SEQ ID NO: 79] This shows the base sequence of primer R4 used in EXAMPLE 8.
[SEQ ID NO: 80] This shows the base sequence of primer R5 used in EXAMPLE 8.
[SEQ ID NO: 81] This shows the base sequence of primer R6 used in EXAMPLE 8.
[SEQ ID NO: 82] This shows the base sequence of primer R7 used in EXAMPLE 8.
[SEQ ID NO: 83] This shows the base sequence of cDNA containing the human TCH136 full-length gene acquired in EXAMPLE 8.
[SEQ ID NO: 84] This shows the base sequence of primer TF used in EXAMPLES 9 and 23.
[SEQ ID NO: 85] This shows the base sequence of primer TR used in EXAMPLES 9 and 23.
[SEQ ID NO: 86] This shows the base sequence of TaqMan prove T1 used in EXAMPLES 9 and 23.
[SEQ ID NO: 87] This shows the base sequence of primer B3 used in EXAMPLE 9.
[SEQ ID NO: 88] This shows the base sequence of primer m099A1 used in EXAMPLE 10.
[SEQ ID NO: 89] This shows the base sequence of primer m099B1 used in EXAMPLE 10.
[SEQ ID NO: 90] This shows the base sequence of a partial sequence of the mouse TCH099 gene identified in EXAMPLE 10.
[SEQ ID NO: 91] This shows the base sequence of primer m099A2 used in EXAMPLE 11.
[SEQ ID NO: 92] This shows the base sequence of primer m099B2 used in EXAMPLE 11.
[SEQ ID NO: 93] This shows the base sequence of TaqMan probe m099T1 used in EXAMPLE 11.
[SEQ ID NO: 94] This shows the base sequence of primer A14d used in EXAMPLE 12.
[SEQ ID NO: 95] This shows the base sequence of primer B19S used in EXAMPLE 12.
[SEQ ID NO: 96] This shows the base sequence of primer BGH RV used in EXAMPLE 12.
[SEQ ID NO: 97] This shows the base sequence of a partial sequence of the rat TCH177 gene identified in EXAMPLE 16.
[SEQ ID NO: 98] This shows the base sequence of primer rTF used in EXAMPLE 17.
[SEQ ID NO: 99] This shows the base sequence of primer rTR used in EXAMPLE 17.
[SEQ ID NO: 100] This shows the base sequence of TaqMan probe rTl used in EXAMPLE 17.
[SEQ ID NO: 101] This shows the base sequence of primer OF used in EXAMPLE 18.
[SEQ ID NO: 102] This shows the base sequence of primer OR used in EXAMPLE 18.
[SEQ ID NO: 103] This shows the base sequence of primer mA1 used in EXAMPLE 21.
[SEQ ID NO: 104] This shows the base sequence of primer mB1 used in EXAMPLE 21.
[SEQ ID NO: 105] This shows the base sequence of a partial sequence of cDNA of the mouse TCH136 gene identified in EXAMPLE 21.
[SEQ ID NO: 106] This shows the base sequence of primer mTF used in EXAMPLE 22.
[SEQ ID NO: 107] This shows the base sequence of primer mTR used in EXAMPLE 22.
[SEQ ID NO: 108] This shows the base sequence of TaqMan probe mT1 used in EXAMPLE 22.

The transformant, Escherichia coli TOP10/pCR-BluntII-TCH099 obtained in EXAMPLE 1 later described has been on deposit since January 25, 2002 under the Accession Number PERM BP-7863 at the National Institute of Advanced Industrial Science and Technology, International Patent Organism Depositary, located at Central 6, 1-1-1 Higashi, Tsukuba-shi, Ibaraki, Japan (postal code 305-8566), and since January 10, 2002 has been on deposit at the Institute for Fermentation (IFO), located at 2-17-85, Juso-honmachi, Yodogawa-ku, Osaka-shi, Osaka, Japan (postal code 532-8686) under the Accession Number IFO 16744.

The transformant, Escherichia coli TOP10/pCR-BluntII-TCH177 obtained in EXAMPLE 4 later described has been on deposit since February 4, 2002 under the Accession Number FERM BP-7873 at the National Institute of Advanced Industrial Science and Technology, International Patent Organism Depositary, located at Central 6, 1-1-1 Higashi, Tsukuba-shi, Ibaraki, Japan (postal code 305-8566), and since December 11, 2001 has been on deposit at the Institute for Fermentation (IFO), located at 2-17-85, Juso-honmachi, Yodogawa-ku, Osaka-shi, Osaka, Japan (postal code 532-8686) under the Accession Number IFO 16737.

The transformant, Escherichia coli TOP10/pCR-BluntII-mTCH177 obtained in EXAMPLE 6 later described has been on deposit since May 27, 2002 under the Accession Number FERM BP-8058 at the National Institute of Advanced Industrial Science and Technology, International Patent Organism Depositary, located at Central 6, 1-1-1 Higashi, Tsukuba-shi, Ibaraki, Japan (postal code 305-8566), and since May 14, 2002 has been on deposit at the Institute for Fermentation (IFO), located at 2-17-85, Juso-honmachi, Yodogawa-ku, Osaka-shi, Osaka, Japan (postal code 532-8686) under the Accession Number IFO 16799.

The transformant, Escherichia coli DH10B/pBluescriptR-TCH136 obtained in EXAMPLE 8 later described has been on deposit since December 19, 2001 under the Accession Number FERM BP-7833 at the National Institute of Advanced Industrial Science and Technology, International Patent Organism Depositary, located at Central 6, 1-1-1 Higashi, Tsukuba-shi, Ibaraki, Japan (postal code 305-8566), and since November 27, 2001 has been on deposit at the Institute for Fermentation (IFO), located at 2-17-85, Juso-horunachi, Yodogawa-ku, Osaka-shi, Osaka, Japan (postal code 532-8686) under the Accession Number IFO 16735.

Hereinafter the present invention is described in more detail with reference to EXAMPLES, but is not deemed to limit the scope of the present invention thereto. The gene manipulation procedures using Escherichia coli were performed according to the methods described in the Molecular Cloning.

## EXAMPLE 1

Cloning of the human TCH099 gene cDNA

Using two primer DNAs, primer A6 (SEQ ID NO: 3) and primer B16 (SEQ ID NO: 4), primary PCR was carried out on human small intestine Marathon-Ready cDNA (manufactured by Clontech) under the following conditions (1) to (5), using Advantage 2 DNA Polymerase (manufactured by Clontech).

(1) 94°C for 3 minutes
(2) 5 cycles of one set to include 94°C for 5 seconds - 72°C for 3 minutes
(3) 5 cycles of one set to include 94°C for 5 seconds - 70°C for 3 minutes
(4) 5 cycles of one set to include 94°C for 5 seconds - 68°C for 3 minutes
(5) 70°C for 10 minutes

Further using this primary PCR product as a template together with primer A6 (SEQ ID NO: 3) and primer B6 (SEQ ID NO: 5), nested PCR was carried out under the following conditions (6) to (10) using Pyrobest DNA polymerase (manufactured by Takara Shuzo Co., Ltd.).

(6) 94°C for 2 minutes
(7) 5 cycles of one set to include 94°C for 5 seconds - 72°C for 4 minutes
(8) 5 cycles of one set to include 94°C for 5 seconds - 70°C for 4 minutes
(9) 25 cycles of one set to include 94°C for 5 seconds - 68°C for 4 minutes
(10) 72°C for 10 minutes

The amplified product obtained was cloned using Zero Blunt TOPO Cloning kit (manufactured by Invitrogen) to obtain plasmid pCR-BluntII-TCH099.

The plasmid was reacted using primer DNAs [primer SP6 (SEQ ID NO: 6), primer T7 (SEQ ID NO: 7), primer A2 (SEQ ID NO: 8), primer A4 (SEQ ID NO: 9), primer F1 (SEQ ID NO: 10), primer B1 (SEQ ID NO: 11), primer R1 (SEQ ID NO: 12), primer B3 (SEQ ID NO: 13)] and BigDye Terminator Cycle Sequencing Kit (manufactured by Applied Biosystems, Inc.), and the base sequence of cDNA fragment inserted was determined using a DNA sequencer, ABI PRISM 3100 DNA ANALYZER (manufactured by Applied Biosystems, Inc.). As a result, the plasmid acquired was identified to have the sequence of 1652 bases (SEQ ID NO: 14). The sequence of 512 amino acids (SEQ ID NO: 1) encoded the cDNA fragment (SEQ ID NO: 2) and the protein containing the amino acid sequence was named human TCH099 protein.

The transformant bearing the plasmid containing the cDNA fragment was named (Escherichia coli TOP10/pCR-BluntII-TCH099.

Using Blast P [Nucleic Acids Res., 25, 3389, 1997], homology search was conducted on OWL database and the cDNA was found to be a novel gene belonging to the glucose transporter family (FIG. 1). The cDNA showed 59% homology on an amino acid level to GLUT5 (J. Biol. Chem., 265, 13276, 1990), which was the glucose transporter reported in human. The protein was thus predicted to have a structure of 12 transmembrane type.

## EXAMPLE 2

Acquisition of the 5' end of human TCH099 gene cDNA

Using the primers in TABLE 1 for primary RACE and primer AP1 (SEQ ID NO: 19), primary RACE was carried out on human small intestine Marathon-Ready cDNA (manufactured by Clontech) under the following conditions (1) to (5), using Advantage 2 DNA Polymerase (manufactured by Clontech).

[TABLE 1]

| Sets of the primers used for RACE | | |
|---|---|---|
| | Primer for primary RACE | Primer for secondary RACE |
| Set 1 | B9 (SEQ ID NO: 16) | B8 (SEQ ID NO: 15) |
| Set 2 | B11 (SEQ ID NO: 17) | B8 (SEQ ID NO: 15) |
| Set 3 | B11 (SEQ ID NO: 17) | B9 (SEQ ID NO: 16) |
| Set 4 | B12 (SEQ ID NO: 18) | B8 (SEQ ID NO: 15) |
| Set 5 | B12 (SEQ ID NO: 18) | B9 (SEQ ID NO: 16) |

(1) 94°C for 3 minutes
(2) 5 cycles of one set to include 94°C for 5 seconds - 72°C for 1 minute
(3) 5 cycles of one set to include 94°C for 5 seconds - 70°C for 1 minute
(4) 25 cycles of one set to include 94°C for 5 seconds - 68°C for 1 minute
(5) 70°C for 10 minutes

Furthermore, secondary RACE was carried out as in the primary RACE, except that this primary RACE product was used as a template together with the primers in TABLE 1 for the secondary RACE and primer AP2 (SEQ ID NO: 20). After the RACE products were subjected to gel electrophoresis, the major bands were purified and the base sequences were determined. As a result, the products obtained from Set 1, Set 4 and Set 5 contained the 5' end of SEQ ID NO: 14, whereas the products obtained from Set 2 and Set 3 were both derived from the same exon, distinct from SEQ ID NO: 14. The sequence obtained using Set 2 is shown by SEQ ID NO: 21 and the amino acid sequence of the partial peptide encoded therein is shown by SEQ ID NO: 22. Since the 3' end of SEQ ID NO: 21 is common to that of SEQ ID NO: 14, the presence of a variant having the 5' end different from the cDNA obtained in EXAMPLE 1 was predicted. The sequence of 521 amino acids (SEQ ID NO: 23) encoded the variant (SEQ ID NO: 24), and the protein containing the amino acid sequence was named human TCH099V protein (FIG 1).

Using Blast N [Nucleic Acids Res., 25, 3389, 1997], the base sequences represented by SEQ ID NO: 14 and SEQ ID NO: 21 were subjected to homology search on the publicly known genome database (htgs). It was revealed that both SEQ ID NO: 14 and SEQ ID NO: 21 were on the first chromosome, and the first exon of SEQ ID NO: 21 was located between the first and second exons of SEQ ID NO: 14.

## EXAMPLE 3

Analysis of the human TCH099 gene product on tissue distribution

Using two primer DNAs, i.e., primer A13 (SEQ ID NO: 24) and primer B18 (SEQ ID NO: 25), which were designed based on the sequence of human TCH099, and TaqMan probe T1 (SEQ ID NO: 26), the expression level of human TCH099 in cDNAs from the respective tissues in human was assayed by TaqMan PCR.

Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.). The cDNAs from the respective tissues in human, which were used for the assay, are shown in TABLE 2.

[TABLE 2]

| cDNA (all manufactured by Clontech) | Tissue |
|---|---|
| Human MTC panel I | heart, brain, placenta, lung, liver, skeletal muscle, kidney, pancreas |
| Human MTC panel II | spleen, thymus, prostate, testis, ovary, small intestine, colon, peripheral leukocyte |
| Human digestive system MTC panel | liver, esophagus, stomach, duodenum, jejunum, ileum, ileocecal part, cecum, ascending colon, transverse colon, descending colon, rectum |

The results are shown in FIG 2.

In the human MTC panel and the MTC panel II, the human TCH099 gene product (mRNA) was expressed slightly in the thymus and peripheral leukocytes and somewhat in the small intestine. Strong expression was observed in the prostate, testis and ovary.

In the human digestive system MTC panel, the expression was observed in all of the sites from the duodenum to the rectum (particularly strong expression was observed in the duodenum and the jejunum). Slight expression was observed also in the liver, esophagus and stomach.

## EXAMPLE 4

Cloning of the human TCH 177 gene cDNA

Using two primer DNAs, primer A3 (SEQ ID NO: 33) and primer B3 (SEQ ID NO: 34), PCR was carried out on Marathon-Ready cDNA, human Brain (manufactured by Clontech), using Pyrobest DNA Polymerase (manufactured by Takara Shuzo Co., Ltd.). Using primer A4 (SEQ ID NO: 35) and primer B3, nested PCR was further conducted to acquire 2 clones, #1 and #2 of plasmid clone pCR-BluntII-TCH177. The plasmid clone was reacted using primer DNAs [primer SP6 (SEQ ID NO: 36), primer T7 (SEQ ID NO: 37), primer A1 (SEQ ID NO: 38) , primer B2 (SEQ ID NO: 39), primer F1 (SEQ ID NO: 40), primer R1 (SEQ ID NO: 41), primer R2 (SEQ ID NO: 42), primer 5-2 (SEQ ID NO: 43) and primer 3-2 (SEQ ID NO: 44)] as well as BigDye Terminator Cycle Sequencing Kit (manufactured by Applied Biosystems, Inc.). The base sequence of the inserted cDNA fragment was determined using a DNA sequencer, ABI PRISM 3100 DNA ANALYZER (manufactured by Applied Biosystems, Inc.). As a result, the 2 clones acquired the same DNA fragments and had the sequence of 1850 bases (SEQ ID NO: 45). The cDNA fragment (SEQ ID NO: 29) encoded the sequence of 589 amino acids (SEQ ID NO: 28), and the protein containing the amino acid sequence was named human TCH177 protein.

The transformant bearing the plasmid containing the cDNA fragment was named Escherichia coli TOP10/pCR-BluntII-TCH177.

Using Blast P [Nucleic Acids Res., 25, 3389, 1997], homology search was conducted on owl and the cDNA was found to be a novel gene belonging to the vesicular glutamate transporter (FIG 3).

Human TCH177 showed 61% homology on a base level and 73% homology on an amino acid level to vesicular glutamate transporter 1 (VGLUT1) (also sometimes called BNPI) (J. Neurochem., 66, 2227, 1996), which was a vesicular glutamate transporter reported in human, and 63% homology on a base level and 72% homology on an amino acid level to vesicular glutamate transporter 2 (VGLUT2) (J. Neurochem., 74, 2622, 2000), respectively.

## EXAMPLE 5

Analysis of the human TCH177 gene product on tissue distribution

Using two primer DNAs, i.e., primer TF (SEQ ID NO: 30) and primer TR (SEQ ID NO: 31), which were designed based on the sequence of human TCH177, and TaqMan probe T1 (SEQ ID NO: 32), the expression level of human TCH177 in cDNAs (Human MTC panel I and Human MTC panel II: manufactured by Clontech) from the respective tissues (heart, brain, placenta, lung, liver, skeletal muscle, kidney, pancreas, spleen, thymus, prostate, testis, ovary, small intestine, colon, peripheral leukocyte) in human was assayed by TaqMan PCR. Using TaqMan Gold RT-PCR Kit (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).

The results are shown in FIG 5. The human TCH177 gene product (mRNA) was expressed somewhat in the heart, brain, placenta, lung, small intestine and peripheral leukocytes, and expressed more strongly in the liver, thymus and colon.

## EXAMPLE 6

Cloning of cDNA encoding the mouse TCH177 protein

Using two primer DNAs, i.e., primer mOF (SEQ ID NO: 48) and primer mOR (SEQ ID NO: 49), PCR was carried out on mouse brain Marathon-Ready cDNA (both manufactured by Clontech Inc.) under the conditions (1) to (3) below, using Pyrobest DNA Polymerase (manufactured by Takara Shuzo Co., Ltd.).

(1) 94°C for 2 minutes
(2) 30 cycles of one set to include 98°C for 10 seconds - 68°C for 30 seconds - 72°C for 4 minutes
(3) 72°C for 10 minutes.

Further using this primary PCR product as a template together with primer OF1 (SEQ ID NO: 50) and primer OR1 (SEQ ID NO: 51), nested PR was carried out under the conditions (4) to (6) below, using Pyrobest DNA Polymerase (manufactured by Takara Shuzo Co., Ltd.).

(4) 94°C for 2 minutes
(5) 30 cycles of one set to include 98°C for 10 seconds - 65°C for 30 seconds - 72°C for 4 minutes
(6) 72°C for 10 minutes.

The amplified product obtained was cloned using Zero Blunt TOPO Cloning Kit (manufactured by Invitrogen) to acquire plasmid pCR-BluntII-mTCH177.

The plasmid was reacted using primer DNAs [primer SP6 (SEQ ID NO: 36), primer T7 (SEQ ID NO: 37), primer mA1 (SEQ ID NO: 52), primer mB1 (SEQ ID NO: 53), primer mF1 (SEQ ID NO: 54), primer mF2 (SEQ ID NO: 55), primer mR1 (SEQ ID NO: 56) and primer mR2 (SEQ ID NO: 57)] and BigDye Terminator Cycle Sequencing Kit (manufactured by Applied Biosystems, Inc.), and the base sequence of the inserted cDNA fragment was determined on a DNA sequencer, ABI PRISM 3100 DNA ANALYZER (manufactured by Applied Biosystems, Inc.). As a result, the clone was found to have the sequence of 1822 bases (SEQ ID NO: 58). The cDNA fragment (SEQ ID NO: 47) encoded the sequence of 601 amino acids (SEQ ID NO: 46), and the protein having the amino acid sequence was named mouse TCH177 protein.

The transformant bearing the plasmid containing the cDNA fragment was named Escherichia coli TOP10/pCR-BluntII-mTCH177.

Using Blast P [Nucleic Acids Res., 25, 3389, 1997], homology search was conducted on owl and the cDNA showed 63% homology on a base level and 71% homology on an amino acid level to human VGLUT2, which is a vesicular glutamate transporter. Mouse TCH177 showed 86% homology on a base level and 92% homology on an amino acid level and found to be a mouse homolog to human TCH177 (FIG. 5). Furthermore, the cDNA showed 65% homology on a base level and 71% homology on an amino acid level to mouse vesicular glutamate transporter 2 (mouse VGLUT2) (also sometimes called mouse DNPI) (J. Biol. Chem., 276, 36764, 2001), which was a vesicular glutamate transporter reported in mouse.

## EXAMPLE 7

Analysis of the mouse TCH177 gene product on tissue distribution

Using two primer DNAs, i.e., primer mTF (SEQ ID NO: 59) and primer mTR (SEQ ID NO: 60), which were designed based on the sequence of mouse TCH177, and TaqMan probe mT1 (SEQ ID NO: 61), the expression level of mouse TCH177 in cDNAs (Mouse MTC panel I and Mouse MTC panel II: manufactured by Clontech) in the respective organs (bone marrow, eye, lymph node, smooth muscle, prostate, thymus, stomach, uterus, heart, brain, spleen, lung, liver, skeletal muscle, kidney, testis, embryo (7 days), embryo (11 days), embryo (15 days), embryo (17 days)) in mice was assayed by TaqMan PCR. Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).

The results are shown in FIG. 6. The mouse TCH177 gene product (mRNA) was somewhat expressed over a broad range of the tissues in Mouse MTC panel I and MTC panel II.

## EXAMPLE 8

Acquisition of the human TCH136 gene cDNA

Using Blast N [Nucleic Acids Res., 25, 3389, 1997], homology search was conducted on the EST database (dbest), and the sequence of Accession No. BF966122 was a hit. Clone IMAGE: 4375480 (derived from the human hippocampus library) corresponding to this sequence was obtained through Invitrogen. The clone was reacted using primer DNAs [primer M13RV (SEQ ID NO: 64), primer T7 (SEQ ID NO: 65), primer A1 (SEQ ID NO: 66), primer A2 (SEQ ID NO: 67), primer B1 (SEQ ID NO: 68), primer F1 (SEQ ID NO: 69), primer F2 (SEQ ID NO: 70), primer F3 (SEQ ID NO: 71), primer F4 (SEQ ID NO: 72), primer F5 (SEQ ID NO: 73), primer F6 (SEQ ID NO: 74), primer F7 (SEQ ID NO: 75), primer R1 (SEQ ID NO: 76), primer R2 (SEQ ID NO: 77), primer R3 (SEQ ID NO: 78), primer R4 (SEQ ID NO: 79), primer R5 (SEQ ID NO: 80), primer R6 (SEQ ID NO: 81) and primer R7 (SEQ ID NO: 82)] and BigDye Terminator Cycle Sequencing Kit (manufactured by Applied Biosystems, Inc.), and the base sequence of cDNA fragment inserted was determined using a DNA sequencer, ABI PRISM 3100 DNA ANALYZER (manufactured by Applied Biosystems, Inc.). As a result, the cDNA fragment had the sequence of 5174 bases (SEQ ID NO: 83). The sequence of 436 amino acids (SEQ ID NO: 62) encoded the cDNA fragment (SEQ ID NO: 63) and the protein containing the amino acid sequence was named human TCH136 protein.
The transformant bearing the plasmid containing the cDNA fragment was named Escherichia coli DH10B/pBluescriptR-TCH136.
Using Blast P [Nucleic Acids Res., 25, 3389, 1997], homology search was conducted on OWL and the cDNA was found to be a novel gene belonging to the potential-dependent $K^+$ channel (FIG. 7). In the figure, the transmembrane domains are shown by TM1 through 6.
The cDNA showed 44% homology on a base level and 37% homology on an amino acid level to Kv6.2 (Receptors Channels., 6, 337, 1999), which was the γ-subunit of potential-dependent $K^+$ channel reported in human. The protein was thus predicted to have a structure of 6 transmembrane type.

## EXAMPLE 9

Analysis of the human TCH136 gene product on tissue distribution

Using two primer DNAs, i.e., primer TF2 (SEQ ID NO: 94) and primer TR2 (SEQ ID NO: 85), which were designed based on the sequence of human TCH136, and TaqMan probe T2 (SEQ ID NO: 86), the expression level of human TCH136 in cDNAs (Human MTC panel I and Human MTC panel II: manufactured by Clontech) from the respective tissues (heart, brain, placenta, lung, liver, skeletal muscle, kidney, pancreas, spleen, thymus, prostate, testis, ovary, small intestine, colon, peripheral leukocyte) in human was assayed by TaqMan PCR. Using TaqMan Gold RT-PCR Kit (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).
The human TCH136 gene product (mRNA) was expressed slightly in the lung, kidney, ovary and small intestine, and somewhat expressed also in the testis. Strong expression was shown in the colon, pancreas and thymus [FIG. 8(A)].
Using two primer DNAs, i.e., primer A2 (SEQ ID NO: 67) and primer B3 (SEQ ID NO: 87), which were designed based on the sequence of human TCH136, PCR was performed on cDNAs (Human MTC panel I and Human MTC panel II: manufactured by Clontech) from the respective tissues (heart, brain, placenta, lung, liver, skeletal muscle, kidney, pancreas, spleen, thymus, prostate, testis, ovary, small intestine, colon, peripheral leukocyte) in human. The amplified band was stained with ethidium bromide to detect expression of human TCH136.
The results are shown in FIG. 8 (B). In addition to the tissues where its expression was confirmed in FIG. 8 (A), the human TCH136 gene product (mRNA) was expressed also in the brain and prostate as well.

## EXAMPLE 10

Identification of partial sequence of the mouse TCH099 gene

Using two primer DNAs, primer m099A1(SEQ ID NO: 88)および primer m099B1(SEQ ID NO: 89), PCR was carried out on mouse testis Marathon-Ready cDNA (manufactured by Clontech) under the following conditions (1) to (5), using Advantage 2 DNA Polymerase (manufactured by Clontech).

(1) 94°C for 3 minutes
(2) 5 cycles of one set to include 94°C for 5 seconds - 72°C for 1 minute
(3) 5 cycles of one set to include 94°C for 5 seconds - 70°C for 1 minute
(4) 25 cycles of one set to include 94°C for 5 seconds - 68°C for 1 minute
(5) 70°C for 10 minutes

After the amplified product was subjected to gel electrophoresis, the fragment of about 0.5 kb was excised and purified on QIAquick Gel Extraction Kit (manufactured by Qiagen). Using primer m099A1 (SEQ ID NO: 88), primer m099B1 (SEQ ID NO: 89) and BigDye Terminator Cycle Sequencing Kit (manufactured by Applied Biosystems, Inc.), the reaction was carried out and the base sequence of the PCR product amplified was determined using a DNA sequencer, ABI PRISM 3100 DNAANALYZER (manufactured by Applied Biosystems, Inc.).
As a result, the partial sequence of the mouse TCH099 gene cDNA having the sequence of 477 bases represented by SEQ ID NO: 90 was identified.

## EXAMPLE 11

(1) Preparation of cDNAs from the respective tissues in normal mice

The total RNA was prepared from the respective tissues in BALB/c mice of 7 weeks old [cerebrum, cerebellum, hippocampus, medulla oblongata, bone marrow, sciatic nerve, skin, skeletal muscle, eyeball, heart, lung, trachea, pancreas, kidney, liver, anterior stomach, posterior stomach, duodenum, jejunoileum, cecum, colon, rectum, spleen, thymus, bone marrow, ovary, uterus, prostate, testis (the ovary and uterus were collected from the female animal and the other tissues were from the male animal, respectively, each tissue in 1 to 10 mice)], using ISOGEN (manufactured by Nippon Gene) or RNeasy Mini Kit (manufactured by Qiagen). Using TaqMan Reverse Transcription Reagents (manufactured by Applied Biosystems, Inc.), reverse transcription was performed to prepare cDNA.

(2) Analysis of the mouse TCH099 gene product on tissue distribution

Using two primer DNAs, i.e., primer m099A2 (SEQ ID NO: 91) and primer m099B2 (SEQ ID NO: 92), which were designed based on the sequence of SEQ ID NO: 90, and TaqMan probe m099T1 (SEQ ID NO: 93), the expression level (copy number) of mouse TCH099 in cDNAs from the respective mouse tissues described above was assayed by TaqMan PCR. The expression level (copy number) of rodent glyceraldehide-3-phosphate dehydrogenase (GAPDH) was also assayed for the same cDNAs, using TaqMan rodent GAPDH control reagents (manufactured by Applied Biosystems, Inc.). Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).
The results are shown in FIG. 9. In the respective tissues of BALB/c mice of 7 weeks old, the mouse TCH099 gene product (mRNA) was somewhat expressed in the sciatic nerve, kidney, ovary, testis, cecum, etc. and highly expressed in the duodenum, jejunoileum, rectum and pancreas.

## EXAMPLE 12

Construction of human TCH099 expression vector

Human TCH099 (SEQ ID NO: 1) expression vector was constructed by the following procedure. Using as a template 100 ng of the plasmid obtained in EXAMPLE 1 and using primer A14d (SEQ ID NO: 94) and primer B19S (SEQ ID NO: 95), PCR was carried out under the conditions (1) to (3) below, using Pyrobest DNA Polymerase (manufactured by Takara Shuzo Co., Ltd.).

(1) 94°C for 3 minutes
(2) 25 cycles of one set to include 98°C for 10 seconds - 65°C for 30 seconds - 72°C for 3 minutes
(3) 72°C for 10 minutes.

After the PCR reaction solution was subjected to gel electrophoresis, the major band was purified. The PCR fragment thus obtained was cloned using pcDNA3.1 Directional TOPO expression kit (manufactured by Invitrogen).

From a plurality of colonies thus obtained, the plasmid was prepared and the reaction was carried out using primer DNAs [primer BGHRV (SEQ ID NO: 96), primer T7 (SEQ ID NO: 7), primer A2 (SEQ ID NO: 8), primer A4 (SEQ ID NO: 9), primer F1 (SEQ ID NO: 10), primer B1 (SEQ ID NO: 11), primer R1 (SEQ ID NO: 12), primer B3 (SEQ ID NO: 13)] and BigDye Terminator Cycle Sequencing Kit (manufactured by Applied Biosystems, Inc.), and the base sequence was confirmed using a DNA sequencer, ABI PRISM 3100 DNA ANALYZER (manufactured by Applied Biosystems, Inc.). The transformant bearing this plasmid was named Escherichia coli TOP10/pCDNA3.1D-TCH099.

## EXAMPLE 13

Preparation of the human TCH099-expressed CHO cell line

Escherichia coli TOP10/pCDNA3.1D(+)-TCH099 was cultured and plasmid DNA was prepared from the cells of this Escherichia coli using EndoFree Plasmid Maxi Kit (manufactured by Qiagen). This plasmid DNA was transfected to CHO dhfr$^-$ cells using FuGENE 6 Transfection Reagent (manufactured by Roche) in accordance with the protocols attached thereto. A mixture of 2 μg of the plasmid DNA and a transfection reagent was added to a Petri dish of 6 cm diameter, in which 3 x $10^5$ CHO dhfr$^-$ cells had been plated 24 hours before the transfection. After incubation for a day in MEMα medium supplemented with 10% fetal calf serum, the cells were stripped by trypsin treatment and after diluting appropriately, the recovered cells were plated on a 10 cm Petri dish. After further 24 hours, 0.5 mg/ml of G418 was added to the medium. For 10 days thereafter, human TCH099 expression cells were selected in the medium containing 0.5 - 1.0 mg/ml of G418. In the G418-containing selective medium, 125 colonies of monoclonal human TCH099 expression cells grown were selected.

## EXAMPLE 14

Assay for the expression level of gene transfected in the human TCH099 gene-expressed CHO cell line using TaqMan PCR

The human TCH099-expressed CHO cell line prepared in EXAMPLE 13 was cultured on a 96-well plate, and the total RNA was prepared from the proliferated cells using SV 96 Total RNA Isolation System (manufactured by Promega Corporation). The total RNA prepared was subjected to reverse transcription using TaqMan Reverse Transcription Reagents (manufactured by Applied Biosystems, Inc.) to prepare cDNA. The expression level of human TCH099 was assayed for the cDNA by TaqMan PCR using primer A13 (SEQ ID NO: 25) and primer B 18 (SEQ ID NO: 26) used in EXAMPLE 3 and TaqMan probe T1 (SEQ ID NO: 27). The reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.). Clone No. 33 was selected as the human TCH099 gene high expression cell line.

## EXAMPLE 15

Analysis of the mouse TCH177 gene product on tissue distribution

Using primer mTF (SEQ ID NO: 59) and primer mTR (SEQ ID NO: 60) used in EXAMPLE 9 and TaqMan probe mT1 (SEQ ID NO: 61) prepared in EXAMPLE 11, the expression level (copy number) of mouse TCH177 in cDNAs from the respective mouse tissues prepared in EXAMPLE 11 was assayed by TaqMan PCR. The expression level (copy number) of rodent glyceraldehide-3-phosphate dehydrogenase (GAPDH) was also assayed for the same cDNAs, using TaqMan rodent GAPDH control reagents (manufactured by Applied Biosystems, Inc.). Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).

The results are shown in FIG. 10.

In the respective tissues of BALB/c of 7 weeks old, the mouse TCH177 gene product (mRNA) was expressed highly in the sciatic nerve, hippocampus, bone marrow, medulla oblongata, cerebrum, eyeball, ovary, uterus and thymus, and the highest expression was observed in the liver.

## EXAMPLE 16

Identification of partial sequence of the rat TCH177 gene

Using two primer DNAs used in EXAMPLE 6, i.e., primer mA1 (SEQ ID NO: 52) and primer mB1 (SEQ ID NO: 53), PCR was carried out on rat brain Marathon-Ready cDNA (manufactured by Clontech Inc.) under the conditions (1) to (3) below, using Advantage 2 DNA Polymerase (manufactured by Clontech).

(1) 95°C for 1 minute
(2) 35 cycles of one set to include 95°C for 30 seconds - 60°C for 30 seconds - 68°C for 3 minutes
(3) 68°C for 3 minutes

After the amplified product was subjected to gel electrophoresis, the fragment of about 0.5 kb was excised and purified on QIAquick Gel Extraction Kit (manufactured by Qiagen). Using primer DNAs, i.e., primer mA1 (SEQ ID NO: 52), primer mB1 (SEQ ID NO: 53) and BigDye Terminator Cycle Sequencing Kit (manufactured by Applied Biosystems, Inc.), the reaction was carried out and the base sequence of the PCR product amplified was determined using a DNA sequencer, ABI PRISM 3100 DNA ANALYZER (manufactured by Applied Biosystems, Inc.). As a result, the partial sequence of the rat TCH177 gene cDNA having the sequence of 536 bases represented by SEQ ID NO: 97 was identified.

## EXAMPLE 17

(1) Preparation of cDNAs from the respective tissues in normal rats

The total RNA was prepared from the respective tissues (cerebrum, cerebellum, liver, kidney, prostate, heart, lung, duodenum, jejunoileum, colon, skin, eyeball) in Wistar male rats of 12 weeks old, using RNeasy Mini Kit (manufactured by Qiagen). The total RNA prepared was subjected to reverse transcription using TaqMan Reverse Transcription Reagents (manufactured by Applied Biosystems, Inc.) to prepare cDNA.

(2) Analysis of the rat TCH177 gene product on tissue distribution

Using two primer DNAs, primer rTF (SEQ ID NO: 98) and primer rTR (SEQ ID NO: 99), which were designed based on the sequence of SEQ ID NO: 97, and TaqMan probe rT1 (SEQ ID NO: 100), the expression level (copy number) of rat TCH177 in cDNAs from the respective rat tissues described above was assayed by TaqMan PCR. The expression level (copy number) of rodent glyceraldehide-3-phosphate dehydrogenase (GAPDH) was also assayed for the same cDNAs, using TaqMan rodent GAPDH control reagents (manufactured by Applied Biosystems, Inc.). Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).
The results are shown in FIG 11. In the respective tissues of Wistar rats of 12 weeks old, the rat TCH177 gene product (mRNA) was somewhat expressed in kidney, lung, duodenum and skin and highly expressed in the cerebrum, liver, jejunoileum, colon, rectum and eyeball.

## EXAMPLE 18

Construction of human TCH177 expression vector

Using as a template 50 ng of the plasmid obtained in EXAMPLE 4 and using primer OF (SEQ ID NO: 101) and primer OR(SEQ ID NO: 102), PCR was carried out under the conditions (1) to (3) below, using Pyrobest DNA Polymerase (manufactured by Takara Shuzo Co., Ltd.). The primer OF at the 5' end and the primer OR at the 3' end were designed to add Hind III site and Eco RI site at the 5' end, respectively, for cloning onto the vector.

(1) 94°C for 2 minutes
(2) 30 cycles of one set to include 98°C for 10 seconds - 57°C for 30 seconds - 72°C for 3.5 minutes
(3) 72°C for 10 minutes.

After the PCR reaction solution was subjected to gel electrophoresis, the major band was purified. The PCR fragment thus obtained was digested with restriction enzymes Hind III and EcoR I by warming at 37°C for an hour. The reaction solution was subjected to gel electrophoresis and then purified. The reaction solution was ligated to the Hind III site and EcoR I site of pcDNA3.1 (+) (manufactured by Invitrogen), which was an expression vector for animal cells, using Takara ligation kit ver.2 (manufactured by Takara Shuzo Co., Ltd.). After treatment by ethanol precipitation, the ligation reaction solution was transfected to Escherichia coli TOP10 (manufactured by Invitrogen), which is competent cells. From a plurality of colonies thus obtained, the plasmid was prepared and the reaction was carried out using primer DNAs [primer BGH RV (SEQ ID NO: 96), primer T7 (SEQ ID NO: 37), primer A1 (SEQ ID NO: 38), primer B2 (SEQ ID NO: 39), primer F1 (SEQ ID NO: 40), primer R1 (SEQ ID NO: 41)] and BigDye Terminator Cycle Sequencing Kit (manufactured by Applied Biosystems, Inc.). The base sequence was confirmed using a DNA sequencer, ABI PRISM 3100 DNA ANALYZER (manufactured by Applied Biosystems, Inc.). The transformant bearing this plasmid was named Escherichia coli TOP10/pCDNA3.1(+)-TCH177.

## EXAMPLE 19

Preparation of the human TCH177-expressed CHO cell line

Escherichia coli TOP10/pCDNA3.1(+)-TCH177 was cultured and the plasmid DNA was prepared from the cells of this Escherichia coli using EndoFree Plasmid Maxi Kit (manufactured by Qiagen). This plasmid DNA was transfected to CHO dhfr$^-$ cells using FuGENE 6 Transfection Reagent (manufactured by Roche) in accordance with the protocols attached thereto. A mixture of 2 μg of the plasmid DNA and a transfection reagent was added to a Petri dish of 6 cm diameter, in which $3 \times 10^5$ CHO dhfr$^-$ cells had been plated 24 hours before the transfection. After incubation for a day in MEMα medium supplemented with 10% fetal calf serum, the cells were stripped by trypsin treatment and after diluting appropriately, the recovered cells were plated on a 10 cm Petri dish. After further 24 hours, 0.5 mg/ml of G418 was added to the medium. For 10 days thereafter, the human TCH177 expression cells were selected in the medium containing 0.5 - 1.0 mg/ml of G418. In the G418-containing selective medium, 80 colonies of monoclonal human TCH177 expression cells grown were selected.

## EXAMPLE 20

Assay for the expression level of gene transfected in the human TCH177 gene-expressed CHO cell line using TaqMan PCR

The human TCH177-expressed CHO cell line prepared in EXAMPLE 19 was cultured on a 48-well plate or a 24-well plate, and the total RNA was prepared from the proliferated cells using RNeasy 96 Kit (manufactured by Qiagen). The total RNA prepared was subjected to reverse transcription using TaqMan Reverse Transcription Reagents (manufactured by Applied Biosystems, Inc.) to prepare cDNA. The expression level of human TCH177 was assayed for the cDNA by TaqMan PCR using primer TF (SEQ ID NO: 30) and primer TR (SEQ ID NO: 31) used in EXAMPLE 5 and TaqMan probe T1 (SEQ ID NO: 32). The reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.). Clone No. 24 was selected as the human TCH177 gene-highly expressed cell line.

## EXAMPLE 21

Identification of partial sequence of the mouse TCH136 gene

Using two primer DNAs, i.e., primer mA1 (SEQ ID NO: 103) and primer mB1 (SEQ ID NO: 104), PCR was carried out on mouse testis Marathon-Ready cDNA (manufactured by Clontech Inc.) under the conditions (1) to (3) below, using Advantage 2 DNA Polymerase (manufactured by Clontech).

(1) 95°C for 1 minute
(2) 35 cycles of one set to include 95°C for 30 seconds - 68°C for 3 minutes
(3) 68°C for 3 minutes

After the amplified product was subjected to gel electrophoresis, the fragment of about 1.0 kb was excised and purified on QIAquick Gel Extraction Kit (manufactured by Qiagen). Using primer DNAs, i.e., primer mA1 (SEQ ID NO: 103) and primer mB1 (SEQ ID NO: 104) and BigDye Terminator Cycle Sequencing Kit (manufactured by Applied Biosystems, Inc.), the reaction was carried out and the base sequence of the PCR product amplified was determined using a DNA sequencer, ABI PRISM 3100 DNA ANALYZER (manufactured by Applied Biosystems, Inc.). As a result, the partial sequence of the mouse TCH136 gene cDNA having the sequence of 950 bases represented by SEQ ID NO: 105 was identified.

## EXAMPLE 22

Analysis of the mouse TCH136 gene product on tissue distribution

Using two primer DNAs, i.e., primer mTF (SEQ ID NO: 106) and primer mTR (SEQ ID NO: 107), which were designed on the basis of the base sequence represented by SEQ ID NO: 105, together with TaqMan probe mT1 (SEQ ID NO: 108), the expression level (copy number) of mouse TCH136 in cDNAs from the respective mouse tissues prepared in EXAMPLE 11 was assayed by TaqMan PCR. The expression level (copy number) of rodent glyceraldehide-3-phosphate dehydrogenase (GAPDH) was also assayed for the same cDNAs, using TaqMan rodent GAPDH control reagents (manufactured by Applied Biosystems, Inc.). Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).

The results are shown in FIG. 12.

In the respective tissues from BALB/c mice of 7 weeks old, the mouse TCH136 gene product (mRNA) was highly expressed in the cerebrum, uterus, ovary, testis, etc.

## EXAMPLE 23

Analysis of expression of the human TCH099, human TCH177 and human TCH136 genes in normal human cells

(1) Preparation of normal human cell cDNA

Normal human cells were purchased the product from Cambrex BioScience Walkersvill and incubated in accordance with the procedures described in the instructions attached to the product. The cells used for the experiments and the media used for incubation of the respective cells are shown in [TABLE 3].

[TABLE 3]

| No. | Cell | Medium |
|---|---|---|
| 1 | Umbilical vein endothelial cell CC-2517 | Bullet Kit EGM CC-3124 |
| 2 | Aortic endothelial cell CC-2535 | Bullet Kit EGM-2 CC-3162 |
| 3 | Coronary artery endothelial cell CC-2585 | Bullet Kit EGM-2MV CC-3202 |
| 4 | Aortic smooth muscle cell CC-2571 | Bullet Kit SmGM-2 CC-3182 |
| 5 | Coronary artery smooth muscle cell CC-2583 | Bullet Kit SmGM-2 CC-3182 |
| 6 | Uterine smooth muscle cell CC-2562 | Bullet Kit SmGM-2 CC-3182 |
| 7 | Bronchial smooth muscle cell CC-2576 | Bullet Kit SmGM-2 CC-3182 |
| 8 | Skeletal muscle satellite cell CC-2561 | Bullet Kit SkGM CC-3160 |
| 9 | Mammary epithelial cell CC-2551 | Bullet Kit MEGM CC-3150 |
| 10 | Bronchial epithelial cell (with RA) CC-2540 | Bullet Kit SAGM CC-3118 |
| 11 | Bronchial epithelial cell (without RA) CC-2541 | Bullet Kit SAGM CC-3118 |
| 12 | Lung fibroblast CC-2512 | Bullet Kit FGM-2 CC-3132 |
| 13 | Kidney proximal tubular epithelial cell CC-2553 | Bullet Kit REGM CC-3190 |
| 14 | Mesangial cell CC-2559 | Bullet Kit MsGM CC-3146 |
| 15 | Kidney cortical epithelial cell CC-2554 | Bullet Kit REGM CC-3190 |

[TABLE 3] (continued)

| No. | Cell | Medium |
|---|---|---|
| 16 | Mesenchymal stem sell PT-2501 | Bullet Kit MSCGM PT-3001 |
| 17 | Knee joint chondrocyte CC-2550 | Bullet Kit CGM CC-3216 |
| 18 | Osteoblast CC-2538 | Bullet Kit OGM CC-3207 |

Each of the cells was incubated in a 75 $cm^2$ culture flask to reach a subconfluent state and the cells were recovered by trypsin-EDTA treatment. From the recovered cells, the total RNA was prepared using ISOGEN (manufactured by Nippon Gene Co., Ltd.) or RNeasy Mini Kit (manufactured by Qiagen). The total RNA prepared was subjected to reverse transcription using TaqMan Reverse Transcription Reagents (manufactured by Applied Biosystems, Inc.) to prepare the cDNA.

(2) Analysis of expression of the human TCH099, human TCH177 and human TCH136 genes in normal human cells commercially available

The expression level (Ct values) in each cDNA described above was assayed by TaqMan PCR as below. For human TCH099, primer A13 (SEQ ID NO: 25) and primer B18 (SEQ ID NO: 26) used in EXAMPLE 3 and TaqMan probe T1 (SEQ ID NO: 27) were used; primer TF (SEQ ID NO: 30) and primer TR (SEQ ID NO: 31) used in EXAMPLE 5 and TaqMan probe T1 (SEQ ID NO: 32) were used for human TCH177; and primer TF (SEQ ID NO: 84) and primer TR (SEQ ID NO: 85) used in EXAMPLE 9 and TaqMan probe T1 (SEQ ID NO: 86) were used for human TCH136. The expression level (Ct values) of rodent glyceraldehide-3-phosphate dehydrogenase (GAPDH) was also assayed for the same cDNAs, using TaqMan GAPDH control reagents (manufactured by Applied Biosystems, Inc.). Using TaqMan Universal PCR Master Mix (manufactured by Applied Biosystems, Inc.), the reaction was carried out by initially reacting at 50°C for 2 minutes, further maintaining at 95°C for 10 minutes and then repeating 40 cycles of one reaction set to include at 95°C for 15 seconds and at 60°C for 1 minute, while detection was simultaneously made on the ABI PRISM 7900 sequence detection system (manufactured by Applied Biosystems, Inc.).

Based on the assay values obtained by the procedures above, the relative expression levels of the human TCH099, human TCH177 and human TCH136 genes to GAPDH were calculated, respectively, in accordance with the following equation.

$$\text{Relative expression level} = 1/2^{A-B}$$

In the equation described above, A represents Ct values of the human TCH099, human TCH177 or human TCH136 gene and B represents Ct values of the GAPDH gene.

The results are shown in [TABLE 4].

[TABLE 4]

| No. | Cell | Human TCH099 | Human TCH177 | Human TCH136 |
|---|---|---|---|---|
| 1 | Umbilical vein endothelial cell CC-2517 | ○ | | ○ |
| 2 | Aortic endothelial cell CC-2535 | ○ | | ○ |
| 3 | Coronary artery endothelial cell CC-2585 | | ○ | ○ |
| 4 | Aortic smooth muscle cell CC-2571 | ○ | | ○ |
| 5 | Coronary artery smooth muscle cell CC-2583 | | ○ | |
| 6 | Uterine smooth muscle cell CC-2562 | | ○ | ○ |
| 7 | Bronchial smooth muscle cell CC-2576 | | ○ | |
| 8 | Skeletal muscle satellite cell CC-2561 | ○ | ○ | ○ |
| 9 | Mammary epithelial cell CC-2551 | ○ | ○ | |
| 10 | Bronchial epithelial cell (with RA) CC-2540 | | | |
| 11 | Bronchial epithelial cell (without RA) CC-2541 | | ○ | |

[TABLE 4] (continued)

| No. | Cell | Human TCH099 | Human TCH177 | Human TCH136 |
|---|---|---|---|---|
| 12 | Lung fibroblast CC-2512 | | ○ | ○ |
| 13 | Kidney proximal tubular epithelial cell CC-2553 | | | ○ |
| 14 | Mesangial cell CC-2559 | | | |
| 15 | Kidney cortical epithelial cell CC-2554 | | | ○ |
| 16 | Mesenchymal stem sell PT-2501 | | ○ | |
| 17 | Knee joint chondrocyte CC-2550 | ○ | | |
| 18 | Osteoblast CC-2538 | | ○ | ○ |

In the table, symbol o designates that the expression was observed.

Human TCH099 was expressed somewhat in the umbilical vein endothelial cells, aortic endothelial cells, aortic smooth muscle cells, skeletal muscle cells, mammary epithelial cells and chondrocytes.

Human TCH177 was expressed somewhat in the coronary artery endothelial cells, uterine smooth muscle cells, bronchial smooth muscle cells, skeletal muscle satellite cells, mammary epithelial cells, bronchial epithelial cells, lung fibroblasts, mesenchymal stem cells and osteoblasts.

Human TCH136 was expressed somewhat in the umbilical vein endothelial cells, aortic endothelial cells, coronary artery endothelial cells, uterine smooth muscle cells, skeletal muscle satellite cells, lung fibroblasts, kidney proximal tubular epithelial cells, kidney cortical epithelial cells and osteoblasts.

## EXAMPLE 24

Measurement of uptake of 2-[1,2-$^3$H (N)]-Deoxy-D-Glucose in the human TCH099-expressed CHO cell line

Using the TCH099-expressed CHO cell line clone No. 33 acquired in EXAMPLE 14, the uptake of 2-[1,2-$^3$H (N)]-Deoxy-D-Glucose (hereinafter also sometimes referred to as 2-[$^3$H]-DOG) was measured.

The TCH099-expressed CHO cell line clone No. 33 was plated on a 24-well collagen-coated plate in 2.5 x $10^5$ cells/well, followed by incubation at 37°C for 24 hours. The medium was removed and the cells were washed 3 times with 1mL of KCl buffer [125 mM KCl, 1.2 mM $KH_2PO_4$, 2.5 mM $CaCl_2$, 1.2 mM $MgSO_4$, 4 mM Glutamine, 0.1 mg/mL bovine serum albumin and 10 mM Hepes (pH 7.2)]. After 1 mL of KCl buffer was supplemented and incubation was performed at 37°C for an hour, the buffer was replaced by 450 µL of KCl buffer (containing 100 nM insulin). Thirty minutes after, 50 µL of 100 µM 2-[$^3$H]-DOG (manufactured by Perkin-Elmer Life Science). Incubation was performed at 37°C for 30 minutes followed by washing 3 times with 1mL of KCl buffer. The cells were lysed in 0.5N NaOH and the radioactivity contained in the lysate was measured with a scintillation counter to determine the amount of 2-[$^3$H]-DOG taken up into the cells. The cells obtained by introducing vector pcDNA3.1(+) into CHO dhfr$^-$ cells (hereinafter sometimes also referred to as Mock cells) were treated in a similar manner to measure the radioactivity as well. For statistics analysis, Student's t-test was performed using SAS software (manufacture by SAS).

The results are shown in FIG. 13.

It became clear that a larger amount of 2-[$^3$H]-DOG was taken up into the human TCH099-expressed CHO cells than Mock cells. The difference was significant ($p = 0.0069$).

## INDUSTRIAL APPLICABILITY

The protein A of the present invention is useful as diagnostic markers, etc. for, e.g., diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. The compound that promotes or inhibits the activity of the protein obtained by the screening method using the protein can be used as agents for the prevention/treatment of, for example, diabetes mellitus, hyperlipemia, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc.

The protein B of the present invention is useful as diagnostic markers, etc. for, e.g., central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e. g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. The compound that promotes or inhibits the activity of the protein obtained by the screening method using the protein can be used as agents for the prevention/treatment of, for example, central nervous system disorders (e.g., Alzheimer' disease, parkinsonian syndrome, schizophrenia, cerebrovascular dementia, cerebral ischemia, epilepsy, etc.), endocrine diseases (e.g., hyperprolactinemia, Basedow's disease, pheochromocytoma, Cushing syndrome, etc.), diabetes mellitus, arteriosclerosis, alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), hepatic diseases (e.g., liver cirrhosis, hepatitis, alcoholic liver disease, etc.), cardiovascular diseases (e.g., cardiac failure, arrhythmia, long QT syndrome, etc.), reproductive diseases (e.g., infertility, benign prostatic hyperplasia, prostatitis, testicular neurosis, hypersensitivity of testis, ovarian dysfunction, etc.), or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc.

The protein C of the present invention is useful as diagnostic markers, etc. for, e.g., central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like. The compound that promotes or inhibits the activity of the protein obtained by the screening method using the protein can be used as agents for the prevention/treatment of, for example, central nervous system disorders (e.g., Alzheimer' disease, parkinsonian syndrome, schizophrenia, cerebrovascular dementia, cerebral ischemia, epilepsy, etc.), alimentary disorders (e.g., irritable bowel syndrome, ulcerative colitis, Crohn's disease, ischemic colitis, gastritis, peptic ulcer, proctitis, reflux esophagitis, etc.), inflammatory diseases (e.g., sepsis, pneumonia, encephalitis, hepatitis, myocarditis, etc.), respiratory disorders (e.g., chronic obstructive pulmonary disease, bronchial asthma, etc.), autoimmune diseases (e.g., myasthenia gravis, glomerulonephritis, multiple sclerosis, Sjögren's syndrome, chronic articular rheumatism, systemic lupus erythematosus, etc.), allergic disorders (e.g., pollinosis, allergic rhinitis, anaphylactic shock, atopic dermatitis, etc.), thymus disorders, immunodeficiency (e.g., immunodeficiency accompanied by leukocyte abnormality, splenic dysfunction or thymic abnormality, etc.), pancreatic disorders (e.g., pancreatic dysfunction such as chronic pancreatitis, cystic fibrosis of the pancreas, etc.), diabetes mellitus, arteriosclerosis, reproductive diseases (e.g., infertility, benign prostatic hyperplasia, prostatitis, testicular neurosis, hypersensitivity of testis, ovarian dysfunction, etc.), cardiovascular diseases (e.g., cardiac failure, arrhythmia, long QT syndrome, etc.), muscular disorders (e.g., muscular atrophy, etc.) or cancer (e.g., lung cancer, renal cancer, liver cancer, non-small-cell lung cancer, ovarian cancer, prostate cancer, gastric cancer, bladder cancer, breast cancer, cervical cancer, colon cancer, rectal cancer, pancreatic cancer, thymoma, etc.) or the like; anti-rejection drugs after organ transplant; etc.

## Claims

**1.** A protein containing the same or substantially the same amino acid sequence as the amino acid sequence represented by SEQ ID NO: 1 or SEQ ID NO: 23, or a salt thereof.

**2.** A protein consisting of the amino acid sequence represented by SEQ ID NO: 1, or a salt thereof.

**3.** A protein consisting of the amino acid sequence represented by SEQ ID NO: 23, or a salt thereof.

**4.** A partial peptide of the protein according to claim 1, or a salt thereof.

**5.** A polynucleotide containing a polynucleotide encoding the protein according to claim 1, or the partial peptide according to claim 4.

**6.** The polynucleotide according to claim 5, which is a DNA.

**7.** A DNA consisting of the base sequence represented by SEQ ID NO: 2, SEQ ID NO: 14 or SEQ ID NO: 24.

**8.** A recombinant vector containing the polynucleotide according to claim 5.

**9.** A transformant transformed by the recombinant vector according to claim 8.

**10.** A method of manufacturing the protein according to claim 1 or the partial peptide according to claim 4, or a salt thereof, which comprises culturing the transformant according to claim 9, producing/accumulating the protein according to claim 1 or the partial peptide according to claim 4, and collecting the same.

**11.** A pharmaceutical comprising the protein according to claim 1 or the partial peptide according to claim 4, or a salt thereof.

**12.** A pharmaceutical comprising the polynucleotide according to claim 5.

**13.** An antibody to the protein according to claim 1 or the partial peptide according to claim 4, or a salt thereof.

**14.** A pharmaceutical comprising the antibody according to claim 13.

**15.** A diagnostic product comprising the antibody according to claim 13.

**16.** A polynucleotide containing a base sequence complementary or substantially complementary to the base sequence of the polynucleotide according to claim 5, or a part of the base sequence.

**17.** A pharmaceutical comprising the polypeptide according to claim 16.

**18.** A method of screening a compound or its salt that promotes or inhibits the activity of the protein according to claim 1 or the partial peptide according to claim 4, or a salt thereof, which comprises using the protein according to claim 1 or the partial peptide according to claim 4, or a salt thereof.

**19.** A kit for screening a compound or its salt that promotes or inhibits the activity of the protein according to claim 1 or the partial peptide according to claim 4, or a salt thereof, comprising the protein according to claim 1 or the partial peptide according to claim 4, or a salt thereof.

**20.** A compound or its salt that promotes or inhibits the activity of the protein according to claim 1 or the partial peptide according to claim 4, or a salt thereof, which is obtainable using the screening method according to claim 18 or the screening kit according to claim 19.

**21.** A pharmaceutical comprising the compound or its salt according to claim 20.

**22.** A method of screening a compound or its salt that promotes or inhibits the expression of a gene for the protein according to claim 1, which comprises using the polypeptide according to claim 5.

**23.** A kit for screening a compound or its salt that promotes or inhibits the expression of a gene for the protein according to claim 1, comprising the polypeptide according to claim 5.

**24.** A compound or its salt that promotes or inhibits the expression of a gene for the protein according to claim 1, which is obtainable using the screening method according to claim 22 or the screening kit according to claim 23.

**25.** A pharmaceutical comprising the compound or its salt according to claim 24.

**26.** The pharmaceutical according to claim 11, 12, 14, 17, 21 or 25, which is an agent for the prevention/treatment of diabetes mellitus, hyperlipemia or arteriosclerosis.

**27.** A method of preventing/treating diabetes mellitus, hyperlipemia or arteriosclerosis, which comprises administering an effective dose of the compound or its salt according to claim 20 or 24 to a mammal.

**28.** Use of the compound or its salt according to claim 20 or 24 for manufacturing an agent for the prevention/treatment of diabetes mellitus, hyperlipemia or arteriosclerosis.

**29.** A protein containing the same or substantially the same amino acid sequence as the amino acid sequence represented by SEQ ID NO: 28 or SEQ ID NO: 46, or a salt thereof.

**30.** A protein consisting of the amino acid sequence represented by SEQ ID NO: 28, or a salt thereof.

**31.** A protein consisting of the amino acid sequence represented by SEQ ID NO: 46, or a salt thereof.

**32.** A partial peptide of the protein according to claim 29, or a salt thereof.

**33.** A polynucleotide containing a polynucleotide encoding the protein according to claim 29, or the partial peptide according to claim 32.

**34.** The polynucleotide according to claim 33, which is a DNA.

**35.** A DNA consisting of the base sequence represented by SEQ ID NO: 29, SEQ ID NO: 45, SEQ ID NO: 47 or SEQ ID NO: 58.

**36.** A recombinant vector containing the polynucleotide according to claim 33.

**37.** A transformant transformed by the recombinant vector according to claim 36.

**38.** A method of manufacturing the protein according to claim 29 or the partial peptide according to claim 32, or a salt thereof, which comprises culturing the transformant according to claim 37, producing/accumulating the protein according to claim 29 or the partial peptide according to claim 32, and collecting the same.

**39.** A pharmaceutical comprising the protein according to claim 29 or the partial peptide according to claim 32, or a salt thereof.

**40.** A pharmaceutical comprising the polynucleotide according to claim 33.

**41.** An antibody to the protein according to claim 29 or the partial peptide according to claim 32, or a salt thereof.

**42.** A pharmaceutical comprising the antibody according to claim 41.

**43.** A diagnostic product comprising the antibody according to claim 41.

**44.** A polynucleotide containing a base sequence complementary or substantially complementary to the base sequence of the polynucleotide according to claim 33, or a part of the base sequence.

**45.** A pharmaceutical comprising the polypeptide according to claim 44.

**46.** A method of screening a compound or its salt that promotes or inhibits the activity of the protein according to claim 29 or the partial peptide according to claim 32, or a salt thereof, which comprises using the protein according to claim 29 or the partial peptide according to claim 32, or a salt thereof.

**47.** A kit for screening a compound or its salt that promotes or inhibits the activity of the protein according to claim 29 or the partial peptide according to claim 32, or a salt thereof, comprising the protein according to claim 29 or the partial peptide according to claim 32, or a salt thereof.

**48.** A compound or its salt that promotes or inhibits the activity of the protein according to claim 29 or the partial peptide according to claim 32, or a salt thereof, which is obtainable using the screening method according to claim 46 or the screening kit according to claim 47.

**49.** A pharmaceutical comprising the compound or its salt according to claim 48.

**50.** A method of screening a compound or its salt that promotes or inhibits the expression of a gene for the protein according to claim 29, which comprises using the polypeptide according to claim 33.

**51.** A kit for screening a compound or its salt that promotes or inhibits the expression of a gene for the protein according to claim 29, comprising the polypeptide according to claim 33.

**52.** A compound or its salt that promotes or inhibits the expression of a gene for the protein according to claim 29, which is obtainable using the screening method according to claim 50 or the screening kit according to claim 51.

**53.** A pharmaceutical comprising the compound or its salt according to claim 52.

**54.** The pharmaceutical according to claim 39, 40, 42, 45, 49 or 53, which is an agent for the prevention/treatment of central nervous system disorders, endocrine diseases or diabetes mellitus.

**55.** A method of preventing/treating central nervous system disorders, endocrine diseases or diabetes mellitus, which comprises administering an effective dose of the compound or its salt according to claim 48 or 52 to a mammal.

**56.** Use of the compound or its salt according to claim 48 or 52 for manufacturing an agent for the prevention/treatment of central nervous system disorders, endocrine diseases or diabetes mellitus.

**57.** A protein containing the same or substantially the same amino acid sequence as the amino acid sequence represented by SEQ ID NO: 62, or a salt thereof.

**58.** A protein consisting of the amino acid sequence represented by SEQ ID NO: 62, or a salt thereof.

**59.** A partial peptide of the protein according to claim 57, or a salt thereof.

**60.** A polynucleotide containing a polynucleotide encoding the protein according to claim 57, or the partial peptide according to claim 59.

**61.** The polynucleotide according to claim 60, which is a DNA.

**62.** A DNA consisting of the base sequence represented by SEQ ID NO: 63 or SEQ ID NO: 83.

**63.** A recombinant vector containing the polynucleotide according to claim 60.

**64.** A transformant transformed by the recombinant vector according to claim 63.

**65.** A method of manufacturing the protein according to claim 57 or the partial peptide according to claim 59, or a salt thereof, which comprises culturing the transformant according to claim 64, producing/accumulating the protein according to claim 57 or the partial peptide according to claim 59, and collecting the same.

**66.** A pharmaceutical comprising the protein according to claim 57 or the partial peptide according to claim 59, or a salt thereof.

**67.** A pharmaceutical comprising the polynucleotide according to claim 60.

**68.** An antibody to the protein according to claim 57 or the partial peptide according to claim 59, or a salt thereof.

**69.** A pharmaceutical comprising the antibody according to claim 68.

**70.** A diagnostic product comprising the antibody according to claim 68.

**71.** A polynucleotide containing a base sequence complementary or substantially complementary to the base sequence of the polynucleotide according to claim 60, or a part of the base sequence.

**72.** A pharmaceutical comprising the polypeptide according to claim 71.

**73.** A method of screening a compound or its salt that regulates the activity of the protein according to claim 57 or the partial peptide according to claim 59, or a salt thereof, which comprises using the protein according to claim 57 or the partial peptide according to claim 59, or a salt thereof.

**74.** A kit for screening a compound or its salt that regulates the activity of the protein according to claim 57 or the partial peptide according to claim 59, or a salt thereof, comprising the protein according to claim 57 or the partial peptide according to claim 59, or a salt thereof.

**75.** A compound or its salt that regulates the activity of the protein according to claim 57 or the partial peptide according to claim 59, or a salt thereof, which is obtainable using the screening method according to claim 73 or the screening kit according to claim 74.

**76.** A pharmaceutical comprising the compound or its salt according to claim 75.

**77.** A method of screening a compound or its salt that regulates the expression of a gene for the protein according to claim 57, which comprises using the polypeptide according to claim 60.

**78.** A kit for screening a compound or its salt that regulates the expression of a gene for the protein according to claim 57, comprising the polypeptide according to claim 60.

**79.** A compound or its salt that regulates the expression of a gene for the protein according to claim 57, which is obtainable using the screening method according to claim 77 or the screening kit according to claim 78.

**80.** A pharmaceutical comprising the compound or its salt according to claim 79.

**81.** The pharmaceutical according to claim 66, 67, 69, 72, 76 or 80, which is an agent for the prevention/treatment of central nervous system disorders or alimentary disorders.

**82.** A method of preventing/treating central nervous system disorders or alimentary disorders, which comprises administering an effective dose of the compound or its salt according to claim 75 or 79to a mammal.

**83.** Use of the compound or its salt according to claim 75 or 79 for manufacturing an agent for the prevention/treatment of central nervous system disorders or alimentary disorders.

# Fig. 1

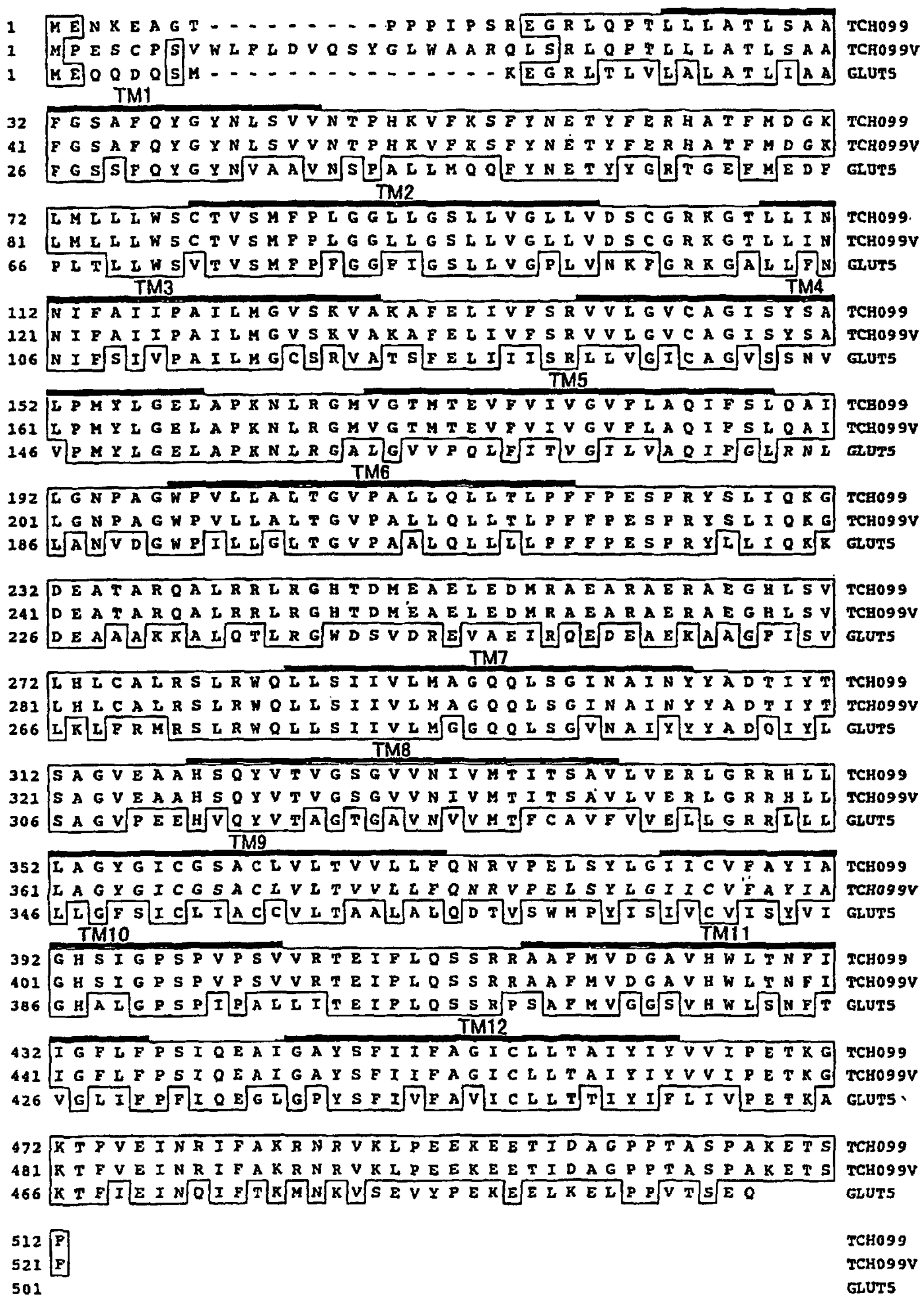

1 MENKEAGT---------PPPIPSREGRLQPTLLLATLSAA TCH099
1 MPESCPSVWLFLDVQSYGLWAARQLSRLQPTLLLATLSAA TCH099V
1 MEQQDQSM---------------KEGRLTLVLALATLIAA GLUT5
TM1
32 FGSAFQYGYNLSVVNTPHKVFKSFYNETYFERHATFMDGK TCH099
41 FGSAFQYGYNLSVVNTPHKVFKSFYNETYFERHATFMDGK TCH099V
26 FGSSFQYGYNVAAVNSPALLMQQFYNETYYGRTGEFMEDF GLUT5
TM2
72 LMLLLWSCTVSMFPLGGLLGSLLVGLLVDSCGRKGTLLIN TCH099
81 LMLLLWSCTVSMFPLGGLLGSLLVGLLVDSCGRKGTLLIN TCH099V
66 PLTLLWSVTVSMFPPFGGFIGSLLVGPLVNKFGRKGALLFN GLUT5
TM3
TM4
112 NIFAIIPAILMGVSKVAKAFELIVFSRVVLGVCAGISYSA TCH099
121 NIFAIIPAILMGVSKVAKAFELIVFSRVVLGVCAGISYSA TCH099V
106 NIFSIVPAILMGCSRVATSFELIIISRLLVGICAGVSSNV GLUT5
TM5
152 LPMYLGELAPKNLRGMVGTMTEVFVIVGVFLAQIFSLQAI TCH099
161 LPMYLGELAPKNLRGMVGTMTEVFVIVGVFLAQIFSLQAI TCH099V
146 VPMYLGELAPKNLRGALGVVPQLFITVGILVAQIFGLRNL GLUT5
TM6
192 LGNPAGWPVLLALTGVPALLQLLTLPFFPESPRYSLIQKG TCH099
201 LGNPAGWPVLLALTGVPALLQLLTLPFFPESPRYSLIQKG TCH099V
186 LANVDGWPILLGLTGVPAALQLLLLPFFPESPRYLLIQKK GLUT5
232 DEATARQALRRLRGHTDMEAELEDMRAEARAERAEGHLSV TCH099
241 DEATARQALRRLRGHTDMEAELEDMRAEARAERAEGHLSV TCH099V
226 DEAAAKKALQTLRGWDSVDREVAEIRQEDEAEKAAGPISV GLUT5
TM7
272 LHLCALRSLRWQLLSIIVLMAGQQLSGINAINYYADTIYT TCH099
281 LHLCALRSLRWQLLSIIVLMAGQQLSGINAINYYADTIYT TCH099V
266 LKLFRMRSLRWQLLSIIVLMGGQQLSGVNAIYYYADQIYL GLUT5
TM8
312 SAGVEAAHSQYVTVGSGVVNIVMTITSAVLVERLGRRHLL TCH099
321 SAGVEAAHSQYVTVGSGVVNIVMTITSAVLVERLGRRHLL TCH099V
306 SAGVPEEHVQYVTAGTGAVNVVMTFCAVFVVELLGRRLLL GLUT5
TM9
352 LAGYGICGSACLVLTVVLLFQNRVPELSYLGIICVFAYIA TCH099
361 LAGYGICGSACLVLTVVLLFQNRVPELSYLGIICVFAYIA TCH099V
346 LLGFSICLIACCVLTAALALQDTVSWMPYISIVCVISYVI GLUT5
TM10
TM11
392 GHSIGPSPVPSVVRTEIFLQSSRRAAFMVDGAVHWLTNFI TCH099
401 GHSIGPSPVPSVVRTEIFLQSSRRAAFMVDGAVHWLTNFI TCH099V
386 GHALGPSPIPALLITEIFLQSSRPSAFMVGGSVHWLSNFT GLUT5
TM12
432 IGFLFPSIQEAIGAYSFIIFAGICLLTAIYIYVVIPETKG TCH099
441 IGFLFPSIQEAIGAYSFIIFAGICLLTAIYIYVVIPETKG TCH099V
426 VGLIFPFIQEGLGPYSFIVFAVICLLTTIYIFLIVPETKA GLUT5
472 KTFVEINRIFAKRNRVKLPEEKEETIDAGPPTASPAKETS TCH099
481 KTFVEINRIFAKRNRVKLPEEKEETIDAGPPTASPAKETS TCH099V
466 KTFIEINQIFTKMNKVSEVYPEKEELKELPPVTSEQ GLUT5
512 F TCH099
521 F TCH099V
501 GLUT5

# Fig. 2

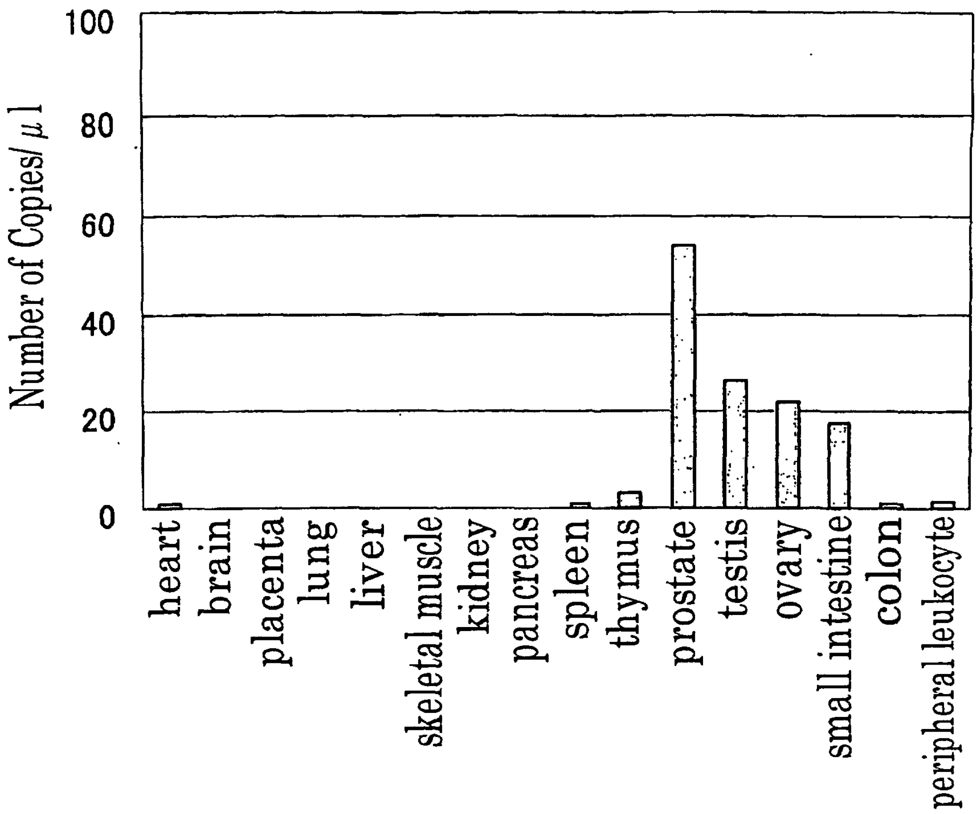
Number of Copies/μl
100
80
60
40
20
0
heart
brain
placenta
lung
liver
skeletal muscle
kidney
pancreas
spleen
thymus
prostate
testis
ovary
small intestine
colon
peripheral leukocyte

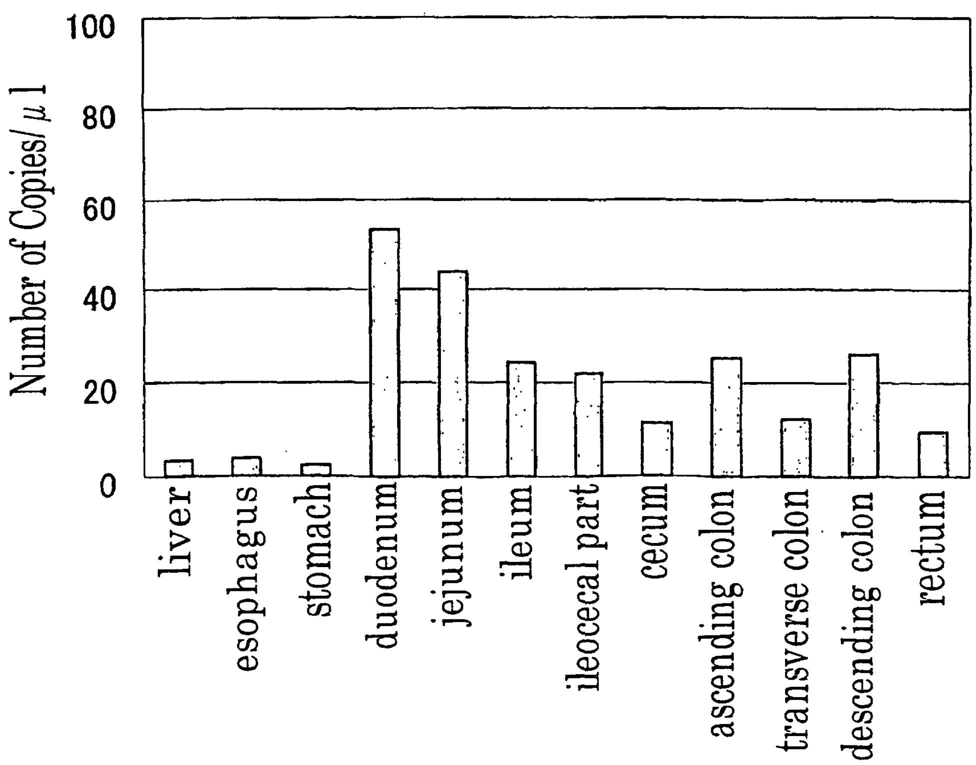
Number of Copies/μl
100
80
60
40
20
0
liver
esophagus
stomach
duodenum
jejunum
ileum
ileocecal part
cecum
ascending colon
transverse colon
descending colon
rectum

# Fig. 3

```
1   MEFR-----Q--------EEFRKLAGRALGKLHRLLEKRQ VGLUT1
1   MESV-----KQRILAPGKEGLKNFAGKSLGQIYRVLEKKQ VGLUT2
1   MPFKAPDTFKEKILKPGKEGVKNAVGDSLGILQRKIDGTT TCH177

28  EGAETLELSADGRPVTTQTRDPPVVDCTCFGLPRRYIIAI VGLUT1
36  DTGETIELTEDGKPLEVPERKAPLCDCTCFGLPRRYIIAI VGLUT2
41  EEEDNIELNEEGRPVQTSRPSPPLCDCHCCGLPKRYIIAI TCH177

68  MSGLGPCISFGIRCNLGVAIVSMVNNSTTHRGGHVVVQKA VGLUT1
76  MSGLGPCISFGIRCNLGVAIVDMVNNSTIHRGGKVIKEKA VGLUT2
81  MSGLGPCISFGIRCNLGVAIVEMVNNSTVYVDGKPEIQTA TCH177

108 QFSWDPETVGLIHGSPFWGYIVTQIPGGFICQKFAANRVF VGLUT1
116 KFNWDPETVGMIHGSPFWGYIITQIPGGYIASRLAANRVF VGLUT2
121 QFNWDPETVGLIHGSPFWGYIMTQIPGGFISNKFAANRVF TCH177
                                TM1
148 GFAIVATSTLNMLIPSAARVHYGCVIFVRILQGLVEGVTY VGLUT1
156 GAAILLTSTLNMLIPSAARVHYGCVIFVRILQGLVEGVTY VGLUT2
161 GAAIFLTSTLNMFIPSAARVHYGCVMCVRILQGLVEGVTY TCH177

188 PACHGIWSKWAPPLERSRLATTAFCGSYAGAVVAMPLAGV VGLUT1
196 PACHGIWSKWAPPLERSRLATTSFCGSYAGAVIAMPLAGI VGLUT2
201 PACHGMWSKWAPPLERSRLATTSFCGSYAGAVVAMPLAGV TCH177
          TM2
228 LVQYSGWSSVFYVYGSFGIFWYLFWLLVSYESPALHPSIS VGLUT1
236 LVQYTGWSSVFYVYGSFGMVWYMFWLLVSYESPAKHPTIT VGLUT2
241 LVQYIGWSSVFYIYGMFGIIWYMFWLLQAYECPAAHPTIS TCH177

268 EEERKYIEDAIGESAKLMNPLTKFSTPWRRFFTSMPVYAI VGLUT1
276 DEERRYIESSIGESANLLGAMEKFKTPWRKFFTSMPVYAI VGLUT2
281 NEEKTYIETSIGEGANVVS-LSKFSTPWKRFFTSLPVYAI TCH177
          TM3                        TM4
308 IVANFCRSWTFYLLLISQPAYFEEVFGFEISKVGLVSALP VGLUT1
316 IVANFCRSWTFYLLLISQPAYFEEVFGFEISKVGMLSAVP VGLUT2
320 IVANFCRSWTFYLLLISQPAYFEEVFGFAISKVGLLSAVP TCH177

348 HLVMTIIVPIGGQIADFLRSRRIMSTTNVRKLMNCGGFGM VGLUT1
356 HLVMTIIVPIGGQIADFLRSKQILSTTTVRKIMNCGGFGM VGLUT2
360 HMVMTIVVPIGGQLADYLRSRQILTTTAVRKIMNCGGFGM TCH177
        TM5                 TM6
388 EATLLLVVGYSHSKGVAISFLVLAVGFSGFAISGFNVNHL VGLUT1
396 EATLLLVVGYSHTRGVAISFLVLAVGFSGFAISGFNVNHL VGLUT2
400 EATLLLVVGFSHTKGVAISFLVLAVGFSGFAISGFNVNHL TCH177

428 DIAPRYASILMGISNGVGTLSGMVCPIIVGAMTKHKTREE VGLUT1
436 DIAPRYASILMGISNGVGTLSGMVCPIIVGAMTKNKSREE VGLUT2
440 DIAPRYASILMGISNGVGTLSGMVCPLIVGAMTRHKTREE TCH177
              TM7
468 WQYVFLIASLVHYGGVIFYGVFASGEKQPWAEPEEMSEEK VGLUT1
476 WQYVFLIAALVHYGGVIFYAIFASGEKQPWADPEETSEEK VGLUT2
480 WQNVFLIAALVHYSGVIFYGVFASGEKQEWADPENLSEEK TCH177

508 CGFVGHDQLAGSDDSEMED-----EAEPPGAPPAPPPSYG VGLUT1
516 CGFIHEDELDEETGDITQNYINYGTTKSYGATTQANGGWP VGLUT2
520 CGIIDQDELAEEIELNHESFASPKKKMSYGATSQNCEVQK TCH177

543 AT----------HSTFQP-PRPPPPVRDY            VGLUT1
556 SGW--EKKEEPVQGEVQD-SHSYKDRVDYS           VGLUT2
560 KEWKGQRGATLDEEELTSYQNEERNFSTIS           TCH177
```

Fig. 4

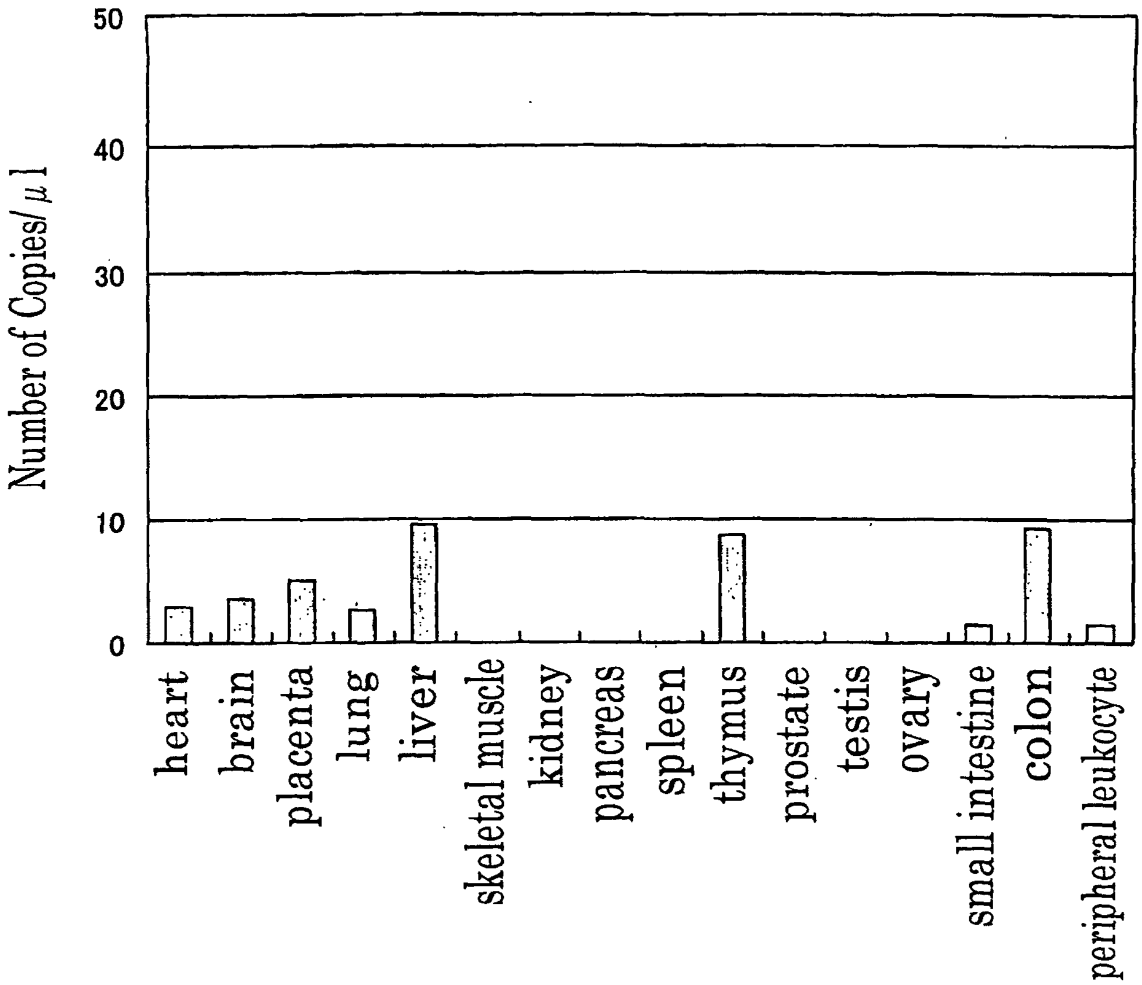
Number of Copies/μl
50
40
30
20
10
0
heart
brain
placenta
lung
liver
skeletal muscle
kidney
pancreas
spleen
thymus
prostate
testis
ovary
small intestine
colon
peripheral leukocyte

# Fig. 5

```
1   M P F K A F D T F K E K I L K P G K E G V K N A V G D S L G I L Q R K I D G T T   hTCH177
1   M P F K A F D T F K E K I L K P G K E G V K N A V G D S L G I L Q R K I D G T N   mTCH177

41  E E E D N I E L N E E G R P V Q T S R P S P P L C D C H C C G L P K R Y I - - -   hTCH177
41  E E E D A I E L N E E G R P V Q T S R A H R P V C D C S C C G I P K R Y I C D C   mTCH177

78  - - - - - - - - - - I A I M S G L G F C I S F G I R C N L G V A I V E M V N N S   hTCH177
81  S C C G I P K R Y I I A V M S G L G F C I S F G I R C N L G V A I V E M V N N S   mTCH177

108 T V Y V D G K P E I Q T A Q F N W D P E T V G L I H G S F F W G Y I M T Q I P G   hTCH177
121 T V Y V D G K P E I Q T A Q F N W D P E T V G L I H G S F F W G Y I V T Q I P G   mTCH177

148 G F I S N K F A A N R V F G A A I F L T S T L N M F I P S A A R V H Y G C V M C   hTCH177
161 G F I S N K F A A S R V F G A A I F L T S T L N M F I P S A A R V H Y G C V M G   mTCH177

188 V R I L Q G L V E G V T Y P A C H G M W S K W A P P L E R S R L A T T S F C G S   hTCH177
201 V R I L Q G L V E G V T Y P A C H G M W S K W A P P L E R S R L A T T S F C G S   mTCH177

228 Y A G A V V A M P L A G V L V Q Y I G W S S V F Y I Y G M F G I I W Y M F W L L   hTCH177
241 Y A G A V V A M P L A G V L V Q Y I G W A S V F Y I Y G M F G I I W Y M F W L L   mTCH177

268 Q A Y E C P A A H P T I S N E E K T Y I E T S I G E G A N V V S L S K F S T P W   hTCH177
281 Q A Y E C P A A H P T I S N A E R T Y I E T S I G E G A N L A S L S K F N T P W   mTCH177

308 K R F F T S L P V Y A I I V A N F C R S W T F Y L L L I S Q P A Y F E E V F G F   hTCH177
321 R R F F T S L P V Y A I I V A N F C R S W T F Y L L L I S Q P A Y F E E V F G F   mTCH177

348 A I S K V G L L S A V P H M V M T I V V P I G G Q L A D Y L R S R Q I L T T T A   hTCH177
361 A I S K V G L L S A V P H M V M T I V V P I G G Q L A D Y L R S R K I L T T T A   mTCH177

388 V R K I M N C G G F G M E A T L L L V V G F S H T K G V A I S F L V L A V G F S   hTCH177
401 V R K I M N C G G F G M E A T L L L V V G F S H T K G V A I S F L V L A V G F S   mTCH177

428 G F A I S G F N V N H L D I A P R Y A S I L M G I S N G V G T L S G M V C P L I   hTCH177
441 G F A I S G F N V N H L D I A P R Y A S I L M G I S N G V G T L S G M V C P L I   mTCH177

468 V G A M T R H K T R E E W Q N V F L I A A L V H Y S G V I F Y G V F A S G E K Q   hTCH177
481 V G A M T K H K T R E E W Q N V F L I A A L V H Y S G V I F Y G V F A S G E K Q   mTCH177

508 E W A D P E N L S E E K C G I I D Q D E L A E E I E L N H E S F A S P K K K M S   hTCH177
521 D W A D P E N L S E D K C G I I D Q D E L A E E T E L N H E T F V S P R K K M S   mTCH177

548 Y G A T S Q N C E V Q K K E W K G Q R G A T L D E E E L T S Y Q N E E R N F S T   hTCH177
561 Y G A T T Q N C E V Q K T E W R Q Q R E S A F D G E E P L S Y Q A E G - D F S E   mTCH177

588 I S                                                                              hTCH177
600 T S                                                                              mTCH177
```

Fig. 6

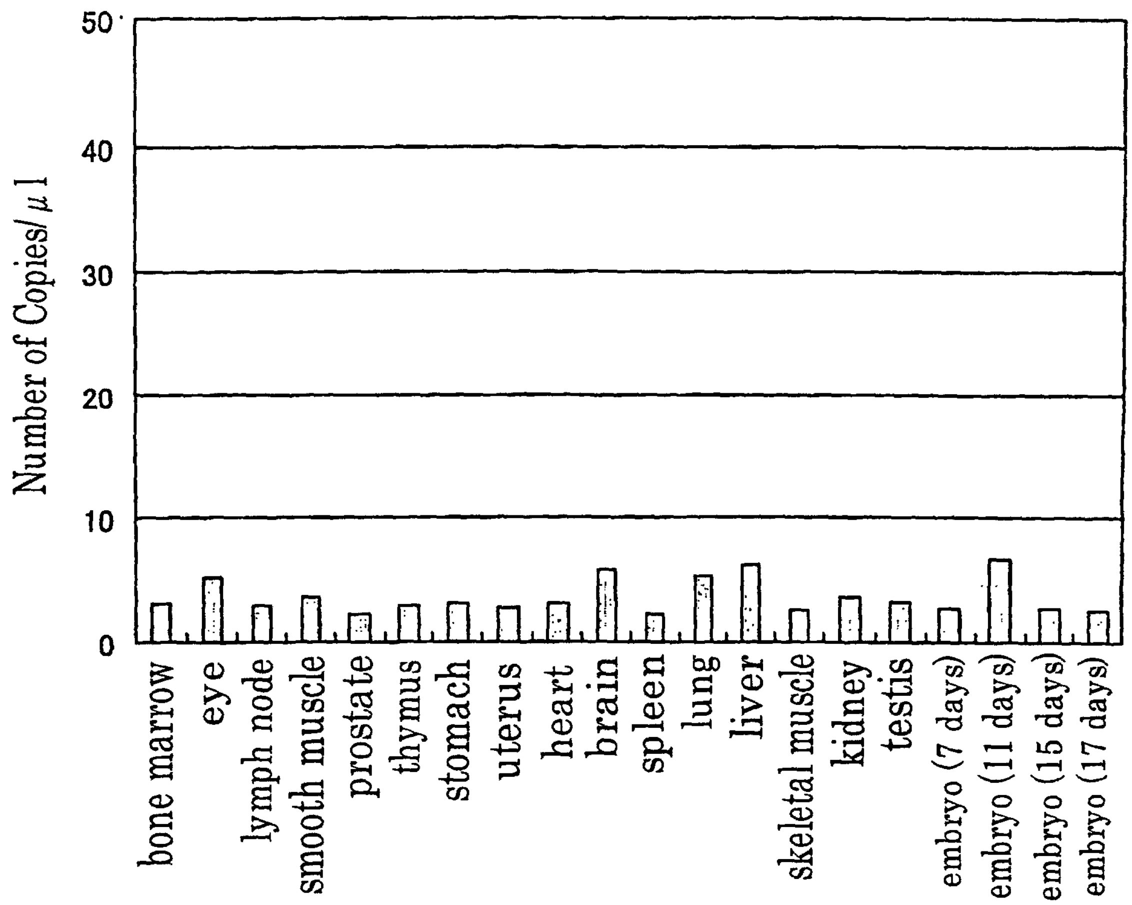
50
40
30
20
10
0
Number of Copies/μl
bone marrow
eye
lymph node
smooth muscle
prostate
thymus
stomach
uterus
heart
brain
spleen
lung
liver
skeletal muscle
kidney
testis
embryo (7 days)
embryo (11 days)
embryo (15 days)
embryo (17 days)

# Fig. 7

```
1   M T - - - F G R S G A A S - - - - - V V L N V G G A R Y S L S R E L L K D F P L  Tch136
1   M E P W P C S P G G G G G T R A R H V I I N V G G C R V R L A W A A L A R C P L  Kv6.2

33  R R V S R L H G C R S E R D V L E V C D D Y D R E R N E Y F F D R H S E A F G F  Tch136
41  A R L E R L R A C R G H D D L L R V C D D Y D V S R D E F F F D R S P C A F R A  Kv6.2

73  I L L Y V R G H G K L R F A P R M C E L S F Y N E M I Y W G L E G A H L E Y C C  Tch136
81  I V A L L R A - G K L R L L R G P C A L A F R D E L A Y W G I D E A R L E R C C  Kv6.2

113 Q R R L D D R M S D T Y T F Y S A D E P G V L G R D E A R P G G A E A A P S R R  Tch136
120 L R R L R R R E E E A - - - - - A E A R A G P T E R G A Q G S P A R A L G P R G  Kv6.2
                                                                   TM1
153 W L E R M R R T F - - - - E E P T S S L A A Q I L A S V S V V F V I V S M V V L  Tch136
155 R L Q R G R R R L R D V V D N P H S G L A G K L F A C V S V S F V A V T A V G L  Kv6.2

189 C A S T L P D W R N A A A D N R S L D D R S R Y S A G P G R E P S G I I E A I C  Tch136
195 C L S T M P D - - - - - - - I R A E E E R G E C S - - P K C R S L F V L E T V C  Kv6.2
          TM2                                                               TM3
229 I G W F T A E C I V R F I V S K N K C E F V K R P L N I I D L L A I T P Y Y I S  Tch136
226 V A W F S F E F L L R S L Q A E S K C A F L R A P L N I I D I L A L L P F Y V S  Kv6.2
                                                                   TM4
269 V L M T V F T G E N S Q - - L Q R A G V T L R V L R M M R I F W V I K L A R H F  Tch136
266 L L L G L A A G P G G T K L L E R A G L V L R L L R A L R V L Y V M R L A R H S  Kv6.2
                                                     TM5
307 I G L Q T L G L T L K R C Y R E M V M L L V F I C V A M A I F S A L S Q L L E H  Tch136
306 L G L R S L G L T M R R C A R E F G L L L L F L C V A M A L F A P L V H L A E R  Kv6.2
                                                     P
347 G L D L E T S N K D F T S I P A A C W W V I I S M T T V G Y G D M Y P I T V P G  Tch136
346 E L G - - - A R R D F S S V P A S Y W W A V I S M T T V G Y G D M V P R S L P G  Kv6.2
                         TM6
387 R I L G G V C V V S G I V L L A L P I T F I Y H S F V Q C Y H E L K F R S A R Y  Tch136
383 Q V V A L S S I L S G I L L M A F P V T S I F H T F S R S Y S E L K E Q Q Q R A  Kv6.2

427 S R S - - - - - - - - - - - - - - - - - - - - - - - - - L S T E F L N            Tch136
423 A S P E P A L Q E D S T H S A T A T E D S S Q G P D S A G L A D D S A D A L W V  Kv6.2

436                                                                                  Tch136
463 R A G R                                                                          Kv6.2
```

Fig. 8

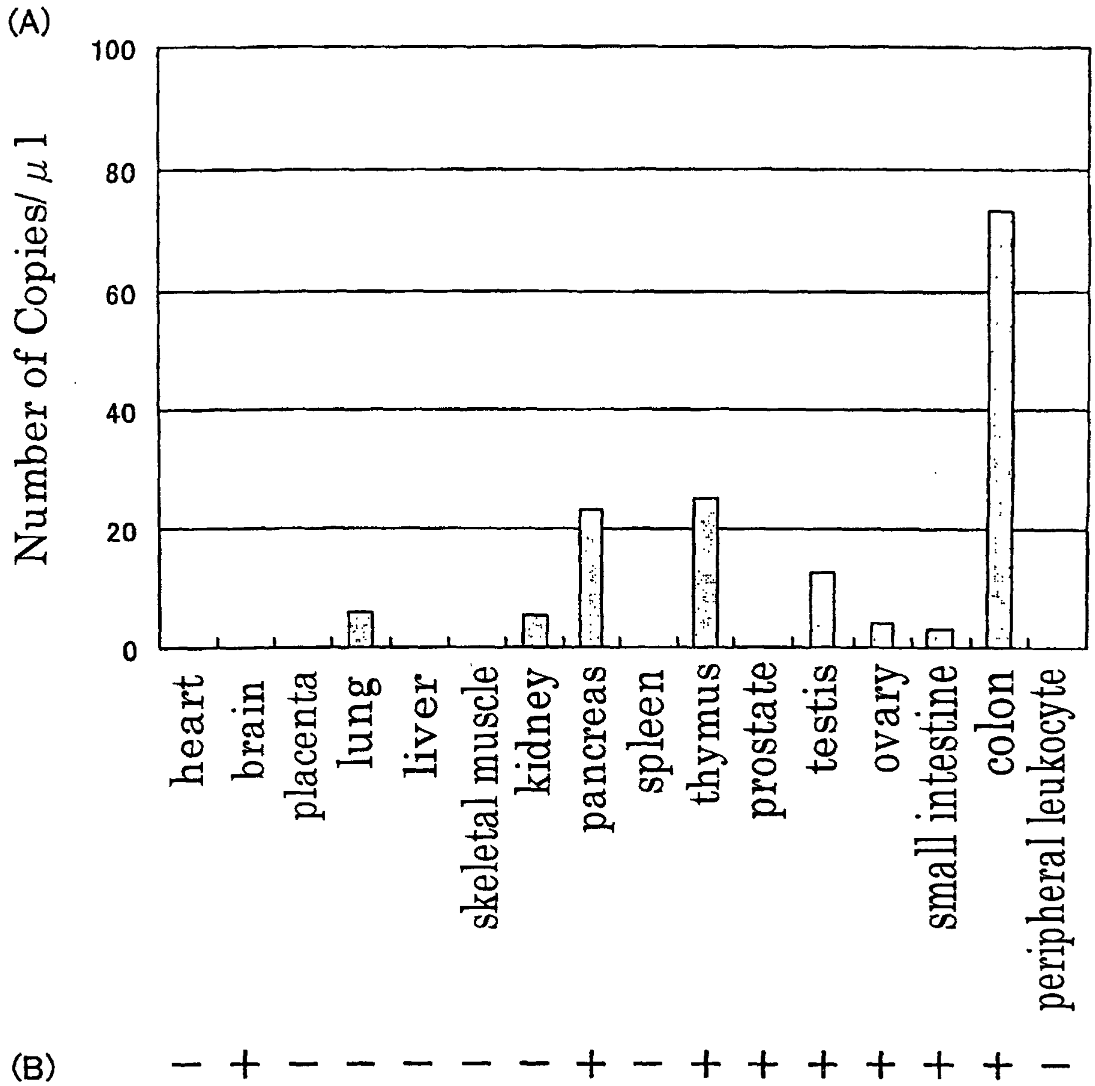
(A)
Number of Copies/μ1
100
80
60
40
20
0
heart
brain
placenta
lung
liver
skeletal muscle
kidney
pancreas
spleen
thymus
prostate
testis
ovary
small intestine
colon
peripheral leukocyte
(B)
− + − − − − − + − + + + + + + −

# Fig. 9

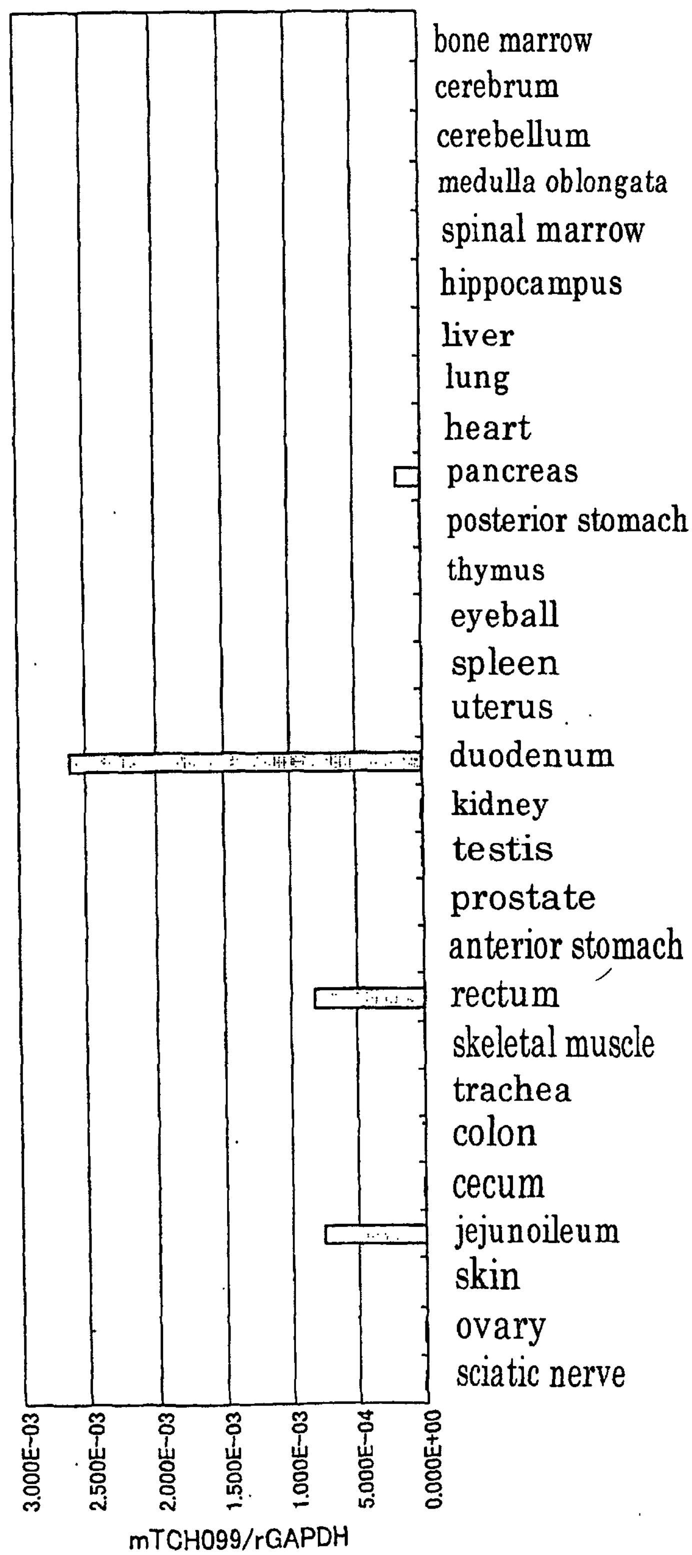
bone marrow
cerebrum
cerebellum
medulla oblongata
spinal marrow
hippocampus
liver
lung
heart
pancreas
posterior stomach
thymus
eyeball
spleen
uterus
duodenum
kidney
testis
prostate
anterior stomach
rectum
skeletal muscle
trachea
colon
cecum
jejunoileum
skin
ovary
sciatic nerve
3.000E-03
2.500E-03
2.000E-03
1.500E-03
1.000E-03
5.000E-04
0.000E+00
mTCH099/rGAPDH

Fig. 10

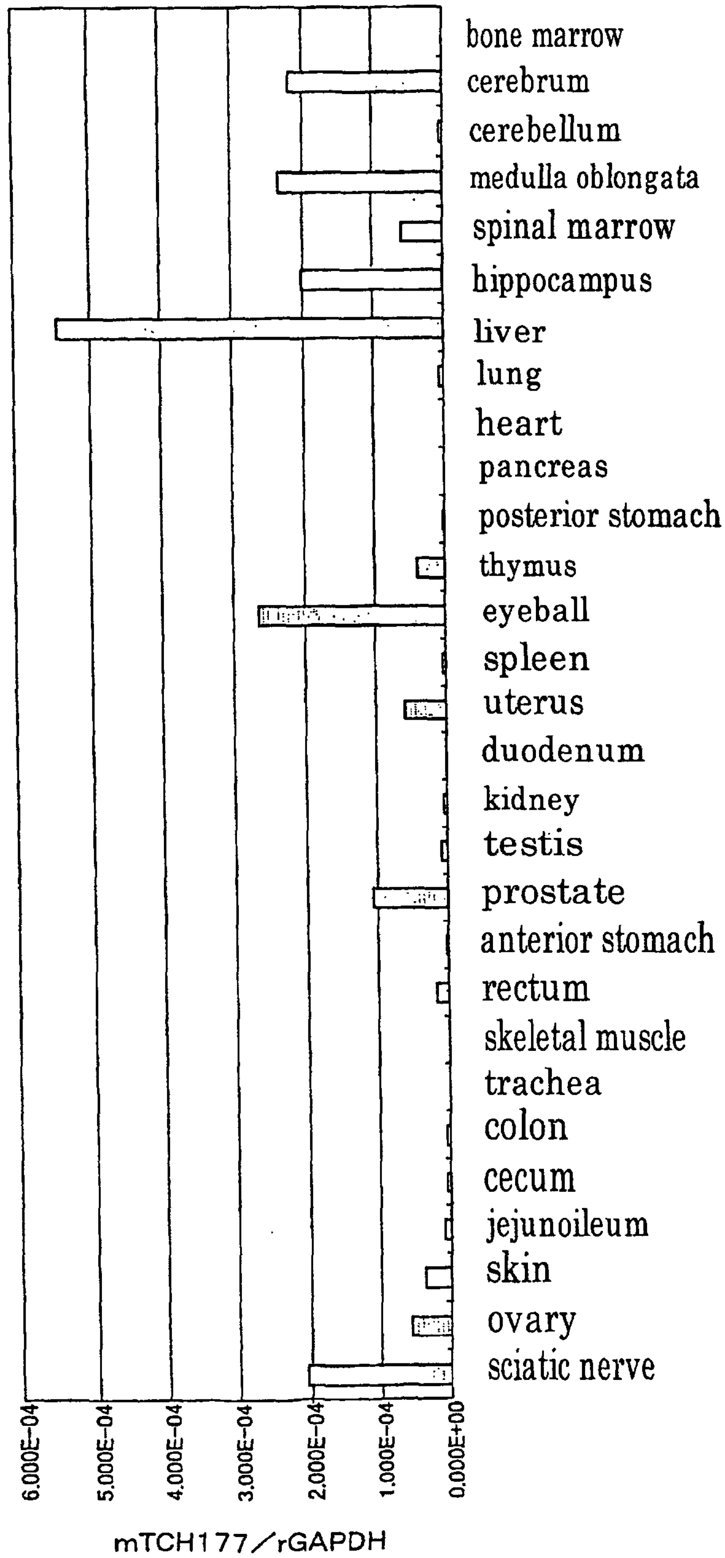
bone marrow
cerebrum
cerebellum
medulla oblongata
spinal marrow
hippocampus
liver
lung
heart
pancreas
posterior stomach
thymus
eyeball
spleen
uterus
duodenum
kidney
testis
prostate
anterior stomach
rectum
skeletal muscle
trachea
colon
cecum
jejunoileum
skin
ovary
sciatic nerve
6.000E-04
5.000E-04
4.000E-04
3.000E-04
2.000E-04
1.000E-04
0.000E+00
mTCH177／rGAPDH

Fig. 11

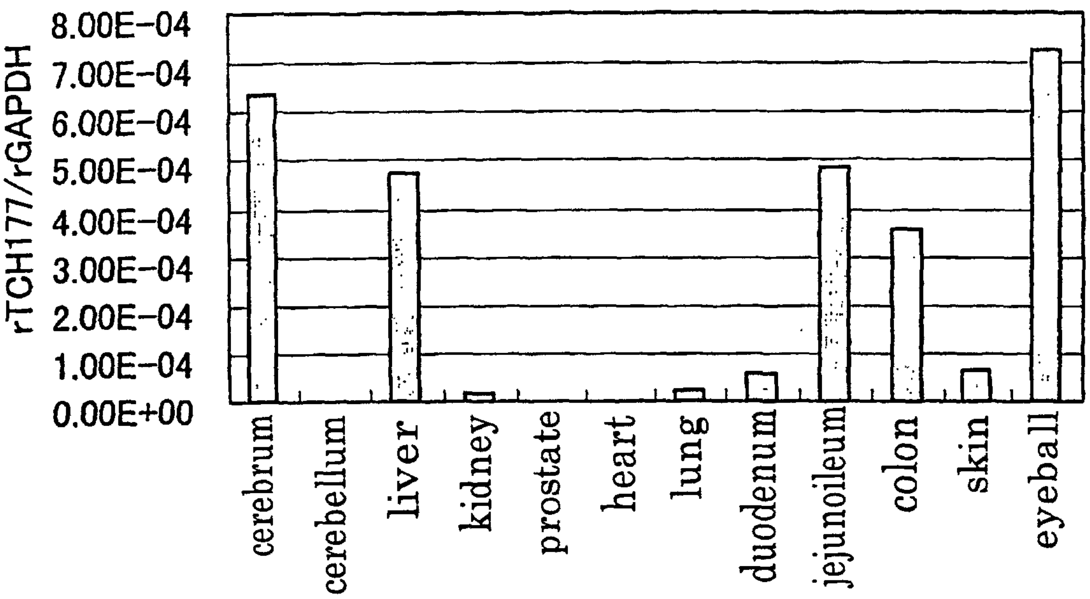
rTCH177/rGAPDH
8.00E-04
7.00E-04
6.00E-04
5.00E-04
4.00E-04
3.00E-04
2.00E-04
1.00E-04
0.00E+00
cerebrum
cerebellum
liver
kidney
prostate
heart
lung
duodenum
jejunoileum
colon
skin
eyeball

Fig. 12

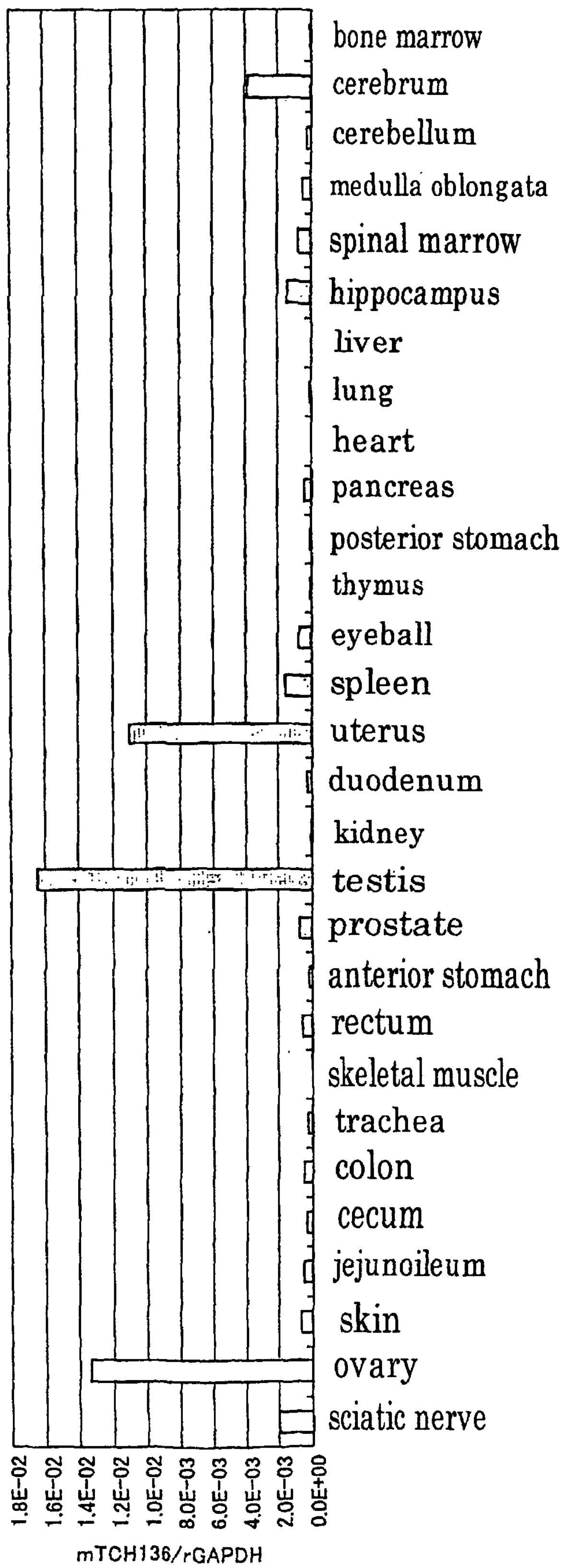
bone marrow
cerebrum
cerebellum
medulla oblongata
spinal marrow
hippocampus
liver
lung
heart
pancreas
posterior stomach
thymus
eyeball
spleen
uterus
duodenum
kidney
testis
prostate
anterior stomach
rectum
skeletal muscle
trachea
colon
cecum
jejunoileum
skin
ovary
sciatic nerve
1.8E-02
1.6E-02
1.4E-02
1.2E-02
1.0E-02
8.0E-03
6.0E-03
4.0E-03
2.0E-03
0.0E+00
mTCH136/rGAPDH

Fig. 13

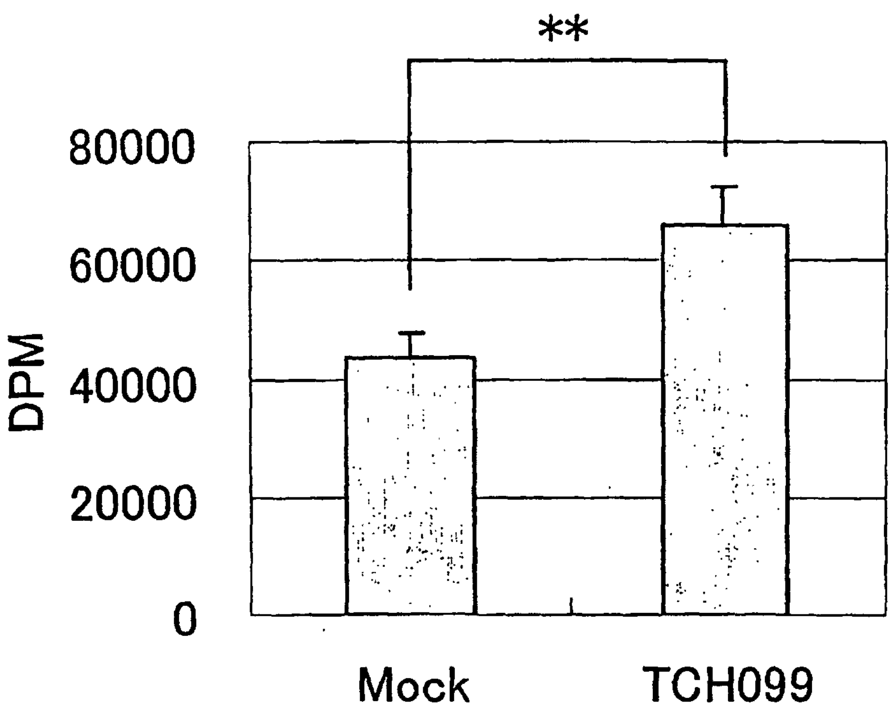

**INTERNATIONAL SEARCH REPORT**

International application No.
PCT/JP02/13290

A. CLASSIFICATION OF SUBJECT MATTER
Int.Cl$^7$ C12N15/09, C12N15/12, C07K14/705, C07K16/28, C12N1/15, C12N1/19, C12N1/21, C12N5/10, C12P21/02, G01N33/50, G01N33/15, A61K38/17, A61K39/395, A61K45/00, A61K48/00,

According to International Patent Classification (IPC) or to both national classification and IPC

B. FIELDS SEARCHED

Minimum documentation searched (classification system followed by classification symbols)
Int.Cl$^7$ C12N15/09, C12N15/12, C07K14/705, C07K16/28, C12N1/15, C12N1/19, C12N1/21, C12N5/10, C12P21/02, G01N33/50, G01N33/15

Documentation searched other than minimum documentation to the extent that such documents are included in the fields searched

Electronic data base consulted during the international search (name of data base and, where practicable, search terms used)
BIOSIS/WPI(DIALOG), MEDLINE(STN), SwissProt/PIR/GeneSeq, GenBank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X | Kayano T. et al., Human facilitative glucose transporters. Isolation, functional characterization, and gene localization of cDNAs encoding an isoform (GLUT5) expressed in small intestine, kidney, muscle, and adipose tissue and an unusual glucose transporter pseudogene-like sequence (GLUT6), J.Biol.Chem., 1990, Vol.265, No.22, pages 13276 to 13282 | 1-19,22-23, 26 |
| P,X | WO 02/04520 A2 (INCYTE GENOMICS, INC.), 17 January, 2002 (17.01.02), SEQ ID NOS: 7, 39 & AU 200173239 A | 1-19,22-23, 26 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 March, 2003 (24.03.03) | 08 April, 2003 (08.04.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13290

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 02/57453 A2 (CURAGEN CORP.), 25 July, 2002 (25.07.02), SEQ ID NOS:23, 24 (Family: none) | 1-19,22-23, 26 |
| P,X | WO 02/46406 A2 (PE CORP.), 13 June, 2002 (13.06.02), SEQ ID NOS:1, 2 & US 2002/0103337 A & AU 200232452 A | 1-19,22-23, 26 |

Form PCT/ISA/210 (continuation of second sheet) (July 1998)

**INTERNATIONAL SEARCH REPORT**

International application No.
PCT/JP02/13290

**Box I Observations where certain claims were found unsearchable (Continuation of item 2 of first sheet)**

This international search report has not been established in respect of certain claims under Article 17(2)(a) for the following reasons:

1. [X] Claims Nos.: 27
because they relate to subject matter not required to be searched by this Authority, namely:
The invention as set forth in the above claim pertains to methods for treatment of the human body by therapy and thus relates to a subject matter which this International Searching Authority is not required to search.

2. [X] Claims Nos.: 20-21, 24-25, 28
because they relate to parts of the international application that do not comply with the prescribed requirements to such an extent that no meaningful international search can be carried out, specifically:
Concerning the "compound" as set forth in the above claims, the description declares no specific "compound" but merely a general screening method. Although the common technical knowledge at the point of the application is taken into consideration, it is completely unknown (continued to extra sheet)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
The inventions as set forth in claims 1 to 28 relate to proteins (glucose transporters) comprising amino acids represented by SEQ ID NO:1 or SEQ ID NO:23. On the other hand, the inventions as set forth in claims 29 to 56 relate to proteins (vesicular glutamate transporters) comprising amino acids represented by SEQ ID NO:28 or SEQ ID NO:46. The inventions as set forth in claims 57 to 83 relate to a protein (potential-dependent $K^+$ channel protein) comprising the amino acids represented by SEQ ID NO:62. Although these proteins are common to each other in being a "specific transporter", there had been publicly known various specific transporters as reported by, for example, J. Biol Chem, 1990, Vol.265, No.22, p.13276-13282 (continued to extra sheet)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.: 1 to 28

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/13290

Continuation of A. CLASSIFICATION OF SUBJECT MATTER
(International Patent Classification (IPC))

Int.Cl[7] A61P3/06, A61P3/10, A61P9/10, A61P25/00, A61P1/00

(According to International Patent Classification (IPC) or to both national classification and IPC)

Continuation of Box No.I-2 of continuation of first sheet(1)

what specific compounds are involved in the scope thereof. Such being the case, the above claims are described in an unclear manner and thus no meaningful international search can be made thereon.

Continuation of Box No.II of continuation of first sheet(1)

and thus the "specific transporter" cannot be considered as a special technical feature.

Thus, it is considered that the inventions as set forth in claims 1 to 83 have no technical relationship involving any special technical feature. Also, these inventions are not regarded as relating to a group of inventions so linked as to form a single general inventive concept.

Such being the case, it is recognized that the present application has 3 groups of inventions in its claims.

Form PCT/ISA/210 (extra sheet) (July 1998)

SEQUENCE LISTING
<110> Takeda Chemical Industries, Ltd.

<120> Novel Proteins And DNAs Thereof

<130> 2994EPOW

<150> JP 2001-389361
<151> 2001-12-21

<150> JP 2001-392577
<151> 2001-12-25

<150> JP 2001-394947
<151> 2001-12-26

<150> JP 2001-395467
<151> 2001-12-26

<150> JP 2002-30010
<151> 2002-02-06

<150> JP 2002-33095
<151> 2001-02-08

<150> JP 2002-165336
<151> 2002-06-06

<160> 108

<210> 1
<211> 512
<212> PRT
<213> Homo sapiens

<400> 1
```
Met Glu Asn Lys Glu Ala Gly Thr Pro Pro Pro Ile Pro Ser Arg Glu
                5                   10                  15
Gly Arg Leu Gln Pro Thr Leu Leu Leu Ala Thr Leu Ser Ala Ala Phe
            20                  25                  30
Gly Ser Ala Phe Gln Tyr Gly Tyr Asn Leu Ser Val Val Asn Thr Pro
        35                  40                  45
His Lys Val Phe Lys Ser Phe Tyr Asn Glu Thr Tyr Phe Glu Arg His
    50                  55                  60
Ala Thr Phe Met Asp Gly Lys Leu Met Leu Leu Leu Trp Ser Cys Thr
65                  70                  75                  80
Val Ser Met Phe Pro Leu Gly Gly Leu Leu Gly Ser Leu Leu Val Gly
                85                  90                  95
Leu Leu Val Asp Ser Cys Gly Arg Lys Gly Thr Leu Leu Ile Asn Asn
            100                 105                 110
Ile Phe Ala Ile Ile Pro Ala Ile Leu Met Gly Val Ser Lys Val Ala
        115                 120                 125
Lys Ala Phe Glu Leu Ile Val Phe Ser Arg Val Val Leu Gly Val Cys
    130                 135                 140
Ala Gly Ile Ser Tyr Ser Ala Leu Pro Met Tyr Leu Gly Glu Leu Ala
145                 150                 155                 160
Pro Lys Asn Leu Arg Gly Met Val Gly Thr Met Thr Glu Val Phe Val
                165                 170                 175
Ile Val Gly Val Phe Leu Ala Gln Ile Phe Ser Leu Gln Ala Ile Leu
            180                 185                 190
Gly Asn Pro Ala Gly Trp Pro Val Leu Leu Ala Leu Thr Gly Val Pro
        195                 200                 205
Ala Leu Leu Gln Leu Leu Thr Leu Pro Phe Phe Pro Glu Ser Pro Arg
    210                 215                 220
Tyr Ser Leu Ile Gln Lys Gly Asp Glu Ala Thr Ala Arg Gln Ala Leu
225                 230                 235                 240
Arg Arg Leu Arg Gly His Thr Asp Met Glu Ala Glu Leu Glu Asp Met
                245                 250                 255
```

Arg Ala Glu Ala Arg Ala Glu Arg Ala Glu Gly His Leu Ser Val Leu
260 265 270
His Leu Cys Ala Leu Arg Ser Leu Arg Trp Gln Leu Leu Ser Ile Ile
275 280 285
Val Leu Met Ala Gly Gln Gln Leu Ser Gly Ile Asn Ala Ile Asn Tyr
290 295 300
Tyr Ala Asp Thr Ile Tyr Thr Ser Ala Gly Val Glu Ala Ala His Ser
305 310 315 320
Gln Tyr Val Thr Val Gly Ser Gly Val Val Asn Ile Val Met Thr Ile
325 330 335
Thr Ser Ala Val Leu Val Glu Arg Leu Gly Arg Arg His Leu Leu Leu
340 345 350
Ala Gly Tyr Gly Ile Cys Gly Ser Ala Cys Leu Val Leu Thr Val Val
355 360 365
Leu Leu Phe Gln Asn Arg Val Pro Glu Leu Ser Tyr Leu Gly Ile Ile
370 375 380
Cys Val Phe Ala Tyr Ile Ala Gly His Ser Ile Gly Pro Ser Pro Val
385 390 395 400
Pro Ser Val Val Arg Thr Glu Ile Phe Leu Gln Ser Ser Arg Arg Ala
405 410 415
Ala Phe Met Val Asp Gly Ala Val His Trp Leu Thr Asn Phe Ile Ile
420 425 430
Gly Phe Leu Phe Pro Ser Ile Gln Glu Ala Ile Gly Ala Tyr Ser Phe
435 440 445
Ile Ile Phe Ala Gly Ile Cys Leu Leu Thr Ala Ile Tyr Ile Tyr Val
450 455 460
Val Ile Pro Glu Thr Lys Gly Lys Thr Phe Val Glu Ile Asn Arg Ile
465 470 475 480
Phe Ala Lys Arg Asn Arg Val Lys Leu Pro Glu Glu Lys Glu Glu Thr
485 490 495
Ile Asp Ala Gly Pro Pro Thr Ala Ser Pro Ala Lys Glu Thr Ser Phe
500 505 510

<210> 2
<211> 1536
<212> DNA
<213> Homo sapiens

<400> 2
atggagaaca aagaggcggg aacccctcca cccattccat ccagggaggg gcggctccag 60
ccgacgctgt tgctggcgac actgagcgcg gcctttggct cagccttcca gtacggctac 120
aacctctctg tggtcaacac gccgcacaag gtcttcaagt cattttacaa cgaaacctac 180
tttgagcgac acgcaacatt catggacggg aagctcatgc tgcttctatg gtcttgcacc 240
gtctccatgt ttcctctggg cggcctgttg ggtcattgc tcgtgggcct gctggttgat 300
agctgcggca gaaaggggac cctgctgatc aacaacatct ttgccatcat ccccgccatc 360
ctgatgggag tcagcaaagt ggccaaggct tttgagctga tcgtcttttc ccgagtggtg 420
ctgggagtct gtgcaggcat ctcctacagc gcccttccca tgtacctggg agaactggcc 480
cccaagaacc tgagaggcat ggtgggaaca atgaccgagg ttttcgtcat cgttggagtc 540
ttcctagcac agatcttcag cctccaggcc atcttgggca acccggcagg ctggccggtg 600
cttctggcgc tcacaggggt gcccgccctg ctgcagctgc tgaccctgcc cttcttcccc 660
gaaagccccc gctactccct gattcagaaa ggagatgaag ccacagcgcg acaagctctg 720
aggaggctga gaggccacac ggacatggag gccgagctgg aggacatgcg tgcggaggcc 780
cgggccgagc gcgccgaggg ccacctgtct gtgctgcacc tctgtgccct gcggtccctg 840
cgctggcagc tcctctccat catcgtgctc atggccggcc agcagctgtc gggcatcaat 900
gcgatcaact actatgcgga caccatctac acatctgcgg gcgtggaggc cgctcactcc 960
caatatgtaa cggtgggctc tggcgtcgtc aacatagtga tgaccatcac ctcggctgtc 1020
cttgtggagc ggctgggacg gcggcacctc ctgctggccg gctacggcat ctgcggctct 1080
gcctgcctgg tgctgacggt ggtgctccta ttccagaaca gggtccccga gctgtcctac 1140
ctcggcatca tctgtgtctt tgcctacatc gcgggacatt ccattgggcc cagtcctgtc 1200
ccctcggtgg tgaggaccga gatcttcctg cagtcctccc ggcgggcagc tttcatggtg 1260
gacggggcag tgcactggct caccaacttc atcataggct tcctgttccc atccatccag 1320
gaggccatcg gtgcctacag tttcatcatc tttgccggaa tctgcctcct cactgcgatt 1380
tacatctacg tggttattcc ggagaccaag ggcaaaacat ttgtggagat aaaccgcatt 1440
tttgccaaga gaaacagggt gaagcttcca gaggagaaag aagaaaccat tgatgctggg 1500
cctcccacag cctctcctgc caaggaaact tccttt 1536

<210> 3
<211> 26

<212> DNA
<213> Artificial Sequence

<220>
<223> Primer

<400> 3
agcaggtgtc acttgggagg tagagc 26

<210> 4
<211> 26
<212> DNA
<213> Artificial Sequence

<220>
<223> Primer

<400> 4
tggtgccaat gtagaatcgg aaaatc 26

<210> 5
<211> 24
<212> DNA
<213> Artificial Sequence

<220>
<223> Primer

<400> 5
ggaggcccat tgtcatagaa ggaa 24

<210> 6
<211> 18
<212> DNA
<213> Artificial Sequence

<220>
<223> Primer

<400> 6
atttaggtga cactatag 18

<210> 7
<211> 19
<212> DNA
<213> Artificial Sequence

<220>
<223> Primer

<400> 7
aatacgactc actataggg 19

<210> 8
<211> 20
<212> DNA
<213> Artificial Sequence

<220>
<223> Primer

<400> 8
tcttttcccg agtggtgctg 20

<210> 9
<211> 26
<212> DNA
<213> Artificial Sequence

<220>
<223> Primer

<400> 9
agctgtccta cctcggcatc atctgt 26

<210> 10
<211> 21
<212> DNA
<213> Artificial Sequence

<220>
<223> Primer

<400> 10
cctctccatc atcgtgctca t 21

<210> 11
<211> 24
<212> DNA
<213> Artificial Sequence
<220>
<223> Primer

<400> 11
cctgttctgg aataggagca ccac 24

<210> 12
<211> 21
<212> DNA
<213> Artificial Sequence

<220>
<223> Primer

<400> 12
gaagactcca acgatgacga a 21

<210> 13
<211> 26
<212> DNA
<213> Artificial Sequence

<220>
<223> Primer

<400> 13
acgcccgcag atgtgtagat ggtgtc 26

<210> 14
<211> 1652
<212> DNA
<213> Homo sapiens

<400> 14
agcaggtgtc acttgggagg tagagcacac ctgttctccc acctgaacaa ggatggagaa 60
caaagaggcg ggaacccctc cacccattcc atccagggag gggcggctcc agccgacgct 120
gttgctggcg acactgagcg cggcctttgg ctcagccttc cagtacggct acaacctctc 180
tgtggtcaac acgccgcaca aggtcttcaa gtcattttac aacgaaacct actttgagcg 240
acacgcaaca ttcatggacg ggaagctcat gctgcttcta tggtcttgca ccgtctccat 300
gtttcctctg ggcggcctgt tggggtcatt gctcgtgggc ctgctggttg atagctgcgg 360
cagaaagggg accctgctga tcaacaacat ctttgccatc atccccgcca tcctgatggg 420
agtcagcaaa gtggccaagg cttttgagct gatcgtcttt tcccgagtgg tgctgggagt 480
ctgtgcaggc atctcctaca gcgcccttcc catgtacctg ggagaactgg cccccaagaa 540
cctgagaggc atggtgggaa caatgaccga ggttttcgtc atcgttggag tcttcctagc 600
acagatcttc agcctccagg ccatcttggg caacccggca ggctggccgg tgcttctggc 660
gctcacaggg gtgcccgccc tgctgcagct gctgaccctg cccttcttcc ccgaaagccc 720

```
ccgctactcc ctgattcaga aaggagatga agccacagcg cgacaagctc tgaggaggct    780
gagaggccac acggacatgg aggccgagct ggaggacatg cgtgcggagg cccgggccga    840
gcgcgccgag ggccacctgt ctgtgctgca cctctgtgcc ctgcggtccc tgcgctggca    900
gctcctctcc atcatcgtgc tcatggccgg ccagcagctg tcgggcatca atgcgatcaa    960
ctactatgcg gacaccatct acacatctgc gggcgtggag gccgctcact cccaatatgt   1020
aacggtgggc tctggcgtcg tcaacatagt gatgaccatc acctcggctg tccttgtgga   1080
gcggctggga cggcggcacc tcctgctggc cggctacggc atctgcggct ctgcctgcct   1140
ggtgctgacg gtggtgctcc tattccagaa cagggtcccc gagctgtcct acctcggcat   1200
catctgtgtc tttgcctaca tcgcgggaca ttccattggg cccagtcctg tcccctcggt   1260
ggtgaggacc gagatcttcc tgcagtcctc ccggcgggca gctttcatgg tggacggggc   1320
agtgcactgg ctcaccaact tcatcatagg cttcctgttc ccatccatcc aggaggccat   1380
cggtgcctac agtttcatca tctttgccgg aatctgcctc ctcactgcga tttacatcta   1440
cgtggttatt ccggagacca agggcaaaac atttgtggag ataaaccgca tttttgccaa   1500
gagaaacagg gtgaagcttc cagaggagaa agaagaaacc attgatgctg ggcctcccac   1560
agcctctcct gccaaggaaa cttcctttta gtggccctgc atgaaggacg ggagcccata   1620
ttcaaggctt ccttctatga caatgggcct cc                                 1652
```

<210> 15
<211> 23
<212> DNA
<213> Artificial Sequence

<220>
<223> Primer

<400> 15
agccaaaggc cgcgctcagt gtc 23

<210> 16
<211> 25
<212> DNA
<213> Artificial Sequence

<220>
<223> Primer

<400> 16
acttgaagac cttgtgcggc gtgtt 25

<210> 17
<211> 26
<212> DNA
<213> Artificial Sequence

<220>
<223> Primer

<400> 17
tggagacggt gcaagaccat agaagc 26

<210> 18
<211> 25
<212> DNA
<213> Artificial Sequence

<220>
<223> Primer

<400> 18
cctttctgcc gcagctatca accag 25

<210> 19
<211> 27
<212> DNA
<213> Artificial Sequence

<220>
<223> Primer

```
<400> 19
ccatcctaat acgactcact atagggc                                          27

<210> 20
<211> 23
<212> DNA
<213> Artificial Sequence

<220>
<223> Primer

<400> 20
actcactata gggctcgagc ggc                                              23

<210> 21
<211> 398
<212> DNA
<213> Homo sapiens

<400> 21
cagctgtgca gacctgtgga gggaggtgaa ggccgtggaa tgcttttagc aaggagcggg      60
tagcgtgctt ccttttcttt caggataacc aggcctggga gagccatgtg gagaagcagc     120
tatgctcaaa cctcatggct cagacatggg ccagggtggc tgagcgaatg ctgggagagc     180
ggatggtgct ggagatgcct ctctcccttc cacccctgct cccctccgct tcccctaccc     240
ttaaagatcg aggggagatg gcctctgaag aatgcccgaa tcctgccctt ctgtctggtt     300
gttcctggat gtgcagagct acgggctgtg ggcagccagg cagctaagtc ggctccagcc     360
gacgctgttg ctggcgacac tgagcgcggc ctttggct                             398

<210> 22
<211> 42
<212> PRT
<213> Homo sapiens

<400> 22
Met Pro Glu Ser Cys Pro Ser Val Trp Leu Phe Leu Asp Val Gln Ser
                5                   10                  15
Tyr Gly Leu Trp Ala Ala Arg Gln Leu Ser Arg Leu Gln Pro Thr Leu
            20                  25                  30
Leu Leu Ala Thr Leu Ser Ala Ala Phe Gly
        35                  40

<210> 23
<211> 521
<212> PRT
<213> Homo sapiens

<400> 23
Met Pro Glu Ser Cys Pro Ser Val Trp Leu Phe Leu Asp Val Gln Ser
                5                   10                  15
Tyr Gly Leu Trp Ala Ala Arg Gln Leu Ser Arg Leu Gln Pro Thr Leu
            20                  25                  30
Leu Leu Ala Thr Leu Ser Ala Ala Phe Gly Ser Ala Phe Gln Tyr Gly
        35                  40                  45
Tyr Asn Leu Ser Val Val Asn Thr Pro His Lys Val Phe Lys Ser Phe
    50                  55                  60
Tyr Asn Glu Thr Tyr Phe Glu Arg His Ala Thr Phe Met Asp Gly Lys
65                  70                  75                  80
Leu Met Leu Leu Leu Trp Ser Cys Thr Val Ser Met Phe Pro Leu Gly
                85                  90                  95
Gly Leu Leu Gly Ser Leu Leu Val Gly Leu Leu Val Asp Ser Cys Gly
            100                 105                 110
Arg Lys Gly Thr Leu Leu Ile Asn Asn Ile Phe Ala Ile Ile Pro Ala
        115                 120                 125
Ile Leu Met Gly Val Ser Lys Val Ala Lys Ala Phe Glu Leu Ile Val
    130                 135                 140
Phe Ser Arg Val Val Leu Gly Val Cys Ala Gly Ile Ser Tyr Ser Ala
145                 150                 155                 160
```

```
Leu Pro Met Tyr Leu Gly Glu Leu Ala Pro Lys Asn Leu Arg Gly Met
                165                 170                 175
Val Gly Thr Met Thr Glu Val Phe Val Ile Val Gly Val Phe Leu Ala
            180                 185                 190
Gln Ile Phe Ser Leu Gln Ala Ile Leu Gly Asn Pro Ala Gly Trp Pro
        195                 200                 205
Val Leu Leu Ala Leu Thr Gly Val Pro Ala Leu Leu Gln Leu Leu Thr
    210                 215                 220
Leu Pro Phe Phe Pro Glu Ser Pro Arg Tyr Ser Leu Ile Gln Lys Gly
225                 230                 235                 240
Asp Glu Ala Thr Ala Arg Gln Ala Leu Arg Arg Leu Arg Gly His Thr
                245                 250                 255
Asp Met Glu Ala Glu Leu Glu Asp Met Arg Ala Glu Ala Arg Ala Glu
            260                 265                 270
Arg Ala Glu Gly His Leu Ser Val Leu His Leu Cys Ala Leu Arg Ser
        275                 280                 285
Leu Arg Trp Gln Leu Leu Ser Ile Ile Val Leu Met Ala Gly Gln Gln
    290                 295                 300
Leu Ser Gly Ile Asn Ala Ile Asn Tyr Tyr Ala Asp Thr Ile Tyr Thr
305                 310                 315                 320
Ser Ala Gly Val Glu Ala Ala His Ser Gln Tyr Val Thr Val Gly Ser
                325                 330                 335
Gly Val Val Asn Ile Val Met Thr Ile Thr Ser Ala Val Leu Val Glu
            340                 345                 350
Arg Leu Gly Arg Arg His Leu Leu Leu Ala Gly Tyr Gly Ile Cys Gly
        355                 360                 365
Ser Ala Cys Leu Val Leu Thr Val Val Leu Leu Phe Gln Asn Arg Val
    370                 375                 380
Pro Glu Leu Ser Tyr Leu Gly Ile Ile Cys Val Phe Ala Tyr Ile Ala
385                 390                 395                 400
Gly His Ser Ile Gly Pro Ser Pro Val Pro Ser Val Val Arg Thr Glu
                405                 410                 415
Ile Phe Leu Gln Ser Ser Arg Arg Ala Ala Phe Met Val Asp Gly Ala
            420                 425                 430
Val His Trp Leu Thr Asn Phe Ile Ile Gly Phe Leu Phe Pro Ser Ile
        435                 440                 445
Gln Glu Ala Ile Gly Ala Tyr Ser Phe Ile Ile Phe Ala Gly Ile Cys
    450                 455                 460
Leu Leu Thr Ala Ile Tyr Ile Tyr Val Val Ile Pro Glu Thr Lys Gly
465                 470                 475                 480
Lys Thr Phe Val Glu Ile Asn Arg Ile Phe Ala Lys Arg Asn Arg Val
                485                 490                 495
Lys Leu Pro Glu Glu Lys Glu Glu Thr Ile Asp Ala Gly Pro Pro Thr
            500                 505                 510
Ala Ser Pro Ala Lys Glu Thr Ser Phe
        515                 520
```

<210> 24
<211> 1563
<212> DNA
<213> Homo sapiens

<400> 24

```
atgcccgaat cctgcccttc tgtctggttg ttcctggatg tgcagagcta cgggctgtgg     60
gcagccaggc agctaagtcg gctccagccg acgctgttgc tggcgacact gagcgcggcc    120
tttggctcag ccttccagta cggctacaac ctctctgtgg tcaacacgcc gcacaaggtc    180
ttcaagtcat tttacaacga aacctacttt gagcgacacg caacattcat ggacgggaag    240
ctcatgctgc ttctatggtc ttgcaccgtc tccatgtttc ctctgggcgg cctgttgggg    300
tcattgctcg tgggcctgct ggttgatagc tgcggcagaa aggggaccct gctgatcaac    360
aacatctttg ccatcatccc cgccatcctg atgggagtca gcaaagtggc caaggctttt    420
gagctgatcg tcttttcccg agtggtgctg ggagtctgtg caggcatctc ctacagcgcc    480
cttcccatgt acctgggaga actggccccc aagaacctga gaggcatggt gggaacaatg    540
accgaggttt tcgtcatcgt tggagtcttc ctagcacaga tcttcagcct ccaggccatc    600
ttgggcaacc cggcaggctg gccggtgctt ctggcgctca caggggtgcc cgccctgctg    660
cagctgctga ccctgccctt cttccccgaa agcccccgct actccctgat tcagaaagga    720
gatgaagcca cagcgcgaca agctctgagg aggctgagag gccacacgga catggaggcc    780
gagctggagg acatgcgtgc ggaggcccgg gccgagcgcg ccgagggcca cctgtctgtg    840
ctgcacctct gtgccctgcg gtccctgcgc tggcagctcc tctccatcat cgtgctcatg    900
```

gccggccagc agctgtcggg catcaatgcg atcaactact atgcggacac catctacaca 960
tctgcgggcg tggaggccgc tcactcccaa tatgtaacgg tgggctctgg cgtcgtcaac 1020
atagtgatga ccatcacctc ggctgtcctt gtggagcggc tgggacggcg gcacctcctg 1080
ctggccggct acggcatctg cggctctgcc tgcctggtgc tgacggtggt gctcctattc 1140
cagaacaggg tccccgagct gtcctacctc ggcatcatct gtgtctttgc ctacatcgcg 1200
ggacattcca ttgggcccag tcctgtcccc tcggtggtga ggaccgagat cttcctgcag 1260
tcctcccggc gggcagcttt catggtggac ggggcagtgc actggctcac caacttcatc 1320
ataggcttcc tgttcccatc catccaggag gccatcggtg cctacagttt catcatcttt 1380
gccggaatct gcctcctcac tgcgatttac atctacgtgg ttattccgga gaccaagggc 1440
aaaacatttg tggagataaa ccgcattttt gccaagagaa acagggtgaa gcttccagag 1500
gagaaagaag aaaccattga tgctgggcct cccacagcct ctcctgccaa ggaaacttcc 1560
ttt 1563

<210> 25
<211> 22
<212> DNA
<213> Artificial Sequence

<220>
<223> Primer

<400> 25
gagaccaagg gcaaaacatt tg 22

<210> 26
<211> 24
<212> DNA
<213> Artificial Sequence

<220>
<223> Primer

<400> 26
ttcttctttc tcctctggaa gctt 24

<210> 27
<211> 29
<212> DNA
<213> Artificial Sequence

<220>
<223> Probe

<400> 27
taaaccgcat ttttgccaag agaaacagg 29

<210> 28
<211> 589
<212> PRT
<213> Homo sapiens

<400> 28
Met Pro Phe Lys Ala Phe Asp Thr Phe Lys Glu Lys Ile Leu Lys Pro
1 5 10 15
Gly Lys Glu Gly Val Lys Asn Ala Val Gly Asp Ser Leu Gly Ile Leu
20 25 30
Gln Arg Lys Ile Asp Gly Thr Thr Glu Glu Glu Asp Asn Ile Glu Leu
35 40 45
Asn Glu Glu Gly Arg Pro Val Gln Thr Ser Arg Pro Ser Pro Pro Leu
50 55 60
Cys Asp Cys His Cys Cys Gly Leu Pro Lys Arg Tyr Ile Ile Ala Ile
65 70 75 80
Met Ser Gly Leu Gly Phe Cys Ile Ser Phe Gly Ile Arg Cys Asn Leu
85 90 95
Gly Val Ala Ile Val Glu Met Val Asn Asn Ser Thr Val Tyr Val Asp
100 105 110
Gly Lys Pro Glu Ile Gln Thr Ala Gln Phe Asn Trp Asp Pro Glu Thr
115 120 125

```
Val Gly Leu Ile His Gly Ser Phe Phe Trp Gly Tyr Ile Met Thr Gln
    130                 135                 140
Ile Pro Gly Gly Phe Ile Ser Asn Lys Phe Ala Ala Asn Arg Val Phe
145                 150                 155                 160
Gly Ala Ala Ile Phe Leu Thr Ser Thr Leu Asn Met Phe Ile Pro Ser
                165                 170                 175
Ala Ala Arg Val His Tyr Gly Cys Val Met Cys Val Arg Ile Leu Gln
            180                 185                 190
Gly Leu Val Glu Gly Val Thr Tyr Pro Ala Cys His Gly Met Trp Ser
        195                 200                 205
Lys Trp Ala Pro Pro Leu Glu Arg Ser Arg Leu Ala Thr Thr Ser Phe
    210                 215                 220
Cys Gly Ser Tyr Ala Gly Ala Val Val Ala Met Pro Leu Ala Gly Val
225                 230                 235                 240
Leu Val Gln Tyr Ile Gly Trp Ser Ser Val Phe Tyr Ile Tyr Gly Met
                245                 250                 255
Phe Gly Ile Ile Trp Tyr Met Phe Trp Leu Leu Gln Ala Tyr Glu Cys
            260                 265                 270
Pro Ala Ala His Pro Thr Ile Ser Asn Glu Glu Lys Thr Tyr Ile Glu
        275                 280                 285
Thr Ser Ile Gly Glu Gly Ala Asn Val Val Ser Leu Ser Lys Phe Ser
    290                 295                 300
Thr Pro Trp Lys Arg Phe Phe Thr Ser Leu Pro Val Tyr Ala Ile Ile
305                 310                 315                 320
Val Ala Asn Phe Cys Arg Ser Trp Thr Phe Tyr Leu Leu Leu Ile Ser
                325                 330                 335
Gln Pro Ala Tyr Phe Glu Glu Val Phe Gly Phe Ala Ile Ser Lys Val
            340                 345                 350
Gly Leu Leu Ser Ala Val Pro His Met Val Met Thr Ile Val Val Pro
        355                 360                 365
Ile Gly Gly Gln Leu Ala Asp Tyr Leu Arg Ser Arg Gln Ile Leu Thr
    370                 375                 380
Thr Thr Ala Val Arg Lys Ile Met Asn Cys Gly Gly Phe Gly Met Glu
385                 390                 395                 400
Ala Thr Leu Leu Leu Val Val Gly Phe Ser His Thr Lys Gly Val Ala
                405                 410                 415
Ile Ser Phe Leu Val Leu Ala Val Gly Phe Ser Gly Phe Ala Ile Ser
            420                 425                 430
Gly Phe Asn Val Asn His Leu Asp Ile Ala Pro Arg Tyr Ala Ser Ile
        435                 440                 445
Leu Met Gly Ile Ser Asn Gly Val Gly Thr Leu Ser Gly Met Val Cys
    450                 455                 460
Pro Leu Ile Val Gly Ala Met Thr Arg His Lys Thr Arg Glu Glu Trp
465                 470                 475                 480
Gln Asn Val Phe Leu Ile Ala Ala Leu Val His Tyr Ser Gly Val Ile
                485                 490                 495
Phe Tyr Gly Val Phe Ala Ser Gly Glu Lys Gln Glu Trp Ala Asp Pro
            500                 505                 510
Glu Asn Leu Ser Glu Glu Lys Cys Gly Ile Ile Asp Gln Asp Glu Leu
        515                 520                 525
Ala Glu Glu Ile Glu Leu Asn His Glu Ser Phe Ala Ser Pro Lys Lys
    530                 535                 540
Lys Met Ser Tyr Gly Ala Thr Ser Gln Asn Cys Glu Val Gln Lys Lys
545                 550                 555                 560
Glu Trp Lys Gly Gln Arg Gly Ala Thr Leu Asp Glu Glu Glu Leu Thr
                565                 570                 575
Ser Tyr Gln Asn Glu Glu Arg Asn Phe Ser Thr Ile Ser
            580                 585
```

<210> 29
<211> 1767
<212> DNA
<213> Homo sapiens

<400> 29

```
atgcctttta aagcatttga taccttcaaa gaaaaaattc tgaaacctgg gaaggaagga     60
gtgaagaacg ccgtgggaga ttctttggga attttacaaa gaaaaatcga tgggacaact    120
gaggaagaag ataacattga gctgaatgaa gaaggaaggc cggtgcagac gtccaggcca    180
```

```
agcccccac tctgcgactg ccactgctgc ggcctcccca agcgttacat cattgctatc   240
atgagtgggc tgggattctg catttccttt gggatccggt gcaatcttgg agttgccatt   300
gtggaaatgg tcaacaatag caccgtatat gttgatggaa aaccggaaat tcagacagca   360
cagtttaact gggatccaga aacagtgggc cttatccatg gatctttttt ctggggctat   420
attatgacac aaattccagg tggtttcatt tcaaacaagt ttgctgctaa cagggtcttt   480
ggagctgcca tcttcttaac atcgactctg aacatgttta ttccctctgc agccagagtg   540
cattacggat gcgtcatgtg tgtcagaatt ctgcaaggtt tagtggaggg tgtgacctac   600
ccagcctgcc atgggatgtg gagtaagtgg gcaccacctt tggagagaag ccgactggcc   660
acaacctctt tttgtggttc ctatgcaggg gcagtggttg ccatgcccct ggctggggtg   720
ttggtgcagt acattggatg gtcctctgtc ttttatattt atggcatgtt tgggattatt   780
tggtacatgt tttggctgtt gcaggcctat gagtgcccag cagctcatcc aacaatatcc   840
aatgaggaga agacctatat agagacaagc ataggagagg gggccaacgt ggttagtcta   900
agtaaattta gtaccccatg gaaaagattt ttcacatctt tgccggttta tgcaatcatt   960
gtggcaaatt tttgcagaag ctggaccttt tatttgctcc tcataagtca gcctgcttat  1020
tttgaagagg tctttggatt tgcaataagt aaggtgggtc tcttgtcagc agtcccacac  1080
atggttatga caatcgttgt acctattgga ggacaattgg ctgattattt aagaagcaga  1140
caaattttaa ccacaactgc tgtcagaaaa atcatgaact gtggaggttt tggcatggag  1200
gcaaccttac tcctggtggt tggcttttcg cataccaaag ggtggctat ctcctttctg   1260
gtacttgctg taggatttag tggcttcgct atttcaggtt ttaatgtcaa ccacctggac  1320
attgccccac gctatgccag cattctcatg gggatctcaa acggagtggg aaccctctct  1380
ggaatggtct gtcccctcat tgtcggtgca atgaccaggc acaagacccg tgaagaatgg  1440
cagaatgtgt tcctcatagc tgccctggtg cattacagtg gtgtgatctt ctatggggtc  1500
tttgcttctg gggagaaaca ggagtgggct gacccagaga atctctctga ggagaaatgt  1560
ggaatcattg accaggacga attagctgag gagatagaac tcaaccatga gagttttgcg  1620
agtcccaaaa agaagatgtc ttatggagcc acctcccaga attgtgaagt ccagaagaag  1680
gaatggaaag gacagagagg agcgaccctt gatgaggaag agctgacatc ctaccagaat  1740
gaagagagaa acttctcaac tatatcc                                      1767
```

<210> 30
<211> 21
<212> DNA
<213> Artificial Sequence

<220>
<223> Primer

<400> 30
gagagttttg cgagtcccaa a 21

<210> 31
<211> 24
<212> DNA
<213> Artificial Sequence

<220>
<223> Primer

<400> 31
ctctctgtcc tttccattcc ttct 24

<210> 32
<211> 28
<212> DNA
<213> Artificial Sequence

<220>
<223> Probe

<400> 32
agccacctcc cagaattgtg aagtccag 28

<210> 33
<211> 23
<212> DNA
<213> Artificial Sequence

<220>
<223> Primer

<400> 33
tgactgttaa cggctcagag gtg 23

<210> 34
<211> 25
<212> DNA
<213> Artificial Sequence

<220>
<223> Primer

<400> 34
agctgggcta caagaaaggc aatag 25

<210> 35
<211> 24
<212> DNA
<213> Artificial Sequence

<220>
<223> Primer

<400> 35
atgcctttta aagcatttga tacc 24

<210> 36
<211> 18
<212> DNA
<213> Artificial Sequence

<220>
<223> Primer

<400> 36
atttaggtga cactatag 18

<210> 37
<211> 19
<212> DNA
<213> Artificial Sequence

<220>
<223> Primer

<400> 37
aatacgactc actataggg 19

<210> 38
<211> 24
<212> DNA
<213> Artificial Sequence

<220>
<223> Primer

<400> 38
aaccggaaat tcagacagca cagt 24

<210> 39
<211> 21
<212> DNA
<213> Artificial Sequence

<220>
<223> Primer

<400> 39

tgggactcgc aaaactctca t 21

<210> 40
<211> 21
<212> DNA
<213> Artificial Sequence

<220>
<223> Primer

<400> 40
tagagacaag cataggagag g 21

<210> 41
<211> 21
<212> DNA
<213> Artificial Sequence

<220>
<223> Primer

<400> 41
cctctcctat gcttgtctct a 21

<210> 42
<211> 20
<212> DNA
<213> Artificial Sequence

<220>
<223> Primer

<400> 42
gtgcctggtc attgcaccga 20

<210> 43
<211> 22
<212> DNA
<213> Artificial Sequence

<220>
<223> Primer

<400> 43
acctggaatt tgtgtcataa ta 22

<210> 44
<211> 25
<212> DNA
<213> Artificial Sequence

<220>
<223> Primer

<400> 44
gaatcattga ccaggacgaa ttagc 25

<210> 45
<211> 1850
<212> DNA
<213> Homo sapiens

<400> 45
atgcctttta aagcatttga taccttcaaa gaaaaaattc tgaaacctgg gaaggaagga 60
gtgaagaacg ccgtgggaga ttctttggga attttacaaa gaaaaatcga tgggacaact 120
gaggaagaag ataacattga gctgaatgaa gaaggaaggc cggtgcagac gtccaggcca 180
agccccccac tctgcgactg ccactgctgc ggcctcccca agcgttacat cattgctatc 240
atgagtgggc tgggattctg catttccttt gggatccggt gcaatcttgg agttgccatt 300

```
gtggaaatgg tcaacaatag caccgtatat gttgatggaa aaccggaaat tcagacagca    360
cagtttaact gggatccaga aacagtgggc cttatccatg gatctttttt ctggggctat    420
attatgacac aaattccagg tggtttcatt tcaaacaagt ttgctgctaa cagggtcttt    480
ggagctgcca tcttcttaac atcgactctg aacatgttta ttccctctgc agccagagtg    540
cattacggat gcgtcatgtg tgtcagaatt ctgcaaggtt tagtggaggg tgtgacctac    600
ccagcctgcc atgggatgtg gagtaagtgg gcaccacctt tggagagaag ccgactggcc    660
acaacctctt tttgtggttc ctatgcaggg gcagtggttg ccatgcccct ggctggggtg    720
ttggtgcagt acattggatg gtcctctgtc ttttatattt atggcatgtt tgggattatt    780
tggtacatgt tttggctgtt gcaggcctat gagtgcccag cagctcatcc aacaatatcc    840
aatgaggaga agacctatat agagacaagc ataggagagg gggccaacgt ggttagtcta    900
agtaaattta gtaccccatg gaaaagattt ttcacatctt tgccggttta tgcaatcatt    960
gtggcaaatt tttgcagaag ctggaccttt tatttgctcc tcataagtca gcctgcttat   1020
tttgaagagg tctttggatt tgcaataagt aaggtgggtc tcttgtcagc agtcccacac   1080
atggttatga caatcgttgt acctattgga ggacaattgg ctgattattt aagaagcaga   1140
caaattttaa ccacaactgc tgtcagaaaa atcatgaact gtggaggttt tggcatggag   1200
gcaaccttac tcctggtggt tggcttttcg cataccaaag gggtggctat ctcctttctg   1260
gtacttgctg taggatttag tggcttcgct atttcaggtt ttaatgtcaa ccacctggac   1320
attgccccac gctatgccag cattctcatg gggatctcaa acggagtggg aaccctctct   1380
ggaatggtct gtcccctcat tgtcggtgca atgaccaggc acaagacccg tgaagaatgg   1440
cagaatgtgt tcctcatagc tgccctggtg cattacagtg gtgtgatctt ctatggggtc   1500
tttgcttctg gggagaaaca ggagtgggct gacccagaga atctctctga ggagaaatgt   1560
ggaatcattg accaggacga attagctgag gagatagaac tcaaccatga gagttttgcg   1620
agtcccaaaa agaagatgtc ttatggagcc acctcccaga attgtgaagt ccagaagaag   1680
gaatggaaag gacagagagg agcgacccttt gatgaggaag agctgacatc ctaccagaat   1740
gaagagagaa acttctcaac tatatcctaa tgtctgagag gcacttctgt cttctcctta   1800
ctttagaacc agaaagtatc catacctatt gcctttcttg tagcccagct              1850
```

<210> 46
<211> 601
<212> PRT
<213> Mus musculus

<400> 46

```
Met Pro Phe Lys Ala Phe Asp Thr Phe Lys Glu Lys Ile Leu Lys Pro
1               5                   10                  15
Gly Lys Glu Gly Val Lys Asn Ala Val Gly Asp Ser Leu Gly Ile Leu
            20                  25                  30
Gln Arg Lys Ile Asp Gly Thr Asn Glu Glu Glu Asp Ala Ile Glu Leu
        35                  40                  45
Asn Glu Glu Gly Arg Pro Val Gln Thr Ser Arg Ala His Arg Pro Val
    50                  55                  60
Cys Asp Cys Ser Cys Cys Gly Ile Pro Lys Arg Tyr Ile Cys Asp Cys
65                  70                  75                  80
Ser Cys Cys Gly Ile Pro Lys Arg Tyr Ile Ile Ala Val Met Ser Gly
                85                  90                  95
Leu Gly Phe Cys Ile Ser Phe Gly Ile Arg Cys Asn Leu Gly Val Ala
            100                 105                 110
Ile Val Glu Met Val Asn Asn Ser Thr Val Tyr Val Asp Gly Lys Pro
        115                 120                 125
Glu Ile Gln Thr Ala Gln Phe Asn Trp Asp Pro Glu Thr Val Gly Leu
    130                 135                 140
Ile His Gly Ser Phe Phe Trp Gly Tyr Ile Val Thr Gln Ile Pro Gly
145                 150                 155                 160
Gly Phe Ile Ser Asn Lys Phe Ala Ala Ser Arg Val Phe Gly Ala Ala
                165                 170                 175
Ile Phe Leu Thr Ser Thr Leu Asn Met Phe Ile Pro Ser Ala Ala Arg
            180                 185                 190
Val His Tyr Gly Cys Val Met Gly Val Arg Ile Leu Gln Gly Leu Val
        195                 200                 205
Glu Gly Val Thr Tyr Pro Ala Cys His Gly Met Trp Ser Lys Trp Ala
    210                 215                 220
Pro Pro Leu Glu Arg Ser Arg Leu Ala Thr Thr Ser Phe Cys Gly Ser
225                 230                 235                 240
Tyr Ala Gly Ala Val Val Ala Met Pro Leu Ala Gly Val Leu Val Gln
                245                 250                 255
Tyr Ile Gly Trp Ala Ser Val Phe Tyr Ile Tyr Gly Met Phe Gly Ile
            260                 265                 270
Ile Trp Tyr Met Phe Trp Leu Leu Gln Ala Tyr Glu Cys Pro Ala Ala
```

```
        275                     280                     285
His Pro Thr Ile Ser Asn Ala Glu Arg Thr Tyr Ile Glu Thr Ser Ile
    290                     295                     300
Gly Glu Gly Ala Asn Leu Ala Ser Leu Ser Lys Phe Asn Thr Pro Trp
305                     310                     315                 320
Arg Arg Phe Phe Thr Ser Leu Pro Val Tyr Ala Ile Ile Val Ala Asn
                325                     330                     335
Phe Cys Arg Ser Trp Thr Phe Tyr Leu Leu Leu Ile Ser Gln Pro Ala
            340                     345                     350
Tyr Phe Glu Glu Val Phe Gly Phe Ala Ile Ser Lys Val Gly Leu Leu
        355                     360                     365
Ser Ala Val Pro His Met Val Met Thr Ile Val Val Pro Ile Gly Gly
    370                     375                     380
Gln Leu Ala Asp Tyr Leu Arg Ser Arg Lys Ile Leu Thr Thr Thr Ala
385                     390                     395                 400
Val Arg Lys Ile Met Asn Cys Gly Gly Phe Gly Met Glu Ala Thr Leu
                405                     410                     415
Leu Leu Val Val Gly Phe Ser His Thr Lys Gly Val Ala Ile Ser Phe
            420                     425                     430
Leu Val Leu Ala Val Gly Phe Ser Gly Phe Ala Ile Ser Gly Phe Asn
        435                     440                     445
Val Asn His Leu Asp Ile Ala Pro Arg Tyr Ala Ser Ile Leu Met Gly
    450                     455                     460
Ile Ser Asn Gly Val Gly Thr Leu Ser Gly Met Val Cys Pro Leu Ile
465                     470                     475                 480
Val Gly Ala Met Thr Lys His Lys Thr Arg Glu Glu Trp Gln Asn Val
                485                     490                     495
Phe Leu Ile Ala Ala Leu Val His Tyr Ser Gly Val Ile Phe Tyr Gly
            500                     505                     510
Val Phe Ala Ser Gly Glu Lys Gln Asp Trp Ala Asp Pro Glu Asn Leu
        515                     520                     525
Ser Glu Asp Lys Cys Gly Ile Ile Asp Gln Asp Glu Leu Ala Glu Glu
    530                     535                     540
Thr Glu Leu Asn His Glu Thr Phe Val Ser Pro Arg Lys Lys Met Ser
545                     550                     555                 560
Tyr Gly Ala Thr Thr Gln Asn Cys Glu Val Gln Lys Thr Glu Trp Arg
                565                     570                     575
Gln Gln Arg Glu Ser Ala Phe Asp Gly Glu Glu Pro Leu Ser Tyr Gln
            580                     585                     590
Ala Glu Gly Asp Phe Ser Glu Thr Ser
        595                     600
```

<210> 47
<211> 1803
<212> DNA
<213> Mus musculus

<400> 47

```
atgccattta aagcatttga taccttcaaa gaaaagattt tgaaacctgg gaaggaagga     60
gtgaagaatg ccgtgggaga ctcgctgggc atcttacaaa gaaaaatcga tgggaccaat    120
gaagaggaag atgccattga gctgaatgaa gaaggaaggc ccgtgcagac gtccagagca    180
catcgccctg tctgtgactg cagctgctgc ggcatcccca agcggtacat ctgtgactgc    240
agctgctgcg gcatccccaa acggtacatc attgctgtca tgagcggcct cggcttctgc    300
atttcctttg ggattcggtg caaccttgga gtggccattg tggaaatggt caacaatagc    360
actgtgtatg tggatgggaa accagaaatt cagacagcgc agtttaactg ggacccagag    420
acggtgggcc tgatccatgg atcttttttc tggggttata ttgtgacaca aattccgggt    480
ggcttcattt caaacaagtt tgctgctagc agggtctttg gagccgccat cttcttgaca    540
tcaactctga acatgtttat cccttctgcg gcgagggtgc attacggctg tgtcatgggt    600
gtgaggattt tgcagggtct ggtggagggt gtgacctacc ctgcctgcca tgggatgtgg    660
agtaagtggg cgcctcccct ggagagaagc cgtctagcca cgacttcctt ctgtggttcc    720
tatgctgggg cagttgttgc tatgcctctt gcaggggtat tggtgcagta cattggctgg    780
gcctctgtct tttacatcta cggtatgttt gggattattt ggtatatgtt ttggctgctg    840
caggcttatg agtgcccggc agctcaccca acaatatcca atgcagaaag gacctacata    900
gagacaagca taggagaagg ggccaacttg gccagtctga gcaaattcaa cacaccatgg    960
agaaggtttt tcacatcctt gcctgtctat gccatcattg tggcaaactt ctgtagaagc   1020
tggaccttct atttgctcct gataagtcag ccagcttact ttgaggaggt ctttggattt   1080
gcaataagta aggtgggtct cttgtcagct gtcccacaca tggtgatgac aatcgtggtg   1140
cccatcggag gacaactggc tgattattta agaagccgaa agattttgac cacaaccgct   1200
```

gtcagaaaga tcatgaattg tggaggcttt ggcatggagg caaccttgct cctggtggtt 1260
ggattttccc ataccaaagg agtggctatc tccttcctgg tgcttgctgt aggatttagt 1320
ggctttgcta tttcaggctt caatgtcaac cacctggaca ttgctccacg ctatgccagc 1380
atcctcatgg ggatctcaaa tggtgtgggg accctctctg gaatggtttg tcccctcatt 1440
gttggtgcaa tgactaagca caagacccgg gaggagtggc agaatgtgtt cctcatagca 1500
gcgctggtac actacagcgg agtcatcttc tacggggtct tcgcttctgg ggaaaaacag 1560
gactgggctg acccagagaa tctctctgag gacaaatgtg gaatcattga ccaagatgag 1620
ttagctgagg agacagaact caaccacgag actttcgtaa gtcccagaaa gaagatgtct 1680
tatggagcca ccacccagaa ttgtgaggtc cagaagaccg agtggagaca acagagagaa 1740
tctgccttcg acggggagga gccattatcc taccaggccg aaggagactt ctcagaaaca 1800
tcc 1803

<210> 48
<211> 23
<212> DNA
<213> Artificial Sequence

<220>
<223> Primer

<400> 48
aggttgcctc ctaagggctc tga 23

<210> 49
<211> 24
<212> DNA
<213> Artificial Sequence

<220>
<223> Primer

<400> 49
gaaaggcgat gggtgcgata cttc 24

<210> 50
<211> 24
<212> DNA
<213> Artificial Sequence

<220>
<223> Primer

<400> 50
atgccattta aagcatttga tacc 24

<210> 51
<211> 24
<212> DNA
<213> Artificial Sequence

<220>
<223> Primer

<400> 51
aggtgaagcc agacatttag gatg 24

<210> 52
<211> 20
<212> DNA
<213> Artificial Sequence

<220>
<223> Primer

<400> 52
aattcagaca gcgcagttta 20

<210> 53

<211> 20
<212> DNA
<213> Artificial Sequence

<220>
<223> Primer

<400> 53
cccttctcct atgcttgtct 20

<210> 54
<211> 20
<212> DNA
<213> Artificial Sequence

<220>
<223> Primer

<400> 54
agacaagcat aggagaaggg 20

<210> 55
<211> 21
<212> DNA
<213> Artificial Sequence

<220>
<223> Primer

<400> 55
cattgaccaa gatgagttag c 21

<210> 56
<211> 20
<212> DNA
<213> Artificial Sequence

<220>
<223> Primer

<400> 56
taaactgcgc tgtctgaatt 20

<210> 57
<211> 21
<212> DNA
<213> Artificial Sequence

<220>
<223> Primer

<400> 57
gctaactcat cttggtcaat g 21

<210> 58
<211> 1822
<212> DNA
<213> Mus musculus

<400> 58
atgccattta aagcatttga taccttcaaa gaaaagattt tgaaacctgg gaaggaagga 60
gtgaagaatg ccgtgggaga ctcgctgggc atcttacaaa gaaaaatcga tgggaccaat 120
gaagaggaag atgccattga gctgaatgaa gaaggaaggc ccgtgcagac gtccagagca 180
catcgccctg tctgtgactg cagctgctgc ggcatcccca agcggtacat ctgtgactgc 240
agctgctgcg gcatccccaa acggtacatc attgctgtca tgagcggcct cggcttctgc 300
atttcctttg ggattcggtg caaccttgga gtggccattg tggaaatggt caacaatagc 360
actgtgtatg tggatgggaa accagaaatt cagacagcgc agtttaactg ggacccagag 420
acggtgggcc tgatccatgg atcttttttc tggggttata ttgtgacaca aattccgggt 480

```
ggcttcattt caaacaagtt tgctgctagc agggtctttg gagccgccat cttcttgaca    540
tcaactctga acatgtttat cccttctgcg gcgagggtgc attacggctg tgtcatgggt    600
gtgaggattt tgcagggtct ggtggagggt gtgacctacc ctgcctgcca tgggatgtgg    660
agtaagtggg cgcctcccct ggagagaagc cgtctagcca cgacttcctt ctgtggttcc    720
tatgctgggg cagttgttgc tatgcctctt gcaggggtat tggtgcagta cattggctgg    780
gcctctgtct tttacatcta cggtatgttt gggattattt ggtatatgtt ttggctgctg    840
caggcttatg agtgcccggc agctcaccca acaatatcca atgcagaaag gacctacata    900
gagacaagca taggagaagg ggccaacttg gccagtctga gcaaattcaa cacaccatgg    960
agaaggtttt tcacatcctt gcctgtctat gccatcattg tggcaaactt ctgtagaagc   1020
tggaccttct atttgctcct gataagtcag ccagcttact ttgaggaggt ctttggattt   1080
gcaataagta aggtgggtct cttgtcagct gtcccacaca tggtgatgac aatcgtggtg   1140
cccatcggag gacaactggc tgattattta agaagccgaa agattttgac cacaaccgct   1200
gtcagaaaga tcatgaattg tggaggcttt ggcatggagg caaccttgct cctggtggtt   1260
ggattttccc ataccaaagg agtggctatc tccttcctgg tgcttgctgt aggatttagt   1320
ggctttgcta tttcaggctt caatgtcaac cacctggaca ttgctccacg ctatgccagc   1380
atcctcatgg ggatctcaaa tggtgtgggg accctctctg gaatggtttg tcccctcatt   1440
gttggtgcaa tgactaagca caagacccgg gaggagtggc agaatgtgtt cctcatagca   1500
gcgctggtac actacagcgg agtcatcttc tacggggtct tcgcttctgg ggaaaaacag   1560
gactgggctg acccagagaa tctctctgag gacaaatgtg gaatcattga ccaagatgag   1620
ttagctgagg agacagaact caaccacgag actttcgtaa gtcccagaaa gaagatgtct   1680
tatggagcca ccacccagaa ttgtgaggtc cagaagaccg agtggagaca acagagagaa   1740
tctgccttcg acggggagga gccattatcc taccaggccg aaggagactt ctcagaaaca   1800
tcctaaatgt ctggcttcac ct                                            1822
```

<210> 59
<211> 20
<212> DNA
<213> Artificial Sequence

<220>
<223> Primer

<400> 59
aggagtgaag aatgccgtgg 20

<210> 60
<211> 19
<212> DNA
<213> Artificial Sequence

<220>
<223> Primer

<400> 60
agggcgatgt gctctggac 19

<210> 61
<211> 26
<212> DNA
<213> Artificial Sequence

<220>
<223> Probe

<400> 61
tgggaccaat gaagaggaag atgcca 26

<210> 62
<211> 436
<212> PRT
<213> Homo sapiens

<400> 62

```
Met Thr Phe Gly Arg Ser Gly Ala Ala Ser Val Val Leu Asn Val Gly
                5                   10                  15
Gly Ala Arg Tyr Ser Leu Ser Arg Glu Leu Leu Lys Asp Phe Pro Leu
            20                  25                  30
Arg Arg Val Ser Arg Leu His Gly Cys Arg Ser Glu Arg Asp Val Leu
```

```
            35                       40                       45
Glu Val Cys Asp Asp Tyr Asp Arg Glu Arg Asn Glu Tyr Phe Phe Asp
    50                      55                      60
Arg His Ser Glu Ala Phe Gly Phe Ile Leu Leu Tyr Val Arg Gly His
65                      70                      75                      80
Gly Lys Leu Arg Phe Ala Pro Arg Met Cys Glu Leu Ser Phe Tyr Asn
                85                      90                      95
Glu Met Ile Tyr Trp Gly Leu Glu Gly Ala His Leu Glu Tyr Cys Cys
            100                     105                     110
Gln Arg Arg Leu Asp Asp Arg Met Ser Asp Thr Tyr Thr Phe Tyr Ser
        115                     120                     125
Ala Asp Glu Pro Gly Val Leu Gly Arg Asp Glu Ala Arg Pro Gly Gly
    130                     135                     140
Ala Glu Ala Ala Pro Ser Arg Arg Trp Leu Glu Arg Met Arg Arg Thr
145                     150                     155                     160
Phe Glu Glu Pro Thr Ser Ser Leu Ala Ala Gln Ile Leu Ala Ser Val
                165                     170                     175
Ser Val Val Phe Val Ile Val Ser Met Val Val Leu Cys Ala Ser Thr
            180                     185                     190
Leu Pro Asp Trp Arg Asn Ala Ala Ala Asp Asn Arg Ser Leu Asp Asp
        195                     200                     205
Arg Ser Arg Tyr Ser Ala Gly Pro Gly Arg Glu Pro Ser Gly Ile Ile
    210                     215                     220
Glu Ala Ile Cys Ile Gly Trp Phe Thr Ala Glu Cys Ile Val Arg Phe
225                     230                     235                     240
Ile Val Ser Lys Asn Lys Cys Glu Phe Val Lys Arg Pro Leu Asn Ile
                245                     250                     255
Ile Asp Leu Leu Ala Ile Thr Pro Tyr Tyr Ile Ser Val Leu Met Thr
            260                     265                     270
Val Phe Thr Gly Glu Asn Ser Gln Leu Gln Arg Ala Gly Val Thr Leu
        275                     280                     285
Arg Val Leu Arg Met Met Arg Ile Phe Trp Val Ile Lys Leu Ala Arg
    290                     295                     300
His Phe Ile Gly Leu Gln Thr Leu Gly Leu Thr Leu Lys Arg Cys Tyr
305                     310                     315                     320
Arg Glu Met Val Met Leu Leu Val Phe Ile Cys Val Ala Met Ala Ile
                325                     330                     335
Phe Ser Ala Leu Ser Gln Leu Leu Glu His Gly Leu Asp Leu Glu Thr
            340                     345                     350
Ser Asn Lys Asp Phe Thr Ser Ile Pro Ala Ala Cys Trp Trp Val Ile
        355                     360                     365
Ile Ser Met Thr Thr Val Gly Tyr Gly Asp Met Tyr Pro Ile Thr Val
    370                     375                     380
Pro Gly Arg Ile Leu Gly Gly Val Cys Val Val Ser Gly Ile Val Leu
385                     390                     395                     400
Leu Ala Leu Pro Ile Thr Phe Ile Tyr His Ser Phe Val Gln Cys Tyr
                405                     410                     415
His Glu Leu Lys Phe Arg Ser Ala Arg Tyr Ser Arg Ser Leu Ser Thr
            420                     425                     430
Glu Phe Leu Asn
        435
```

<210> 63
<211> 1308

<212> DNA
<213> Homo sapiens

<400> 63

```
atgaccttcg ggcgcagcgg ggcggcctcg gtggtgctga acgtgggcgg cgcccggtat     60
tcgctgtccc gggagctgct gaaggacttc ccgctgcgcc gcgtgagccg gctgcacggc    120
tgccgctccg agcgcgacgt gctcgaggtg tgcgacgact acgaccgcga gcgcaacgag    180
tacttcttcg accggcactc ggaggccttc ggcttcatcc tgctctacgt gcgcggccac    240
ggcaagctgc gcttcgcgcc gcggatgtgc gagctctcct tctacaacga gatgatctac    300
tggggcctgg agggcgcgca cctcgagtac tgctgccagc gccgcctcga cgaccgcatg    360
tccgacacct acaccttcta ctcggccgac gagccgggcg tgctgggccg cgacgaggcg    420
cgcccccggcg gggccgaggc ggctccctcc aggcgctggc tggagcgcat gcggcggacc    480
ttcgaggagc ccacgtcgtc gctggccgcg cagatcctgg ctagcgtgtc ggtggtgttc    540
```

gtgatcgtgt ccatggtggt gctgtgcgcc agcacgttgc ccgactggcg caacgcagcc 600
gccgacaacc gcagcctgga tgaccggagc aggtactccg ccggccctgg gagggagccc 660
tccgggataa ttgaagctat ctgcataggt tggttcactg ccgagtgcat cgtgaggttc 720
attgtctcca aaaacaagtg tgagtttgtc aagagacccc tgaacatcat tgatttactg 780
gcaatcacgc cgtattacat ctctgtgttg atgacagtgt ttacaggcga gaactctcaa 840
ctccagaggg ctggagtcac cttgagggta cttagaatga tgaggatttt ttgggtgatt 900
aagcttgccc gtcacttcat tggtcttcag acactcggtt tgactctcaa acgttgctac 960
cgagagatgg ttatgttact tgtcttcatt tgtgttgcca tggcaatctt tagtgcactt 1020
tctcagcttc ttgaacatgg gctggacctg gaaacatcca acaaggactt taccagcatt 1080
cctgctgcct gctggtgggt gattatctct atgactacag ttggctatgg agatatgtat 1140
cctatcacag tgcctggaag aattcttgga ggagtttgtg ttgtcagtgg aattgttcta 1200
ttggcattac ctatcacttt tatctaccat agctttgtgc agtgttatca tgagctcaag 1260
tttagatctg ctaggtatag taggagcctc tccactgaat tcctgaat 1308

<210> 64
<211> 19
<212> DNA
<213> Artificial Sequence

<220>
<223> Primer

<400> 64
ggaaacagct atgaccatg 19

<210> 65
<211> 19
<212> DNA
<213> Artificial Sequence

<220>
<223> Primer

<400> 65
aatacgactc actataggg 19

<210> 66
<211> 23
<212> DNA
<213> Artificial Sequence
<220>
<223> Primer

<400> 66
ctactagcag cgccggagat act 23

<210> 67
<211> 21
<212> DNA
<213> Artificial Sequence

<220>
<223> Primer

<400> 67
cttcggcttc atcctgctct a 21

<210> 68
<211> 24
<212> DNA
<213> Artificial Sequence

<220>
<223> Primer

<400> 68
gtcggccgag tagaaggtgt aggt 24

<210> 69
<211> 21
<212> DNA
<213> Artificial Sequence

<220>
<223> Primer

<400> 69
gcaatcacgc cgtattacat c 21

<210> 70
<211> 21
<212> DNA
<213> Artificial Sequence

<220>
<223> Primer

<400> 70
aaagactttg atgctgcttc a 21

<210> 71
<211> 21
<212> DNA
<213> Artificial Sequence

<220>
<223> Primer

<400> 71
tgttctccct tttccatttt g 21

<210> 72
<211> 21
<212> DNA
<213> Artificial Sequence

<220>
<223> Primer

<400> 72
gctttttcaa ccagttacca t 21

<210> 73
<211> 21
<212> DNA
<213> Artificial Sequence

<220>
<223> Primer

<400> 73
tggattaaag ctttgtggtt c 21

<210> 74
<211> 21
<212> DNA
<213> Artificial Sequence

<220>
<223> Primer

<400> 74
taccaaccca gcttctcaac g 21

<210> 75
<211> 21

<212> DNA
<213> Artificial Sequence

<220>
<223> Primer

<400> 75
acgtttcgat ttctgactgt g 21

<210> 76
<211> 21
<212> DNA
<213> Artificial Sequence

<220>
<223> Primer

<400> 76
gatgtaatac ggcgtgattg c 21

<210> 77
<211> 21
<212> DNA
<213> Artificial Sequence

<220>
<223> Primer

<400> 77
tgaagcagca tcaaagtctt t 21

<210> 78
<211> 21
<212> DNA
<213> Artificial Sequence

<220>
<223> Primer

<400> 78
caaaatggaa aagggagaac a 21

<210> 79
<211> 21
<212> DNA
<213> Artificial Sequence

<220>
<223> Primer

<400> 79
atggtaactg gttgaaaaag c 21

<210> 80
<211> 21
<212> DNA
<213> Artificial Sequence

<220>
<223> Primer

<400> 80
gaaccacaaa gctttaatcc a 21

<210> 81
<211> 21
<212> DNA
<213> Artificial Sequence

<220>
<223> Primer

<400> 81
cgttgagaag ctgggttggt a 21

<210> 82
<211> 21
<212> DNA
<213> Artificial Sequence

<220>
<223> Primer

<400> 82
cacagtcaga aatcgaaacg t 21

<210> 83
<211> 5174
<212> DNA
<213> Homo sapiens

<400> 83
agcgggcctc tcgcctctgg acggcggcgg ggcggccgcc ggattctgcg gccgcaggga 60
gcgctcggag acggggagct attccgccct cggcggctcc attcggcgcc cgcagccctc 120
agggggtcgg ccccgcggct tgggagaggg caccgcggcc tcggtgtgcg cagccctcgg 180
gcgcgagggt cggcggcgcg gacacagccg cgttcccagc cggtggggct cagcgctggc 240
gccggcaagg actccccggc cacccgcagg taccgccggg cggagggcgc gctactagca 300
gcgccggaga tactcgagcc cagggacccc cgggccagcg gagggcagga gcggagcccc 360
gagggagcgc gggccccgac ggcgcgctcc cccgtcagcc acgggcaggc aggccccgcg 420
tggcggcttg gggtgggggg ctgcagcggg gccctcgggc cgaaagtccc ccgggcggcc 480
agccatgacc ttcgggcgca gcggggcggc ctcggtggtg ctgaacgtgg gcggcgcccg 540
gtattcgctg tcccgggagc tgctgaagga cttcccgctg cgccgcgtga gccggctgca 600
cggctgccgc tccgagcgcg acgtgctcga ggtgtgcgac gactacgacc gcgagcgcaa 660
cgagtacttc ttcgaccggc actcggaggc cttcggcttc atcctgctct acgtgcgcgg 720
ccacggcaag ctgcgcttcg cgccgcggat gtgcgagctc tccttctaca acgagatgat 780
ctactggggc ctggagggcg cgcacctcga gtactgctgc cagcgccgcc tcgacgaccg 840
catgtccgac acctacacct tctactcggc cgacgagccg ggcgtgctgg gccgcgacga 900
ggcgcgcccc ggcggggccg aggcggctcc ctccaggcgc tggctggagc gcatgcggcg 960
gaccttcgag gagcccacgt cgtcgctggc cgcgcagatc ctggctagcg tgtcggtggt 1020
gttcgtgatc gtgtccatgg tggtgctgtg cgccagcacg ttgcccgact ggcgcaacgc 1080
agccgccgac aaccgcagcc tggatgaccg gagcaggtac tccgccggcc ctgggaggga 1140
gccctccggg ataattgaag ctatctgcat aggttggttc actgccgagt gcatcgtgag 1200
gttcattgtc tccaaaaaca agtgtgagtt tgtcaagaga cccctgaaca tcattgattt 1260
actggcaatc acgccgtatt acatctctgt gttgatgaca gtgtttacag gcgagaactc 1320
tcaactccag agggctggag tcaccttgag ggtacttaga atgatgagga ttttttgggt 1380
gattaagctt gcccgtcact tcattggtct tcagacactc ggtttgactc tcaaacgttg 1440
ctaccgagag atggttatgt tacttgtctt catttgtgtt gccatggcaa tctttagtgc 1500
actttctcag cttcttgaac atgggctgga cctggaaaca tccaacaagg actttaccag 1560
cattcctgct gcctgctggt gggtgattat ctctatgact acagttggct atggagatat 1620
gtatcctatc acagtgcctg gaagaattct tggaggagtt tgtgttgtca gtggaattgt 1680
tctattggca ttacctatca cttttatcta ccatagcttt gtgcagtgtt atcatgagct 1740
caagtttaga tctgctaggt atagtaggag cctctccact gaattcctga attaatgcat 1800
tgcaaatcaa ttcttgcata cacttcatag aaagactttg atgctgcttc atatttatgt 1860
gtttcttgct gggtgagcac tgcagtggca ttgtcatcat cttggtaggg taaaaattat 1920
ccttcccagc cgaagggata aaacagttta cttgttatgg agtaaataga attgagactg 1980
caaaggaaga ataatgactc ctagagtaaa ctttaggacc cggttttatt tagacttgtt 2040
ttcccgtttc cttgaatgat tacacatttt taaaaaatac attatttgaa cattttaaaa 2100
cagaaaggta ctattttcca aatgtttttc catcttatga attcagtaga agcttggaac 2160
ttatagtgtt ttttgtttga gagtaacatt ttcatttcta aatgttttat aatttctcat 2220
atcaatgtca gaagtatcct ggaaacatat gtcacatgcg ggaactgttt aacaaatact 2280
ttaaaaattt ggccaaaatt taaactgtat aatggagcta gatacaagca agaatagtat 2340
ttgaaagact tttccagcat acttctcaat tctttgcttt atttttgtgc caattattca 2400
ccttatcgtg ccgcttcatg gaagcttgag tatgttctcc cttttccatt ttggatttat 2460
ctctttactg taatgactca aaaggtattt aagaattgac gagagcttgt gttgtttagc 2520
atcttactgg ataatatttg aattcattgc tgttcctagg tgataactgt cctaatattt 2580
agatgtccaa acaagaatac ttccaacata aaaattataa taggaataat ttgagatgac 2640

tcaatattac aacctcttct tctcttaacc tcctccccca aacactagag gtttaataag 2700
acttatcaga tgaaaggata tttatatagc cttttagtag caaagtcata cttacgtgtt 2760
gtcactggat tatcataaaa gggagaaatt aaatattact gtactcttag ttgctgtgta 2820
gctaagtcaa ttttaagcca gtaaaagcga tggatacata atgatttgat ctgatcttta 2880
actattgtga atcacagcta caccaaaact cttcttgtaa gaatactgac taatatgcca 2940
tgttaatctg gctagattat taggactaga taatgtaaaa gtgatgattg tttagtaact 3000
aaattttagc aacagaaatt agaattttgc tttttcaacc agttaccata aagaagttag 3060
tgtatatata aacacaaata attagtgaca gattcataaa aaattgaatg ttgtacacag 3120
taattttgtc agaggtagag aagacaggga ttgggaagtg gtgggtgatg gaggacctgg 3180
atatatttat caaataaagg gttaccagaa gtgttcatta aaggaatttt agccatcatc 3240
tagttcaagc ctcaactatt acaggtagaa aatcagggca ggagagaata taattgtgaa 3300
ggagtcaggg ctaacacctg gatctccaga aacctagccc agcaggttaa tcttcacaca 3360
tctctgggtt ctgagaaaag cctggaaaaa tcacacttct ttgtcattgt catgctgagg 3420
taataatagc aaaactgttt tctttccctt aatttccttt cctaagctta tgtaatagtt 3480
tggccattaa atatcttgcc ctattttccc tattactgct agtatgctac ttcttacata 3540
cccaaaagaa attcagttat ttattgtata tttattgtat tctaatataa ttgaaataaa 3600
tggcatggat ttattttttc ttaactattt ggattaaagc tttgtggttc atgcaaacaa 3660
tgtgcagatg atagcacctc catattacta ataaaaatat gataaccatc aaaaaaaaaa 3720
aaaaaaaaaa aaaaaaaaaa aaaaaaaaaa aaaaaaaaaa aacaaaaaaa aaaaaaaact 3780
ctccagcgct ggatccggcc ataagggcct gatccttcga ggggggcccc ggtactgaga 3840
gatcccctca taatttcccc aaagcgtaac catgtgtgaa taaattttga gctagtaggg 3900
ttgcagccac gagtaagtct tcccttgtta ttgtgtagcc agaatgccgc aaaacttcca 3960
tgcctaagcg aactgttgag agtacgtttc gatttctgac tgtgttagcc tggaagtgct 4020
tgtcccaacc ttgtttctga gcatgaacgc ccgcaagcca acatgttagt tgaagcatca 4080
gggcgattag cagcatgata tcaaaacgct ctgagctgct cgttcggcta tggcgtaggc 4140
ctagtccgta ggcaggactt ttcaagtctc ggaaggtttc ttcaatctgc attcgcttcg 4200
aatagatatt aacaagttgt ttgggtgttc gaatttcaac aggtaagtta gttgctagaa 4260
cccatggctc ctttgccgac gctgagtaga ttttaggtga cgggtggtga caatgagtcc 4320
gtgtcgagcg ctgatttttt cggcctttag agcgagattt atacaataga atttggcatg 4380
agattggatt gcttttagtc agcctcttat agcctaaagt ctttgagtga ctagatgaca 4440
tatcatgtaa gttgctgata ggtttccagt tttccgctcc taggtctgca tattgtactt 4500
ttcctcttac tcgacttaac cagtaccaac ccagcttctc aacggattta taccatggca 4560
ctttaaagcc agcatcactg acaatgagcg gtgtggtgtt actcggtaga atgctcgcaa 4620
ggtcggctag aaattggtca tgagctttct ttgaacattg ctctgaaagc gggaacgctt 4680
tctcataaag agtaacagaa cgaccgtgta gtgcgactga agctcgcaat accataagtc 4740
gtttttgctc acgaatatca gaccagtcaa caagtacaat gggcatcgta ttgcccgaac 4800
agataaagct agcatgccaa cggtatacag cgagtcgctc tttgtggagg tgacgattac 4860
ctaacaatcg gtcgattcgt ttgatgttat gttttgttct cgctttggtt ggcaggttac 4920
ggccaagttc ggtaagagtg agagtttttac agtcaagtaa tgcgtggcaa gccaacgtta 4980
agctgttgag tcgttttaag tgtaattcgg ggcagaattg gtaaagagag tcgtgtaaaa 5040
tatcgagttc gcacatcttg ttgtctgatt attgattttt cgcgaaacca tttgatcata 5100
tgacaagatg tgtatccacc ttaacttaat gatttttacc aaaatcatta ggggattcat 5160
cagggcccgg tacc 5174

<210> 84
<211> 24
<212> DNA
<213> Artificial Sequence

<220>
<223> Primer

<400> 84
tgtgccaatt attcacctta tcgt 24

<210> 85
<211> 27
<212> DNA
<213> Artificial Sequence

<220>
<223> Primer

<400> 85
caagctctcg tcaattctta aatacct 27

<210> 86
<211> 30

<212> DNA
<213> Artificial Sequence

<220>
<223> Probe

<400> 86
cgcttcatgg aagcttgagt atgttctccc 30

<210> 87
<211> 20
<212> DNA
<213> Artificial Sequence

<220>
<223> Primer

<400> 87
agcaccacca tggacacgat 20

<210> 88
<211> 25
<212> DNA
<213> Artificial Sequence

<220>
<223> Primer

<400> 88
cgggatcaac gcggtcaact actac 25

<210> 89
<211> 25
<212> DNA
<213> Artificial Sequence

<220>
<223> Primer

<400> 89
atacagacgc cagcgaagac gagaa 25

<210> 90
<211> 477
<212> DNA
<213> Mus musculus

<400> 90
cgggatcaac gcggtcaact actacgcaga tgtcatctac acctcagctg gtgtggaccc 60
cacccagtcc cagtatgtga ccctgggctc tggtgtcatc aacttggtga tgactctcgt 120
ctcggccgtt atcatagagc gtttggggcg acggattctc ctgctgtctg gctatgccat 180
ctgctgctct gcctgcctgg tactgaccgt ggcactcctc ctccagagca cggcccctga 240
gctgtcttac cttagtatcg tctgtgtgtt ctcctacatc gtgggacatt ccataggacc 300
cagtcctgtc ccctcggtgg tgaggacaga gattgtcctg cagtcctctc ggacagccgc 360
cttcactgtg gacggggctg tgcactggct caccaacttc atcgtggggt tgacgtttcc 420
atccatccag gtggccatcg gagcctacag ctttctcgtc ttcgctggcg tctgtat 477

<210> 91
<211> 21
<212> DNA
<213> Artificial Sequence

<220>
<223> Primer

<400> 91
tgggctctgg tgtcatcaac t 21

<210> 92
<211> 22
<212> DNA
<213> Artificial Sequence

<220>
<223> Primer

<400> 92
tagccagaca gcaggagaat cc 22

<210> 93
<211> 27
<212> DNA
<213> Artificial Sequence

<220>
<223> Probe

<400> 93
tgatgactct cgtctcggcc gttatca 27

<210> 94
<211> 30
<212> DNA
<213> Artificial Sequence

<220>
<223> Primer

<400> 94
caccgtcgac atggagaaca aagaggcggg 30

<210> 95
<211> 30
<212> DNA
<213> Artificial Sequence

<220>
<223> Primer

<400> 95
actagtctaa aaggaagttt ccttggcagg 30

<210> 96
<211> 18
<212> DNA
<213> Artificial Sequence

<220>
<223> Primer

<400> 96
tagaaggcac agtcgagg 18

<210> 97
<211> 536
<212> DNA
<213> Rattus norvegicus

<400> 97
taattcagac agcgcagttt aactgggatc cagagacggt gggtcttatc catggatctt 60
ttttctgggg ttatattgtg acacaaattc ccggtggctt catttcaaac aagtttgctg 120
ctaacagggt ctttggagct gccatcttct tgacgtcaac cctgaacatg ttcatccctt 180
ccgcggccag ggtgcattac ggctgtgtca tgtgtgtgag gattttgcag ggtctggtgg 240
agggtgtgac ctacccagcc tgccacggga tgtggagtaa gtgggcacct cccctggaga 300
gaagtcgtct agccacaacc tctttttgtg gttcctatgc cggggcagtc gttgctatgc 360
cccttgcagg agtattggtg cagtacattg gctgggcctc tgtcttttat atttacggga 420

tgtttggaat tatttggtac atgttttggc tgctgcaggc ttatgagtgt ccagcagttc 480
acccaacaat atccaatgaa gaacggacct acatagagac aagcatagga gaaggg 536

<210> 98
<211> 18
<212> DNA
<213> Artificial Sequence

<220>
<223> Primer

<400> 98
tcccggtggc ttcatttc 18

<210> 99
<211> 18
<212> DNA
<213> Artificial Sequence

<220>
<223> Primer

<400> 99
cgcggaaggg atgaacat 18

<210> 100
<211> 29
<212> DNA
<213> Artificial Sequence

<220>
<223> Probe

<400> 100
cagctccaaa gaccctgtta gcagcaaac 29

<210> 101
<211> 39
<212> DNA
<213> Artificial Sequence

<220>
<223> Primer

<400> 101
cggtaagctt gtcgacatgc cttttaaagc atttgatac 39

<210> 102
<211> 39
<212> DNA
<213> Artificial Sequence

<220>
<223> Primer

<400> 102
ccgatctaga actagtttag gatatagttg agaagtttc 39

<210> 103
<211> 23
<212> DNA
<213> Artificial Sequence

<220>
<223> Primer

<400> 103
atgtgcgagc tctccttcta caa 23

<210> 104
<211> 25
<212> DNA
<213> Artificial Sequence

<220>
<223> Primer

<400> 104
ttaattcagg aactcagctg agagg 25

<210> 105
<211> 950
<212> DNA
<213> Mus musculus

<220>
<221> variation
<222> 726
<223> n is a, g, t or c

<220>
<221> variation
<222> 731
<223> n is a, g, t or c

<220>
<221> variation
<222> 795
<223> n is a, g, t or c

<220>
<221> variation
<222> 807
<223> n is a, g, t or c

<220>
<221> variation
<222> 836
<223> n is a, g, t or c

<220>
<221> variation
<222> 888
<223> n is a, g, t or c

<400> 105
gatctactgg ggcctggagg gtgcgcacct ggagtactgc tgccagcgcc gcctagacga 60
ccgcatgtcc gacacccaca cctttcacgc ggcagacgag ctgggccgcg agcagcctcg 120
tcccgccgga cccgaggcgg ccccctcccg gcgctggctg gagcgcatgc ggcggacctt 180
cgaggagccc acgtcgtcgc tggccgcgca gatcctggcc agcgtgtccg tggtgttcgt 240
gatcgtgtcc atggtggtgc tgtgcgccag cacgctgccc gactggcgtg cggcggttgc 300
tgacaaccgc agtctggatg accggagcag gataattgaa gctatctgca tagggtggtt 360
caccgcggag tgcatcgtgc ggttcatcgt ctccaaaaac aagtgtgagt ttgtcaagag 420
acccctgaac atcattgact tactggcaat cacgccctat tacatctctg tgctaatgac 480
agtgtttaca ggcgagaact ctcaactcca gagggctggg gtcaccttga gggtcctccg 540
aatgatgcgg atcttctggg tgatcaagct tgcccggcac ttcattggtc tgcagacact 600
gggcttgact ctcaagcgat gctaccgaga gatggctatg ttacttgtct tcatctgtgt 660
tgccatggca atctttagtg cactctctca gctccttgaa catgcgctgg acctggaaac 720
atccancaag natttcgcca gcatccccgc tgcctgctgg tgggtgatta tctctataac 780
tacagttggc tatgnagata tgtatcntat cacggtgcct ggaagaattc cttggnagga 840
agtttgtgtt gtacagtggt ggattgttac tgttggcatt acccatcnct ttcatcctac 900
catagctttg tgcagtgcta ccacgaaggc tcaagtttag atcggactag 950

<210> 106
<211> 19
<212> DNA

```
<213> Artificial Sequence

<220>
<223> Primer

<400> 106
tgctgacaac cgcagtctg                                                    19

<210> 107
<211> 19
<212> DNA
<213> Artificial Sequence

<220>
<223> Primer

<400> 107
gatgaaccgc acgatgcac                                                    19

<210> 108
<211> 25
<212> DNA
<213> Artificial Sequence

<220>
<223> Probe

<400> 108
cgcggtgaac caccctatgc agata                                             25
```